(19) Europäisches Patentamt
European Patent Office
Office européen des brevets

(11) EP 4 385 979 A1

(12) EUROPEAN PATENT APPLICATION

(43) Date of publication:
**19.06.2024 Bulletin 2024/25**

(21) Application number: **22213346.4**

(22) Date of filing: **14.12.2022**

(51) International Patent Classification (IPC):
*C07D 231/14* (2006.01) *C07D 401/12* (2006.01)
*C07D 403/12* (2006.01) *C07D 405/14* (2006.01)
*A61P 29/00* (2006.01) *A61K 31/4155* (2006.01)

(52) Cooperative Patent Classification (CPC):
**C07D 401/12; A61P 29/00; C07D 231/14; C07D 403/12; C07D 405/14**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Grünenthal GmbH**
**52078 Aachen (DE)**

(72) Inventors:
- **Krüger, Sebastian**
  **52078 Achen (DE)**
- **Mülbaier, Marcel**
  **52078 Achen (DE)**
- **Dialer, Clemens**
  **52078 Achen (DE)**
- **Pettersson, Martin**
  **52078 Achen (DE)**

(54) **MULTICYCLIC INHIBITORS OF NAV1.8**

(57) The invention relates to compounds according to general formula (I)

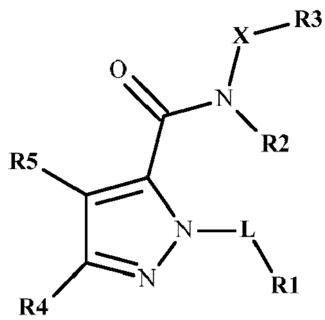

(I)

which act as inhibitors of $Na_V1.8$ and can be used in the treatment of pain.



Processed by Luminess, 75001 PARIS (FR)

## Description

The invention relates to compounds according to general formula (I)

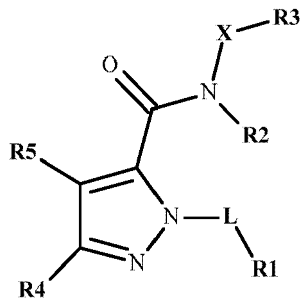

(I)

which act as inhibitors of $Na_V1.8$ and can be used in the treatment of pain.

Normal pain sensation (nociception) serves primarily as a survival mechanism, the body's way of self-protection, alerting against (further) tissue damage and disease from noxious stimuli. For instance, acute pain can arise when the external environment (temperature, pressure, chemicals) activates modality specific receptors (nociceptors) and ion channels within the skin. The peripheral terminals of pain-signaling neurons - whose cell bodies are found in the dorsal root ganglia [DRG] and trigeminal ganglia [TG] - convert the external stimuli into electrochemical generator potentials. Specific voltage-gated sodium channels ($Na_V$s) integrate and amplify these generator signals until the threshold for an action potential [AP] is reached. Thus, what starts as a noxious stimuli in the periphery eventually leads to action potential firing that travels towards the central nervous system, synapsing first onto neurons in the spinal cord and then towards the brain. $Na_V$s also function to support propagation of action potentials to the central terminals within the spinal cord. At the end of its travels along the somatosensory pathway, the action potential signal is interpreted as pain by the brain (Lumpkin and Caterina, Nature (2007), Vol.445 pp 858-865; and Crawford and Caterina Toxicologic Pathology (2020) 48(1)-174; Goodwin G and McMahon S.B. Nature Reviews Neuroscience (2021) Vol 22 pp 263-274; Bennett D.L. et al. Physiol Rev 99 (2019) Vol 99 pp 1079-1151).

Abnormal persistent neuropathic pain arises as a consequence of a lesion or disease of this somatosensory pathway. In response to nerve injury or inflammation, abnormal changes in ion channel expression can cause hyper-excitability of pain-signaling neurons and their nerves/axons, thus resulting in pathological pain.

The voltage-gated $Na_V1.8$ sodium channel is a therapeutic target for analgesia because of its restricted expression profile (almost exclusive to peripheral sensory tissues), its placement along the pain pathway (free nerve endings, sciatic nerve, and DRG), prominent physiological role in pain signaling (supports upstroke of AP and facilitates repetitive AP firing), and supporting genetic/pharmaco-phenotypic evidence (human/animal studies showing changes in $Na_V1.8$ function cause parallel changes in pain sensitivity).

Regarding its expression profile along the pain pathway, because $Na_V1.8$ was first found predominantly in peripheral sensory neurons of the dorsal root ganglia (DRG) and trigeminal ganglia (TG), it was originally termed SNS (sensory neuron specific) (Akopian A.N. et al Nature (1996) Vol 379 pp 257-261) or PN3 (peripheral nerve 3) (Sangameswaran L. et al J. Biol. Chem. (1996) Vol 271 pp 5953-5956). As well, $Na_V1.8$ is localized at free nerve endings, where pain signaling is initiated in the skin (Persson A.K. et al Mol. Pain. (2010) 6:84) and is diffusely localized along the entire length of non-myelinated axons of sciatic nerve (Rush A.M. et al. Eur.J.Neurosci (2005) Vol 22 pp 39-49).

In contrast, $Na_V1.8$ has minimal expression in nonneuronal tissue, such as heart and skeletal muscle, and in the CNS, including brain and spinal cord (9, 10, 338, 406) (Akopian A.N. op. cit.; Akopian A.N. et al. Nat. Neurosci (1999) Vol 2 pp 541-548; Novakovic S.D. et al. J. Neurosci. (1998) Vol 18, pp 2184-2187; and Sagameswaran L. op. cit.).

Regarding its physiological role, $Na_V1.8$ contributes the majority of the inward current during the rising phase of an all-or-none action potential in nociceptive sensory neurons (Blair N.T. et al. J. Neurosci. (2003) Vol 23 pp 10338-10350 and Renganathan M et al. J. Neurophysiol (2001) Vol 86 pp 629-640) - and also contributes most of the current in subsequent spikes during repetitive firing in DRG neurons (Choi J.S. J. Neurophysiol (2011) Vol 106 pp 3173-3184; and Tan Z.Y. et al. J. Neurosci. (2014) Vol 34 pp 7190-7197).

Regarding genetic and pharmacology studies, gain-of-function mutations in $Na_V1.8$ were found in patients with chronic neuropathic pain such as small fiber neuropathy (Faber C.G. et al. (2012) Ann. Neurol Vol 71 pp 26-39; Han C et al. J. Neurol Neurosurg Psychiatry (2014) Vol 85 pp 499-505; and, Kist A.M. et al. PLoS One (2016) Vol 11 e0161789); Eijkenboom I. et al J. Neurol Neurosurg Psychiatry (2019) 90 (3) pp 342-352); loss-of-function (gene knockout) studies in mice reduced pain sensitivity, notably in nociception (Laird J.M. et al. J. Neurosci (2002) J. Neurosci Vol 22 pp

8352-8356; Jarvis M.F. et al Proc Natl Acad Sci USA (2007) Vol 104 pp 8520-8525; Joshi S.K. et al Pain (2006) Vol 123 pp 75-82) and in neuropathic models (Roza C. et al J Physiol (2003) Vol 550 pp 921-926); $Na_V$1.8-selective small molecule inhibitors reduced pain in rodents, specifically in inflammatory and neuropathic models (Jarvis et al. op. cit.; Kort M.E. et al Bioorg Med Chem Lett (2010) Vol 20 pp 6812-6815; Scanio M.J. et al Bioorg Med Chem (2010) Vol 18 pp 7816-7825; Payne C.E. et al Br J Pharmacol (2015) Vol 172 pp 2654-2670).

Currently, non-selective $Na_V$ channel inhibitors are used to treat epilepsy, cardiac arrhythmia, and chronic pain (Hille, B. J. Gen. Physiol. (1977) Vol. 69 pp. 497-515; Hille. B, Ion Channels of Excitable Membranes (1992) pp. 391-421; Sunderland, Mass., Sinauer Associates, Inc. 3rd ed.; Hondeghem L.M. and Katzung B.G. Annu. Rev. Pharmacol. Toxicol. (1984) Vol. 24. Pp. 387-423; Catterall W.A. Trends Pharmacol. Sci. (1987) Vol. 8 pp.57-65) - however, all of these analgesics have limited efficacy owing to dose-limiting adverse side-effects related to inhibiting $Na_V$1.1/$Na_V$1.2/1.6 (seizure liability), inhibiting $Na_V$1.4 (muscle weakness/paralysis), inhibiting $Na_V$1.5 (arrhythmia risk).

There is a need to develop a $Na_V$1.8-selective small molecule inhibitor as an effective and safe analgesic.

$Na_V$1.8 inhibitors are also known from WO 2020/092187, WO 2020/092667, WO 2009/049180, WO 2009/049183, WO 2009/049181 and WO 2021/113627.

It was an object of the invention to provide novel compounds which are inhibitors, preferably selective inhibitors, of $Na_V$1.8, and which preferably have advantages over the compounds of the prior art. The novel compounds should in particular be suitable for use in the treatment of pain.

This object has been achieved by the subject-matter of the patent claims.

It was surprisingly found that the compounds according to the invention are highly potent and selective inhibitors of the $Na_V$1.8 channel.

The invention relates to a compound according to general formula (I)

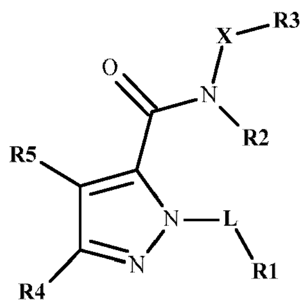

(I)

wherein

**L** represents $CH_2$, $CH(CH_3)$ or $CH(CH_2CH_3)$;

**R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl; with the proviso that when R1 represents $C_{3-10}$-cycloalkyl or 4 to 10-membered heterocycloalkyl, said $C_{3-10}$-cycloalkyl or 4 to 10-membered heterocycloalkyl is substituted with at least two substituients, wherein said at least two substituents are independently from one another selected from $C_{1-6}$-alkyl, $CF_3$, $CF_2H$, and $CFH_2$;

**R2** represents H or $C_{1-6}$-alkyl;

X represents phenyl, or 5 to 10-membered heteroaryl;

**R3** represents $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), $S(O)$-$NH_2$, $S(O)$-$N(H)(C_{1-6}$-alkyl), $S(O)$-$N(C_{1-6}$-alkyl$)_2$, $S(O)_2$-$NH_2$, $S(O)_2$-$N(H)(C_{1-6}$-alkyl), $S(O)_2$-$N(H)(C_{3-6}$-cycloalkyl), $S(O)_2$-$N(C_{1-6}$-alkyl$)_2$, $C(O)$-$NH_2$, $C(O)$-$N(H)(C_{1-6}$-alkyl), $C(O)$-$N(H)(C_{3-6}$-cycloalkyl), $C(O)$-$N(C_{1-6}$-alkyl$)_2$, $C(O)$-$N(C_{1-6}$-alkyl$)(C_{3-6}$-cycloalkyl), $OCF_3$, $OCF_2H$, CN, OH, O-$C_{3-6}$-cycloalkyl, O-(4 to 6-membered heterocycloalkyl), $S(O)$-$C_{1-6}$-alkyl, $S(O)$-($C_{3-6}$-cycloalkyl), $S(O)$-(4 to 6-membered heterocycloalkyl), $S(O)$-phenyl, or $S(O)$-(5 or 6-membered heteroaryl);

**R4** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to

11-membered bicycloalkyl, 5 to 11-membered spiroalkyl orbisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

**R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

wherein $C_{1-6}$-alkyl and $C_{1-6}$-alkylene in each case independently from one another is linear or branched;

wherein $C_{1-6}$-alkyl, $C_{1-6}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl, and 5 to 11-membered heterobicycloalkyl in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;

wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl;

in the form of the free compound or a physiologically acceptable salt thereof.

In a preferred embodiment, the compound according to the invention is present in form of the free compound. For the purpose of specification, "free compound" preferably means that the compound according to the invention is not present in form of a salt. Methods to determine whether a chemical substance is present as the free compound or as a salt are known to the skilled artisan such as $^{14}N$ or $^{15}N$ solid state NMR, x-ray diffraction, x-ray powder diffraction, IR, Raman, XPS. $^{1}H$-NMR recorded in solution may also be used to consider the presence of protonation.

In another preferred embodiment, the compound according to the invention is present in form of a physiologically acceptable salt. For the purposes of this specification, the term "physiologically acceptable salt" preferably refers to a salt obtained from a compound according to the invention and a physiologically acceptable acid or base.

According to the invention, the compound according to the invention may be present in any possible form including solvates, cocrystals and polymorphs. For the purposes of this specification, the term "solvate" preferably refers to an adduct of (i) a compound according to the invention and/or a physiologically acceptable salt thereof with (ii) distinct molecular equivalents of one or more solvents.

Further, the compound according to the invention may be present in form of the racemate, enantiomers, diastereomers, tautomers or any mixtures thereof.

The compounds according to the invention may have one or more stereocenter. The person skilled in art knows by looking at a chemical structure whether the depicted compound has one or more stereocenters or not.

For some compounds according to the invention that have one or more stereocenters and which chemical structures are disclosed in the examples of the present application, the chemical structure includes bold bonds and/or hashed bonds to indicate the relative structural orientation of those substituents connected by the bold bonds and/or hashed bonds to the superior structure. If the bold bonds and/or hashed bonds are depicted in form of a wedge, the absolute stereochemical configuration of the compound is known and thereby indicated. If the bold bonds and/or hashed bonds are depicted as a straight bond (i.e. no wedge), the absolute stereochemical configuration of the compound has not been determined. In that case, the bold bonds and/or hashed bonds merely serve to indicate that this particular compound is present as one enantiomer or one diastereomer (e.g. cis-diastereomer (i.e. mixture of two cisenantiomers) or trans-diastereomer (i.e. mixture of two trans-enantiomers)). All compounds according to the invention that have one or more stereocenters but which chemical structures disclosed in the examples of the present application do not include bold bonds and/or hashed bonds, are present as a mixture of the respective stereoisomers.

The invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are $^{2}H$ (deuterium), $^{3}H$

(tritium), $^{13}C$ and $^{14}C$. Isotopic isomers of a compound of the invention can generally be prepared by conventional procedures known to a person skilled in the art.

According to the invention, the terms "$C_{1-6}$-alkyl" and "$C_{1-4}$-alkyl" preferably mean acyclic and preferably saturated hydrocarbon residues, which can be linear (i.e. unbranched) or branched and which can be unsubstituted or mono- or polysubstituted (e.g. di- or trisubstituted), and which contain 1 to 6 (i.e. 1, 2, 3, 4, 5 or 6) and 1 to 4 (i.e. 1, 2, 3 or 4) carbon atoms, respectively. Preferably, $C_{1-6}$-alkyl and $C_{1-4}$-alkyl are saturated. Preferred $C_{1-6}$-alkyl groups are selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl. Particularly preferred $C_{1-6}$-alkyl groups are selected from $C_{1-4}$-alkyl groups. Preferred $C_{1-4}$-alkyl groups are selected from the group consisting of methyl, ethyl, n-propyl, 2-propyl, n-butyl, isobutyl, sec-butyl and tert-butyl.

According to the invention, the terms "$C_{1-6}$-alkylene" and "$C_{1-4}$-alkylene" relate to linear or branched and preferably saturated aliphatic residues which can be unsubstituted or mono- or polysubstituted (e.g. di- or trisubstituted) and which are preferably selected from the group consisting of $CH_2$, $CH(CH_3)$, $CH_2CH_2$, $CH_2CH(CH_3)$, $CH(CH_3)CH_2$, $CH_2CH_2CH_2$, $CH(CH_3)CH_2CH_2$, $CH_2CH(CH_3)CH_2$, $CH_2CH_2CH(CH_3)$, $CH_2CH_2CH_2CH_2$, $CH(CH_3)CH_2CH_2CH_2$, $CH_2CH(CH_3)CH_2CH_2$, $CH_2CH_2CH(CH_3)CH_2$, $CH_2CH_2CH_2CH(CH_3)$, $CH_2CH_2CH_2CH_2CH_2$, $CH(CH_3)CH_2CH_2CH_2CH_2$, $CH_2CH(CH_3)CH_2CH_2CH_2$, $CH_2CH_2CH(CH_3)CH_2CH_2$, $CH_2CH_2CH_2CH(CH_3)CH_2$, $CH_2CH_2CH_2CH_2CH(CH_3)$, and $CH_2CH_2CH_2CH_2CH_2CH_2$; more preferably $CH_2$, $CH(CH_3)$, $CH_2CH_2$, $CH_2CH(CH_3)$ and $CH(CH_3)CH_2$, most preferably $CH_2$ and $CH(CH_3)$, and in particular $CH_2$. Preferably, $C_{1-6}$-alkylene is selected from $C_{1-4}$-alkylene.

According to the invention, the terms "$C_{3-10}$-cycloalkyl" and "$C_{3-6}$-cycloalkyl" preferably mean monocyclic aliphatic hydrocarbons containing 3, 4, 5, 6, 7, 8, 9 or 10 carbon atoms as ring members and 3, 4, 5 or 6 carbon atoms as ring members, respectively, wherein the hydrocarbons in each case can be saturated or unsaturated (but not aromatic), unsubstituted or mono- or polysubstituted.

Preferably, $C_{3-10}$-cycloalkyl and $C_{3-6}$-cycloalkyl are saturated. The $C_{3-10}$-cycloalkyl and $C_{3-6}$-cycloalkyl can be bound to the respective superordinate general structure via any desired and possible ring member of the cycloalkyl group. The $C_{3-10}$-cycloalkyl and $C_{3-6}$-cycloalkyl are not condensed with further ring systems and are not bridged.

Preferred $C_{3-10}$-cycloalkyl groups are selected from the group consisting of cyclopropyl, cyclobutyl, cyclopentyl, cyclohexyl, cycloheptyl, cyclooctyl, cyclononyl, and cyclodecyl. Preferred $C_{3-10}$-cycloalkyl groups are selected from $C_{3-6}$-cycloalkyl groups.

According to the invention, the term "4 to 11-membered bicycloalkyl" preferably means bicyclic aliphatic hydrocarbons containing 4, 5, 6, 7, 8, 9, 10 or 11 carbon atoms as ring members, wherein the hydrocarbons in each case can be saturated or unsaturated (but not aromatic), unsubstituted or mono- or polysubstituted. The bicyclic system may share a common bond (annealed rings) or may be bridged.

Preferably, 4 to 11-membered bicycloalkyl is saturated. The 4 to 11-membered bicycloalkyl can be bound to the respective superordinate general structure via any desired and possible ring member of the 4 to 11-membered bicycloalkyl group.

Preferred 4 to 11-membered bicycloalkyl groups are selected from the group consisting of bicycle[1.1.0]butyl, bicylo[1.1.1]pentyl, bicyclo[2.1.0]pentyl, bicyclo[3.1.0]hexyl, bicyclo[2.2.0]hexyl, bicyclo[2.1.1]hexyl, bicyclo[4.1.0]heptyl, bicyclo[2.2.1]heptyl, and bicyclo[3.3.0]octyl.

According to the invention, the term "5 to 11-membered spiroalkyl or dispiroalkyl" means spirocyclic or dispirocyclic aliphatic hydrocarbons containing 5, 6, 7, 8, 9, 10 or 11 carbon atoms as ring members, wherein the hydrocarbons in each case can be saturated or unsaturated (but not aromatic), unsubstituted or mono- or polysubstituted. The spirocyclic system shares a common carbon atom connecting the rings. In case of a dispirocyclic system, there are three rings, whereas two of these three rings share a common carbon atom connecting these rings.

Preferably, 5 to 11-membered spiroalkyl or dispiroalkyl is saturated. The 5 to 11-membered spiroalkyl or dispiroalkyl can be bound to the respective superordinate general structure via any desired and possible ring member of the 5 to 11-membered spiroalkyl or dispiroalkyl group.

Preferred 5 to 11-membered spiroalkyl or dispiroalkyl groups are selected from the group consisting of spiro[2.2]pentyl, spiro[2.3]hexyl, spiro[3.3]heptyl, and dispiro[2.0.2.1]heptyl.

According to the invention, the terms "4 to 10-membered heterocycloalkyl" and "4 to 6-membered heterocycloalkyl" preferably mean monocyclic, heterocycloaliphatic saturated or unsaturated (but not aromatic) residues having 4 to 10, i.e. 4, 5, 6, 7, 8, 9 or 10 ring members and 4 to 6, i.e. 4, 5 or 6 ring members, respectively, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. Preferably, the 4 to

10-membered heterocycloalkyl and the 4 to 6-membered heterocycloalkyl contain only one heteroatom or heteroatom group within the ring.

Preferably, 4 to 10-membered heterocycloalkyl and 4 to 6-membered heterocycloalkyl are saturated. The 4 to 10-membered heterocycloalkyl and 4 to 6-membered heterocycloalkyl are not condensed with further ring systems and are not bridged.

The 4 to 10-membered heterocycloalkyl and the 4 to 6-membered heterocycloalkyl group can be bound to the superordinate general structure via any desired and possible ring member of the heterocycloaliphatic residue if not indicated otherwise. In a preferred embodiment, 4 to 10-membered heterocycloalkyl and 4 to 6-membered heterocycloalkyl are bound to the superordinate general structure via a carbon atom.

Preferred 4 to 10-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

Preferred 4 to 6-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, N-methylpyridinonyl, pyrazolidinyl, and pyranyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

According to the invention, the term "5 to 11-membered heterobicycloalkyl" preferably means bicyclic heteroaliphatic saturated or unsaturated (but not aromatic) hydrocarbons containing 5, 6, 7, 8, 9, 10 or 11 ring members, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. The bicyclic system may share a common bond (annealed rings) or may be bridged.

Preferably, 5 to 11-membered heterobicycloalkyl is saturated. The 5 to 11-membered heterobicycloalkyl can be bound to the respective superordinate general structure via any desired and possible ring member of the 5 to 11-membered heterobicycloalkyl group.

A preferred 5 to 11-membered heterobicycloalkyl group is 2-oxa-bicyclo[2.1.1]hexyl.

According to the invention, the term "5- to 10-membered heteroaryl" and "5- or 6-membered heteroaryl", respectively, preferably means a 5 to 10-membered or 5- or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5- to 10-membered heteroaryl and 5- or 6-membered heteroaryl is bound to the suprordinate general structure via a carbon atom of the heterocycle. The heteroaryl can also be condensed with a further ring system, e.g. saturated or (partially) unsaturated (hetero)cyclic, aromatic or heteroaromatic ring systems, which can in turn be unsubstituted or mono- or polysubstituted, if not indicated otherwise.

Preferably, the 5- to 10-membered heteroaryl is selected from the group consisting of pyridyl (i.e. 2-pyridyl, 3-pyridyl, 4-pyridyl), pyrazolyl, pyrrolo[2,3-b]pyridyl, pyridonyl (pyridinonyl), thienyl (thiophenyl), thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, thiadiazolyl, N-methylpyridinonyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, indolyl, isoquinolinyl, naphthyridinyl, phenazinyl, phenothiazinyl, phthalazinyl, purinyl, phenazinyl, tetrazolyl and triazinyl; more preferably pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, and thiadiazolyl. Particularly preferably, 5 to 10-membered heteroaryl is selected

from 5- or 6-membered heteroaryl.

Preferably, the 5- or 6-membered heteroaryl is selected from the group consisting of pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, thiadiazolyl, N-methylpyridinonyl, tetrazolyl and triazinyl; more preferably pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, and thiadiazolyl.

As pyridones and 2-hydroxypyridine are tautomers, for the purpose of the specification the definition of pyridines that may optionally be substituted with OH covers pyridones.

In connection with the terms "$C_{1-6}$-alkyl", "$C_{1-4}$-alkyl", "$C_{1-6}$-alkylene", "$C_{1-4}$-alkylene", "$C_{3-10}$-cycloalkyl", "$C_{3-6}$-cycloalkyl", "4 to 11-membered bicycloalkyl", "5 to 11-membered spiroalkyl or dispiroalkyl", "4 to 10-membered heterocycloalkyl", "4 to 6-membered heterocycloalkyl", and "5 to 11-membered heterobicycloalkyl", the term "substituted" refers in the sense of the invention, with respect to the corresponding residues or groups, to the single substitution (monosubstitution) or multiple substitution (polysubstitution), e.g. disubstitution, trisubstitution or tetrasubstitution; more preferably to monosubstitution, disubstitution or trisubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of $CF_3$, $CH_2CF_3$ or disubstituted as in the case of 1,1-difluorocyclohexyl, or at various points, as in the case of 1-chloro-3-fluoro-cyclohexyl. The multiple substitution can be carried out using the same or using different substituents.

If a residue occurs multiply within a molecule, then this residue can have respectively different meanings for various substituents: if, for example, both **R4** and **R5** denote $C_{1-6}$-alkyl, then $C_{1-6}$-alkyl can e.g. represent methyl for **R4** and can represent 2-propyl for **R5**.

According to the invention, $C_{1-6}$-alkyl, $C_{1-4}$-alkyl, $C_{1-6}$-alkylene, $C_{1-4}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl, and 5 to 11-membered heterobicycloalkyl in each case independently from one another are unsubstituted or mono- or polysubstituted, more preferably unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$; preferably $CH_3$, $CF_3$, $CF_2H$, $CFH_2$, and $OCH_3$; more preferably $CF_3$, $CF_2H$, and $CFH_2$; and most preferably $CF_3$.

Preferably, $C_{1-6}$-alkylene groups are unsubstituted.

According to the invention, phenyl, 5 to 10-membered heteroaryl and 5 or 6-membered heteroaryl in each case independently from one another are unsubstituted or mono- or polysubstituted, more preferably unsubstituted or mono- or disubstituted, with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl; preferably F, $CH_3$, $CF_3$, $CF_2H$, $CFH_2$, and $OCH_3$; more preferably F, $CF_3$, $CF_2H$, and $CFH_2$; and most preferably F.

According to the invention, **L** represents $CH_2$, $CH(CH_3)$ or $CH(CH_2CH_3)$.

In preferred embodiments, **L** represents $CH_2$.

According to the invention, **R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl; with the proviso that when **R1** represents $C_{3-10}$-cycloalkyl or 4 to 10-membered heterocycloalkyl, said $C_{3-10}$-cycloalkyl or 4 to 10-membered heterocycloalkyl is substituted with at least two substituients, wherein said at least two substituents are independently from one another selected from $C_{1-6}$-alkyl, $CF_3$, $CF_2H$, and $CFH_2$.

In preferred embodiments, **R1** represents $C_{3-10}$-cycloalkyl, substituted with two, three, four, or more substituents independently from one another selected from F, $CH_3$, $OCH_3$, CN, $CHF_2$ and $CF_3$; provided that at least two substituents are independently from one another selected from $CH_3$, $CHF_2$ and $CF_3$.

Preferably, **R1** is selected from dimethyl cyclopropyl, trifluoromethyl methyl cyclopropyl, dimethyl difluoro cyclopropyl, dimethyl cyclobutyl, trifluoromethyl cyclobutyl, trifluoromethyl methyl cyclobutyl, dimethyl difluoro cyclobutyl, dimethyl cyclopentyl, trifluoromethyl cyclopentyl, trifluoromethyl methyl cyclopentyl, dimethyl difluoro cyclopentyl, dimethyl cyclohexyl, trifluoromethyl cyclohexyl, trifluoromethyl methyl cyclohexyl, dimethyl difluoro cyclohexyl, dimethyl cycloheptyl, trifluoromethyl cycloheptyl, trifluoromethyl methyl cycloheptyl, dimethyl difluoro cycloheptyl, dimethyl cyclooctyl, trifluoromethyl cyclooctyl, trifluoromethyl methyl cyclooctyl, dimethyl difluoro cyclooctyl, dimethyl cyclononyl, trifluoromethyl cyclononyl, trifluoromethyl methyl cyclononyl, dimethyl difluoro cyclononyl, dimethyl cyclodecyl, trifluoromethyl cyclodecyl, trifluoromethyl methyl cyclodecyl, and dimethyl difluoro cyclodecyl.

In preferred embodiments, **R1** represents $C_{3-10}$-cycloalkyl, substituted with at least two substituents independently from one another selected from $CH_3$, $CHF_2$ and $CF_3$ and optionally substituted with one, two, three, four, or more substituents independently from one another selected from F, $OCH_3$, and CN, wherein the carbon atom of $C_{3-10}$-cycloalkyl,

which is linked to **L**, is substituted with $CH_3$.

More preferably, **R1** is selected from dimethyl cyclopropyl, trifluoromethyl methyl cyclopropyl, trifluoromethyl methyl cyclobutyl, and dimethyl difluoro cyclopentyl.

In further preferred embodiments, **R1** represents 4 to 11-membered bicycloalkyl, unsubstituted or substituted with one, two, three, four, or more substituents independently from one another selected from F, $CH_3$, $OCH_3$, CN, $CHF_2$ and $CF_3$.

Preferably, **R1** is selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethyl bicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluorobicyclo[2.1.0]pentyl, bicyclo[3.1.0]hexyl, methyl bicyclo[3.1.0]hexyl, trifluoromethyl bicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1]hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo [3.3.0]octyl, difluoro bicyclo [3.3.0]octyl, methyl difluoro bicyclo[3.3.0]octyl, and trifluoromethyl difluoro bicyclo[3.3.0]octyl.

More preferably, **R1** is selected from bicyclo[1.1.1]pentyl, fluoro bicylo[1.1.1]pentyl, methoxy bicylo[1.1.1]pentyl, difluoromethyl bicylo[1.1.1]pentyl, trifluoromethyl bicylo[1.1.1]pentyl, bicyclo[2.1.0]pentyl, difluoro bicyclo [2.1.0]pentyl, difluoro bicyclo[3.1.0]hexyl, methyl difluoro bicyclo[3.1.0]hexyl, trifluoromethyl difluoro bicyclo[3.1.0]hexyl, methyl bicyclo[2.2.0]hexyl, bicyclo[2.1.1]hexyl, fluoro bicyclo[2.1.1]hexyl, difluoro bicyclo[2.1.1]hexyl, difluoro bicyclo[4.1.0]heptyl, fluoro bicyclo[2.2.1]heptyl, bicyclo [3.3.0]octyl, or fluoro bicyclo [3.3.0]octyl.

In other preferred embodiments, **R1** represents 5 to 11-membered spiroalkyl or dispiroalkyl, unsubstituted or substituted with one, two, three, four, or more substituents independently from one another selected from F, $CH_3$, $OCH_3$, CN, $CHF_2$ and $CF_3$.

Preferably, **R1** is selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.0.2.1]heptyl.

More preferably, **R1** is selected from spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, or dispiro[2.0.2.1]heptyl.

In still further preferred embodiments, **R1** represents 4 to 10-membered heterocycloalkyl, substituted with two, three, four, or more substituents independently from one another selected from F, $CH_3$, $OCH_3$, CN, $CHF_2$ and $CF_3$; provided that at least two substituents are independently from one another selected from $CH_3$, $CHF_2$ and $CF_3$.

Preferably, **R1** is selected from oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl; in each case substituted with at least two substituients, wherein said at least two substituents are independently from one another selected from $C_{1-6}$-alkyl, $CF_3$, $CF_2H$, and $CFH_2$.

More preferably, **R1** represents dimethyl oxetanyl.

In yet further preferred embodiments, **R1** represents 5 to 11-membered heterobicycloalkyl, unsubstituted or substituted with one, two, three, four, or more substituents independently from one another selected from F, $CH_3$, $OCH_3$, CN, $CHF_2$ and $CF_3$.

Preferably, **R1** represents 2-oxa-bicyclo[2.1.1]hexyl or methyl 2-oxa-bicyclo[2.1.1]hexyl.

According to the invention, **R2** represents H or $C_{1-6}$-alkyl.

In preferred embodiments, **R2** represents H.

According to the invention, **X** represents phenyl, or 5 to 10-membered heteroaryl.

In preferred embodiments, **X** represents phenyl, wherein phenyl is unsubstituted or mono- or polysubstituted with one or more substituents selected from the group consisting of F, Cl, Br, CN, $CH_3$, $CF_3$, $CHF_2$, $CH_2F$, OH, $OCH_3$,

$OCF_3$, $OCF_2H$, and $OCFH_2$; preferably F.

In further preferred embodiments, **X** represents 5 to 10-membered heteroaryl selected from the group consisting of pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, and thiadiazolyl; preferably pyrazolyl or pyridyl; wherein said 5 to 10-membered heteroaryl is unsubstituted or mono- or polysubstituted with one or more substituents selected from the group consisting of F, Cl, Br, CN, $CH_3$, $CF_3$, $CHF_2$, $CH_2F$, OH, $OCH_3$, $OCF_3$, $OCF_2H$, and $OCFH_2$; preferably F.

According to the invention, **R3** represents $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)-N($C_{1-6}$-alkyl)$_2$, $S(O)_2$-$NH_2$, $S(O)_2$-N(H)($C_{1-6}$-alkyl), $S(O)_2$-N(H)($C_{3-6}$-cycloalkyl), $S(O)_2$-N($C_{1-6}$-alkyl)$_2$, C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), $OCF_3$, $OCF_2H$, CN, OH, O-$C_{3-6}$-cycloalkyl, O-(4 to 6-membered heterocycloalkyl), S(O)-$C_{1-6}$-alkyl, S(O)-($C_{3-6}$-cycloalkyl), S(O)-(4 to 6-membered heterocycloalkyl), S(O)-phenyl, or S(O)-(5 or 6-membered heteroaryl).

In preferred embodiments, **R3** represents $S(O)_2$-$C_{1-6}$-alkyl, $S(O)_2$-$NH_2$, or C(O)-$NH_2$.

According to the invention, **R4** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 11-membered bicycloalkyl, 5 to 11-membered spiroalkyl or bisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl).

In preferred embodiments, **R4** represents H.

In further preferred embodiments, **R4** represents $C_{1-6}$-alkyl.

Preferably, R4 represents $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, or $CH(CH_3)_2$.

More preferably, **R4** represents $CF_2CH_3$, $CH_3$ or $CF_3$.

In other preferred embodiments, **R4** represents $C_{3-10}$-cycloalkyl, unsubstituted or substituted with one, two, three, four, or more substituents independently from one another selected from F, $CH_3$, $OCH_3$, CN, $CHF_2$ and $CF_3$.

Preferably, **R4** is selected from cyclopropyl, methyl cyclopropyl, difluoromethyl cyclopropyl, trifluoromethyl cyclopropyl, cyano cyclopropyl, methoxy cyclopropyl, fluoro cyclopropyl, difluoro cyclopropyl, trifluoro cyclopropyl, cyclobutyl, methyl cyclobutyl, difluoromethyl cyclobutyl, trifluoromethyl cyclobutyl, cyano cyclobutyl, methoxy cyclobutyl, fluoro cyclobutyl, difluoro cyclobutyl, trifluoro cyclobutyl, cyclopentyl, methyl cyclopentyl, difluoromethyl cyclopentyl, trifluoromethyl cyclopentyl, cyano cyclopentyl, methoxy cyclopentyl, fluoro cyclopentyl, difluoro cyclopentyl, trifluoro cyclopentyl, cyclohexyl, methyl cyclohexyl, difluoromethyl cyclohexyl, trifluoromethyl cyclohexyl, cyano cyclohexyl, methoxy cyclohexyl, fluoro cyclohexyl, difluoro cyclohexyl, and trifluoro cyclohexyl.

More preferably, **R4** represents cyclopropyl.

In still further preferred embodiments, **R4** represents 4 to 11-membered bicycloalkyl, unsubstituted or substituted with one, two, three, four, or more substituents independently from one another selected from F, $CH_3$, $OCH_3$, CN, $CHF_2$ and $CF_3$.

Preferably, **R4** represents bicyclo[1.1.1]pentyl.

In yet further preferred embodiments, **R4** represents 5 to 11-membered spiroalkyl, unsubstituted or substituted with one, two, three, four, or more substituents independently from one another selected from F, $CH_3$, $OCH_3$, CN, $CHF_2$ and $CF_3$.

Preferably, **R4** represents spiro[2.2]pentyl or spiro[2.3]hexyl.

In other preferred embodiments, **R4** represents O-$C_{1-6}$-alkyl, unsubstituted or substituted with one, two, three, four, or more F.

Preferably, **R4** is selected from O-$CHF_2$, O-$CH_2F$, O-$CF_3$, O-$CH_2CHF_2$, O-$CH_2CH_2F$, O-$CH_2CF_3$, O-$CF_2CH_3$, O-$CHFCH_3$, O-$CF_2CF_3$, O-$CHFCF_3$, O-$CH_3$, O-$CH_2CH_3$, or O-$CH(CH_3)_2$.

According to the invention, **R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl).

In preferred embodiments, **R5** represents $C_{1-6}$-alkyl or Cl; preferably $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$ or Cl.

Preferably, **R5** represents $CF_3$ or $CH_3$.

In a particularly preferred embodiment, the compound according to the invention is selected from the group consisting of

| Ex. | Name |
|---|---|
| 1 | 4-(1-((3 -fluorobicyclo[1.1.1]pentan-1-yl)methyl)-3-methyl-4-(trifluoromethyl)-1H-pyrazole-5-carboxamido)picolinamide |
| 2 | 4-(3-(1,1-difluoroethyl)-1-((3 -fluorobicyclo [1.1.1]pentan-1-yl)methyl)-4-methyl-1H-pyrazole-5-carboxamido)picolinamide |
| 3 | 1-((2-oxabicyclo[2.1.1]hexan-1-yl)methyl)-3-cyclopropyl-4-(trifluoromethyl)-1H-pyrazole-5-carboxamide |
| 4 | 3-(1,1- 4-(3-(1,1-difluoroethyl)-4-methyl-1-((3-(trifluoromethyl)bicyclo[1.1.1]pentan-1yl)methyl)-1H-pyrazole-5-carboxamido)picolinamide |
| 5 | 4-(3-cyclopropyl-1-((1-methyl-2-oxabicyclo[2.1.1]hexan-4-yl)methyl)-4-(trifluoromethyl)-1H-pyrazole-5-carboxamido)picolinamide |
| 6 | 4-(1-((3-methoxybicyclo[1.1.1]pentan-1-yl)methyl)-3-methyl-4-(trifluoromethyl)-1H-pyrazole-5-carboxamido)picolinamide |
| 7 | 4-(1-((3 -(difluoromethyl)bicyclo [1.1.1]pentan-1-yl)methyl)-3-methyl-4-(trifluoromethyl)-1H-pyrazole-5-carboxamido)picolinamide |
| 8 | 4-(4-methyl-3-(trifluoromethyl)-1-((3 -(trifluoromethyl)bicyclo [1.1.1]pentan-1-yl)methyl)-1H-pyrazole-5-carboxamido)picolinamide |
| 9 | 4-(3-cyclopropyl-4-(trifluoromethyl)-1-((3 -(trifluoromethyl)bicyclo [1.1.1]pentan-1-yl)methyl)-1H-pyrazole-5-carboxamido)picolinamide |
| 10 | 4-(1-((4,4-difluoro-1,2-dimethylcyclopentyl)methyl)-3-(1,1-difluoroethyl)-4-methyl-1H-pyrazole-5-carboxamido)picolinamide |
| 14 | 4-(3-cyclopropyl-1-((3-methoxybicyclo[1.1.1]pentan-1-yl)methyl)-4-(trifluoromethyl)-1H-pyrazole-5-carboxamido)picolinamide |
| 15 | 4-(3-cyclopropyl-1-((3 -fluorobicyclo [1.1.1]pentan-1-yl)methyl)-4-(trifluoromethyl)-1H-pyrazole-5-carboxamido)picolinamide |
| 16 | 4-(3-cyclopropyl-1-((4-methyl-2-oxabicyclo[2.1.1]hexan-1-yl)methyl)-4-(trifluoromethyl)-1H-pyrazole-5-carboxamido)picolinamide |
| 17 | 4-(3-cyclopropyl-1-((4,4-dimethyloxetan-2-yl)methyl)-4-(trifluoromethyl)-1H-pyrazole-5-carboxamido)-picolinamide |
| 18 | 4-(3-methyl-4-(trifluoromethyl)-1-((3 -(trifluoromethyl)bicyclo [1.1.1]pentan-1-yl)methyl)-1H-pyrazole-5-carboxamido)picolinamide |
| 19 | 3-cyclopropyl-1-((4-methyl-2-oxabicyclo[2.1.1]hexan-1-yl)methyl)-N-(2-(methylsulfonyl)pyridin-4-yl)-4-(trifluoromethyl)-1H-pyrazole-5-carboxamide |
| 20 | 3-cyclopropyl-1-((3 -fluorobicyclo [1.1.1]pentan-1-yl)methyl)-N-(2-(methylsulfonyl)pyridin-4-yl)-4-(trifluoromethyl)-1H-pyrazole-5-carboxamide |
| 21 | 3-cyclopropyl-1-((3 -fluorobicyclo [1.1.1]pentan-1-yl)methyl)-N-(2-(methylsulfonyl)pyridin-4-yl)-4-(trifluoromethyl)-1H-pyrazole-5-carboxamide |
| 22 | 3 -cyclopropyl-1-((4,4-dimethyloxetan-2-yl)methyl)-N-(2-(methylsulfonyl)pyridin-4-yl)-4-(trifluoromethyl)-1H-pyrazole-5-carboxamide |
| 23 | 3-cyclopropyl-1-((1-methyl-2-oxabicyclo[2.1.1]hexan-4-yl)methyl)-N-(2-(methylsulfonyl)pyridin-4-yl)-4-(trifluoromethyl)-1H-pyrazole-5-carboxamide |
| 24 | 1-((2-oxabicyclo[2.1.1]hexan-1-yl)methyl)-3-cyclopropyl-N-(2-(methylsulfonyl)pyridin-4-yl)-4-(trifluoromethyl)-1H-pyrazole-5-carboxamide |
| 25 | 3 -(1,1-difluoroethyl)-4-methyl-N-(2-(methylsulfonyl)pyridin-4-yl)-1-((3 -(trifluoromethyl)bicyclo-[1.1.1]pentan-1yl)methyl)-1H-pyrazole-5-carboxamide |
| 26 | 3-methyl-N-(2-(methylsulfonyl)pyridin-4-yl)-4-(trifluoromethyl)-1-((3 -(trifluoromethyl)bicyclo-[1.1.1]pentan-1yl)methyl)-1H-pyrazole-5-carboxamide |
| 27 | 4-methyl-N-(2-(methylsulfonyl)pyridin-4-yl)-3-(trifluoromethyl)-1-((3 -(trifluoromethyl)bicyclo-[1.1.1]pentan-1yl)methyl)-1H-pyrazole-5-carboxamide |
| 28 | 3-cyclopropyl-N-(2-(methylsulfonyl)pyridin-4-yl)-4-(trifluoromethyl)-1-((3-(trifluoromethyl)bicyclo-[1.1.1]pentan-1yl)methyl)-1H-pyrazole-5-carboxamide |

(continued)

| | |
|---|---|
| 29 | 3-(1,1-difluoroethyl)-1-((3-fluorobicyclo[1.1.1]pentan-1-yl)methyl)-4-methyl-N-(2-(methylsulfonyl)-pyridin-4-yl)-1H-pyrazole-5-carboxamide |
| 30 | 1-((2-oxabicyclo[2.1.1]hexan-1-yl)methyl)-N-(3-sulfamoylphenyl)-4-(trifluoromethyl)-1H-pyrazole-5-carboxamide |
| 31 | 1-((1-methyl-2-oxabicyclo[2.1.1]hexan-4-yl)methyl)-N-(3-sulfamoylphenyl)-4-(trifluoromethyl)-1H-pyrazole-5-carboxamide |
| 32 | 1-(bicyclo [1.1.1]pentan-1-ylmethyl)-N-(3-sulfamoylphenyl)-4-(trifluoromethyl)-1H-pyrazole-5-carboxamide |
| 33 | 1-((2-oxabicyclo [2.1.1]hexan-1-yl)methyl)-3-methyl-N-(3-sulfamoylphenyl)-4-(trifluoromethyl)-1H-pyrazole-5-carboxamide |
| 34 | 1-(spiro[2.3]hexan-5-ylmethyl)-N-(3-sulfamoylphenyl)-4-(trifluoromethyl)-1H-pyrazole-5-carboxamide |
| 35 | 1-(bicyclo[2.1.1]hexan-1-ylmethyl)-N-(3-sulfamoylphenyl)-4-(trifluoromethyl)-1H-pyrazole-5-carboxamide |
| 36 | 1-(bicyclo[2.1.1]hexan-1-ylmethyl)-N-(3-carbamoyl-4-fluorophenyl)-3-methyl-4-(trifluoromethyl)-1H-pyrazole-5-carboxamide |
| 37 | 1-((2-oxabicyclo[2.1.1]hexan-1-yl)methyl)-N-(3-carbamoyl-4-fluorophenyl)-4-(trifluoromethyl)-1H-pyrazole-5-carboxamide |
| 38 | 1-(bicyclo[1.1.1]pentan-1-ylmethyl)-N-(3 -carbamoyl-4-fluorophenyl)-4-(trifluoromethyl)-1H-pyrazole-5-carboxamide |
| 39 | 1-((2-oxabicyclo[2.1.1]hexan-1-yl)methyl)-N-(3-carbamoyl-4-fluorophenyl)-3-methyl-4-(trifluoromethyl)-1H-pyrazole-5-carboxamide |
| 40 | 1-(bicyclo[1.1.1]pentan-1-ylmethyl)-N-(3 -carbamoyl-4-fluorophenyl)-3 -methyl-4-(trifluoromethyl)-1H-pyrazole-5-carboxamide |
| 41 | N-(3-carbamoyl-4-fluorophenyl)-1-(spiro[2.3]hexan-5-ylmethyl)-4-(trifluoromethyl)-1H-pyrazole-5-carboxamide |
| 42 | 1-(bicyclo[2.1.1]hexan-1-ylmethyl)-N-(3-carbamoyl-4-fluorophenyl)-4-(trifluoromethyl)-1H-pyrazole-5-carboxamide |
| 43 | 1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide |
| 47 | 1-(bicyclo [1.1.1]pentan-1-ylmethyl)-N-(1-(methylsulfonyl)-1H-pyrazol-4-yl)-4-(trifluoromethyl)-1H-pyrazole-5-carboxamide |
| 48 | N-(1-(methylsulfonyl)-1H-pyrazol-4-yl)-1-(spiro[2.3]hexan-5-ylmethyl)-4-(trifluoromethyl)-1H-pyrazole-5-carboxamide |
| 49 | 1-(bicyclo[2.1.1]hexan-1ylmethyl)-N-(1-(methylsulfonyl)-1H-pyrazol-4-yl)-4-(trifluoromethyl)-1H-pyrazole-5 -carboxamide |
| 50 | 3-cyclopropyl-1-(spiro[22]pentan-1-ylmethyl)-N-(2-sulfamoylpyridin-4-yl)-4-(trifluoromethyl)-1H-pyrazole-5-carboxamide |
| 51 | 3-cyclopropyl-1-((2,2-difluorospiro[22]pentan-1-yl)methyl)-N-(2-sulfamoylpyridin-4-yl)-4-(trifluoromethyl)-1H-pyrazole-5-carboxamide |
| 52 | 3-cyclopropyl-1-(dispiro[2.0$2^4$.1$^3$]heptan-7-ylmethyl)-N-(2-sulfamoylpyridin-4-yl)-4-(trifluoromethyl)-1H-pyrazole-5-carboxamide |
| 53 | 3 -cyclopropyl-1 -((1,2-dimethylcyclopropyl)methyl)-N-(2-sulfamoylpyridin-4-yl)-4-(trifluoromethyl)-1H-pyrazole-5-carboxamide |
| 54 | 3-cyclopropyl-1-((1-methyl-2-(trifluoromethyl)cyclopropyl)methyl)-N-(2-sulfamoylpyridin-4 yl)-4-(trifluoromethyl)-1H-pyrazole-5-carboxamide |
| 55 | 1-(bicyclo [2.1.0]pentan-1-ylmethyl)-3-cyclopropyl-N-(2-sulfamoylpyridin-4-yl)-4-(trifluoromethyl)-1H-pyrazole-5-carboxamide |
| 56 | 3-cyclopropyl-1-(spiro[2.3]hexan-5-ylmethyl)-N-(2-sulfamoylpyridin-4-yl)-4-(trifluoromethyl)-1H-pyrazole-5-carboxamide |
| 57 | 3-cyclopropyl-1-((5-methyspiro[2.3]hexan-5-yl)methyl)-N-(2-sulfamoylpyridin-4-yl)-4-(trifluorome- |

(continued)

| | |
|---|---|
| | thyl)-1H-pyrazole-5-carboxamide |
| 58 | 3-cyclopropyl-1-((1-methyl-3-(trifluoromethyl)cyclobutyl)methyl)-N-(2-sulfamoylpyridin-4-yl)-4-(trifluoromethyl)-1H-pyrazole-5-carboxamide |
| 59 | 3-cyclopropyl-1-((1-fluorospiro[2.3]hexan-5-yl)methyl)-N-(2-sulfamoylpyridin-4-yl)-4-(trifluoromethyl)-1H-pyrazole-5-carboxamide |
| 60 | 3-cyclopropyl-1-((5,5-difluorobicyclo[2.1.0]pentan-2-yl)methyl)-N-(2-sulfamoylpyridin-4-yl)-4-(trifluoromethyl)-1H-pyrazole-5-carboxamide |
| 61 | 3-cyclopropyl-1-(spiro[2.3]hexan-4-ylmethyl)-N-(2-sulfamoylpyridin-4-yl)-4-(trifluoromethyl)-1H-pyrazole-5-carboxamide |
| 62 | 3-cyclopropyl-1-((6,6-difluoro-4-methylspiro[2.3]hexan-4-yl)methyl)-N-(2-sulfamoylpyridin-4-yl)-4-(trifluoromethyl)-1H-pyrazole-5-carboxamide |
| 63 | 3-cyclopropyl-1-(spiro[3.3]heptan-1-ylmethyl)-N-(2-sulfamoylpyridin-4-yl)-4-(trifluoromethyl)-1H-pyrazole-5-carboxamide |
| 64 | 3-cyclopropyl-1-((2-methylbicyclo[22.0]hexan-2-yl)methyl)-N-(2-sulfamoylpyridin-4-yl)-4-(trifluoromethyl)-1H-pyrazole-5-carboxamide |
| 65 | 3 -cyclopropyl-1-((3,3 -difluorobicyclo [3. 1.0] hexan-1-yl)methyl)-N-(2-sulfamoylpyridin-4-yl)-4-(trifluoromethyl)-1H-pyrazole-5-carboxamide |
| 66 | 3-cyclopropyl-1-((4,4-difluorobicyclo[3.1.0] hexan-1-yl)methyl)-N-(2-sulfamoylpyridin-4-yl)-4-(trifluoromethyl)-1H-pyrazole-5-carboxamide |
| 67 | 3-cyclopropyl-1-((5-fluorooctahydropentalen-2-yl)methyl)-N-(2-sulfamoylpyridin-4-yl)-4-(trifluoromethyl)-1H-pyrazole-5-carboxamide |
| 68 | 3-cyclopropyl-1-((4,4-difluoro-1,2-dimethylcyclopentyl)methyl)-N-(2-sulfamoylpyridin-4-yl)-4-(trifluoromethyl)-1H-pyrazole-5-carboxamide |
| 69 | 3-cyclopropyl-1-((3,3-difluoro-5-methylbicyclo[3.1.0]hexan-1-yl)methyl)-N-(2-sulfamoylpyridin-4-yl)-4-(trifluoromethyl)-1H-pyrazole-5-carboxamide |
| 70 | 3-cyclopropyl-1-((3,3-difluoro-5-(trifluoromethyl)bicyclo[3.1.0]hexan-1-yl)methyl)-N-(2-sulfamoylpyridin-4-yl)-4-(trifluoromethyl)-1H-pyrazole-5-carboxamide |
| 71 | 3-cyclopropyl-1-((2,2-difluorobicyclo[2.1.1]hexan-1-yl)methyl)-N-(2-sulfamoylpyridin-4-yl)-4-(trifluoromethyl)-1H-pyrazole-5-carboxamide |
| 72 | 3-cyclopropyl-1-((3,3 -difluorobicyclo [2.1.1] hexan-1-yl)methyl)-N-(2-sulfamoylpyridin-4-yl)-4-(trifluoromethyl)-1H-pyrazole-5-carboxamide |
| 73 | 3-cyclopropyl-1-((4-fluorobicyclo[2.1.1]hexan-1-yl)methyl)-N-(2-sulfamoylpyridin-4-yl)-4-(trifluoromethyl)-1H-pyrazole-5-carboxamide |
| 74 | 3-cyclopropyl-1-((4-fluorobicyclo[2.1.1]hexan-1-yl)methyl)-N-(2-sulfamoylpyridin-4-yl)-4-(trifluoromethyl)-1H-pyrazole-5-carboxamide |
| 75 | 3-cyclopropyl-1-((4,4-difluorobicyclo[4.1.0]heptan-1-yl)methyl)-N-(2-sulfamoylpyridin-4-yl)-4-(trifluoromethyl)-1H-pyrazole-5-carboxamide |
| 76 | 3-(1,1-difluoroethyl)-4-methyl-1-(spiro[2.2]pentan-1ylmethyl)-N-(2-sulfamoylpyridin-4-yl)-1H-pyrazole-5-carboxamide |
| 77 | 3-(1,1-difluoroethyl)-1-((2,2-difluorospiro[2.2]pentan-1-yl)methyl)-4-methyl-N-(2-sulfamoylpyridin-4-yl)-1H-pyrazole-5-carboxamide |
| 78 | 3-(1,1-difluoroethyl)-1-(dispiro[2.0.$2^4$.$1^3$]heptan-7-ylmethyl)-4-methyl-N-(2-sulfamoylpyridin-4-yl)-1H-pyrazole-5-carboxamide |
| 79 | 3-(1,1 -difluoroethyl)-1 -((1,2-dimethylcyclopropyl)methyl)-4-methyl-N-(2-sulfamoylpyridin-4-yl)- 1H-pyrazole-5-carboxamide |
| 80 | 3-(1,1-difluoroethyl)-4-methyl-1-((1-methyl-2-(trifluoromethyl)cyclopropyl)methyl)-N-(2-sulfamoylpyridin-4-yl)-1H-pyrazole-5-carboxamide |
| 81 | 1-(bicyclo [2.1.0]pentan-1-ylmethyl)-3-(1,1-difluoroethyl)-4-methyl-N-(2-sulfamoylpyridin-4-yl)-1H-pyrazole-5-carboxamide |
| 82 | 3-(1,1-difluoroethyl)-4-methyl-1-(spiro[2.3]hexan-5ylmethyl)-N-(2-sulfamoylpyridin-4-yl)-1H-pyrazole-5-carboxamide |

(continued)

| | |
|---|---|
| 83 | 3-(1,1-difluoroethyl)-4-methyl-1-((5-methylspiro[2.3]hexan-5-yl)methyl)-N-(2-sulfamoylpyridin-4-yl)-1H-pyrazole-5-carboxamide |
| 84 | 3-(1,1 -difluoroethyl)-4-methyl-1 -((1 -methyl-3 -(trifluoromethyl)cyclobutyl)methyl)-N-(2-sulfamoylpyridin-4-yl)-1H-pyrazole-5-carboxamide |
| 85 | 3-(1,1-difluoroethyl)-1-((1-fluorospiro[2.3]hexan-5-yl)methyl)-4-methyl-N-(2-sulfamoylpyridin-4-yl)-1H-pyrazole-5-carboxamide |
| 86 | 1-((5,5-difluorobicyclo[2.1.0]pentan-2-yl)methyl)-3-(1,1-difluoroethyl)-4-methyl-N-(2-sulfamoylpyridin-4-yl)-1H-pyrazole-5-carboxamide |
| 87 | 3-(1,1-difluoroethyl)-4-methyl-1-(spiro[2.3]hexan-4 ylmethyl)-N-(2-sulfamoylpyridin-4 yl)-1H-pyrazole-5-carboxamide |
| 88 | 1-((6,6-difluoro-4-methylspiro[2.3]hexan-4-yl)methyl)-3-(1,1-difluoroethyl)-4-methyl-N-(2-sulfamoylpyridin-4-yl)-1H-pyrazole-5-carboxamide |
| 89 | 3-(1,1-difluoroethyl)-4-methyl-1-(spiro[3.3]heptan-1ylmethyl)-N-(2-sulfamoylpyridin-4-yl)-1H-pyrazole-5-carboxamide |
| 90 | 3-(1,1-difluoroethyl)-4-methyl-1-((2-methylbicyclo[2.2.0]hexan-2-yl)methyl)-N-(2-sulfamoylpyridin-4-yl)-1H-pyrazole-5-carboxamide |
| 91 | 1-((3,3-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide |
| 92 | 1-((4,4-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide |
| 93 | 3-(1,1-difluoroethyl)-1-((5-fluorooctahydropentalen-2-yl)methyl)-4-methyl-N-(2-sulfamoylpyridin-4-yl)-1H-pyrazole-5-carboxamide |
| 94 | 1 -((3,3 -difluoro-5-methylbicyclo [3.1.0]hexan-1 -yl)methyl)-3 -(1,1-difluoroethyl)-4-methyl-N-(2-sulfamoylpyridin-4-yl)-1H-pyrazole-5-carboxamide |
| 95 | 1-((3,3-difluoro-5-(trifluoromethyl)bicyclo[3.1.0]hexan-1-yl)methyl)-3-(1,1-difluoroethyl)-4-methyl-N-(2-sulfamoylpyridin-4-yl)-1H-pyrazole-5-carboxamide |
| 96 | 1-((2,2-difluorobicyclo[2.1.1]hexan-1-yl)methyl)-3-(1,1-difluoroethyl)-4-methyl-N-(2-sulfamoylpyridin-4-yl)-1H-pyrazole-5-carboxamide |
| 97 | 1-((3,3-difluorobicyclo[2.1.1]hexan-1-yl)methyl)-3 -(1,1-difluoroethyl)-4-methyl-N-(2-sulfamoylpyridin-4-yl)-1H-pyrazole-5-carboxamide |
| 98 | 3-(1,1-difluoroethyl)-1-((4-fluorobicyclo [2.1.1]hexan-1yl)methyl)-4-methyl-N-(2-sulfamoylpyridin-4-yl)-1H-pyrazole-5-carboxamide |
| 99 | 3-(1,1-difluoroethyl)-1-((4-fluorobicyclo[22.1]heptan-1-yl)methyl)-4-methyl-N-(2-sulfamoylpyridin-4-yl)-1H-pyrazole-5-carboxamide |
| 100 | 1-((4,4-difluorobicyclo[4.1.0]heptan-1-yl)methyl)-3 -(1,1-difluoroethyl)-4-methyl-N-(2-sulfamoylpyridin-4-yl)-1H-pyrazole-5-carboxamide |
| 101 | 4-(3-(1,1-difluoroethyl)-4-methyl-1-(spiro[2.2]pentan-1-ylmethyl)-1H-pyrazole-5-carboxamido)picolinamide |
| 102 | 4-(3-(1,1-difluoroethyl)-1-((2,2-difluorospiro [2.2]pentan-1-yl)methyl)-4-methyl-1H-pyrazole-5-carboxamido)picolinamide |
| 103 | 4-(3-(1,1-difluoroethyl)-1-(dispiro[2.02$^{4}$.1$^{3}$]heptan-7-ylmethyl)-4-methyl-1H-pyrazole-5-carboxamido)picolinamide |
| 104 | 4-(3-(1,1-difluoroethyl)-1-((1,2-dimethylcyclopropyl)methyl)-4-methyl-1H-pyrazole-5-carboxamido)picolinamide |
| 105 | 4-(3-(1,1-difluoroethyl)-4-methyl-1-((1-methyl-2-(trifluoromethyl)cyclopropyl)methyl)-1H-pyrazole-5-carboxamido)picolinamide |
| 106 | 4-(3-(1,1-difluoroethyl)-4-methyl-1-((1-methyl-2-(trifluoromethyl)cyclopropyl)methyl)-1H-pyrazole-5-carboxamido)picolinamide |
| 107 | 4-(3-(1,1-difluoroethyl)-4-methyl-1-(spiro[2.3]hexan-5-ylmethyl)-1H-pyrazole-5-carboxamido)picolinamide |
| 108 | 4-(3-(1,1-difluoroethyl)-4-methyl-1-((5-methylspiro[2.3]hexan-5-yl)methyl)-1H-pyrazole-5-carboxa- |

(continued)

| | |
|---|---|
| | mido)picolinamide |
| 109 | 4-(3-(1,1-difluoroethyl)-4-methyl-1-((1-methyl-3-(trifluoromethyl)cyclobutyl)methyl)-1H-pyrazole-5-carboxamido)picolinamide |
| 110 | 4-(3-(1,1-difluoroethyl)-1-((1-fluorospiro[2.3]hexan-5-yl)methyl)-1H-pyrazole-5-carboxamido)picolinamide |
| 111 | 4-(1-((5,5-difluorobicyclo[2.1.0]pentan-2-yl)methyl)-3-(1,1-difluoroethyl)-4-methyl-1H-pyrazole-5-carboxamido)picolinamide |
| 112 | 4-(3-(1,1-difluoroethyl)-4-methyl-1-(spiro[2.3]hexan-4-ylmethyl)-1H-pyrazole-5-carboxamido)picolinamide |
| 113 | 4-(1-((6,6-difluoro-4-methylspiro[2.3]hexan-4-yl)methyl)-3-(1,1-difluoroethyl)-4-methyl-1H-pyrazole-5 -carboxamido)picolinamide |
| 114 | 4-(3-(1,1-difluoroethyl)-4-methyl-1-(spiro[3.3]heptan-1-ylmethyl)-1H-pyrazole-5-carboxamido)picolinamide |
| 115 | 4-(3-(1,1-difluoroethyl)-4-methyl-1-((2-methylbicyclo[2.2.0]hexan-2-yl)methyl)-1H-pyrazole-5-carboxamido)picolinamide |
| 116 | 4-(1-((3,3-difluorobicyclo[3.1.0]hexan-1-yl)methyl)-3-(1,1-difluoroethyl)-4-methyl-1H-pyrazole-5-carboxamido)picolinamide |
| 117 | 4-(1-((4,4-difluorobicyclo[3.1.0]hexan-1-yl)methyl)-3-(1,1-difluoroethyl)-4-methyl-1H-pyrazole-5-carboxamido)picolinamide |
| 118 | 4-(1-((3,3-difluoro-5-methylbicyclo[3.1.0]hexan-1-yl)methyl)-3-(1,1-difluoroethyl)-4-methyl-1H-pyrazole-5-carboxamido)picolinamide |
| 119 | 4-(1-((3,3-difluoro-5-(trifluoromethyl)bicyclo[3.1.0]hexan-1-yl)methyl)-3-(1,1-difluoroethyl)-4-methyl-1H-pyrazole-5-carboxamido)picolinamide |
| 120 | 4-(1-((2,2-difluorobicyclo[2.1.1]hexan-1-yl)methyl)-3-(1,1-difluoroethyl)-4-methyl-1H-pyrazole-5-carboxamido)picolinamide |
| 121 | 4-(1-((2,2-difluorobicyclo[2.1.1]hexan-1-yl)methyl)-3-(1,1-difluoroethyl)-4-methyl-1H-pyrazole-5-carboxamido)picolinamide |
| 122 | 4-(3-(1,1-difluoroethyl)-1 -((4-fluorobicyclo[2.1.1]hexan-1-yl)methyl)-4-methyl-1H-pyrazole-5-carboxamido)picolinamide |
| 123 | 4-(3-(1,1-difluoroethyl)-1-((4-fluorobicyclo[2.2.1]heptan-1-yl)methyl)-4-methyl-1H-pyrazole-5-carboxamido)picolinamide |
| 124 | 4-(1-((4,4-difluorobicyclo[4.1.0]heptan-1-yl)methyl)-3-(1,1-difluoroethyl)-4-methyl-1H-pyrazole-5-carboxamido)picolinamide |

in the form of the free compound or a physiologically acceptable salt thereof.

In a preferred embodiment, the compound accorsding to the invention is an inhibitor of $Na_v1.8$. In the sense of the invention, the term "inhibitor of $Na_v1.8$" preferably means that the respective compound exhibits in a patch clamp assay an IC50 value on $Na_v1.8$ of at most 10 μM ($10 \cdot 10^{-6}$ mol/L); more preferably at most 1 μM; still more preferably at most 500 nM ($10^{-9}$ mol/L); even more preferably at most 100 nM; and most preferably at most 10 nM.

A preferred assay for testing compounds for their potency and method for determining an IC50 on $Na_v1.8$ is described in the experimental part down below.

In a preferred embodiment, the compound according to the invention is a selective inhibitor of $Na_v1.8$. In the sense of the invention, the term "selective inhibitor of $Na_v1.8$" preferably means that the respective compound preferably does not exhibit any inhibitory activity on $Na_v1.1$, $Na_v1.2$, $Na_v1.4$, $Na_v1.5$ and $Na_v1.6$. The skilled artisan knows suitable ways to determine whether a compound exhibits inhibitory effects on any of $Na_v1.1$, $Na_v1.2$, $Na_v1.4$, $Na_v1.5$ and $Na_v1.6$.

The invention therefore relates to a compound according to the invention for use in the inhibition of $Na_v1.8$.

Therefore, another aspect of the invention relates to a compound according to the invention for use in the treatment of pain. Still another aspect of the invention relates to a method of treatment of pain; comprising the administration of a therapeutically effective amount of a compound according to the invention to a subject in need thereof, preferably a human.

A further aspect of the invention relates to a compound according to the invention as medicament.

Another aspect of the invention relates to a pharmaceutical dosage form comprising a compound according to

the invention. Preferably, the pharmaceutical dosage form comprises a compound according to the invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the invention is preferably prepared with the aid of conventional means, devices, methods and processes known in the art. The amount of the compound according to the invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the invention are administered per kg of the patient's body weight.

Therefore, another aspect of the invention relates to the pharmaceutical dosage form according to the invention for use in the treatment of pain. Still another aspect of the invention relates to a method of treatment of pain; comprising the administration of a pharmaceutical dosage form according to the invention to a subject in need thereof, preferably a human.

**Experimental protocols**

The following abbreviations are used in the descriptions of the experimental protocols: ABPR = automatic back pressure regulator; aq. = aqueous; Boc = tert-butyloxycarbonyl; DAST = diethylaminosulfur trifluoride, DCM = dichloromethane; DIAD = diisopropylazodicarboxylate; DIPEA = N,N-diisopropylethylamine; DMF = N,N-dimethylformamide; DMSO = dimethylsulfoxide; EtOAc = ethyl acetate; $Et_2O$ = diethyl ether; EtOH = ethanol; h = hour; HATU = 1-[bis(dimethylamino)methylene]-1H-1,2,3-triazolo[4,5-b]pyridinium 3-oxide hexafluorophosphate; Hex = hexane; HPLC = high-performance liquid chromatography; LAH = lithium aluminium hydride, LC-MS = liquid chromatography-mass spectrometry; LiHMDS = Lithium bis(trimethylsilyl)amide; MeCN = acetonitrile; MeI = methyl iodide; MeOH = methanol; $MeSO_2Cl$ = methanesulfonyl chloride; min = minute; Ms = methanesulfonyl; MTBE = methyl tert-butyl ether; NIS = N-iodosuccinimide; NMR = nuclear magnetic resonance; prep. = preparative; rt = room temperature; $R_t$ = retention time; sat. = saturated; SEM = 2-(trimethylsilyl)ethoxymethyl; SFC = supercritical fluid chromatography; SM = starting material; TEA = triethylamine; Tf = trifluoromethylsulfonyl; THF = tetrahydrofurane; $TMSCF_3$ = trifluoromethyltrimethylsilane; Ts = p-toluenesulfonyl.

**General synthesis schemes**

As illustrated in Scheme 1, compounds of the invention can be prepared by N-alkylation of alkyl 1*H* pyrazole-5-carboxylates of the general formula (A) with appropriately functionalized alkylation reagents of the general formula (B) (where Y is leaving group, such as Br, Cl, OTs, OMs, OTf) under basic conditions to afford compounds of general formula (C). Alternatively, intermediates of the general formula (C) can be prepared using a Mitsunobu reaction between alkyl 1*H*-pyrazole-5-carboxylates of type (A) and alcohols of the general formula (B) (where Y is OH) (Chem. Rev. 2009, 109, 6, 2551-2651). Intermediates of the general formula (C) can be hydrolized to carboxylic acids of the general formula (D).

**Scheme 1:** L, R1, R4, R5 are as defined in claim 1.

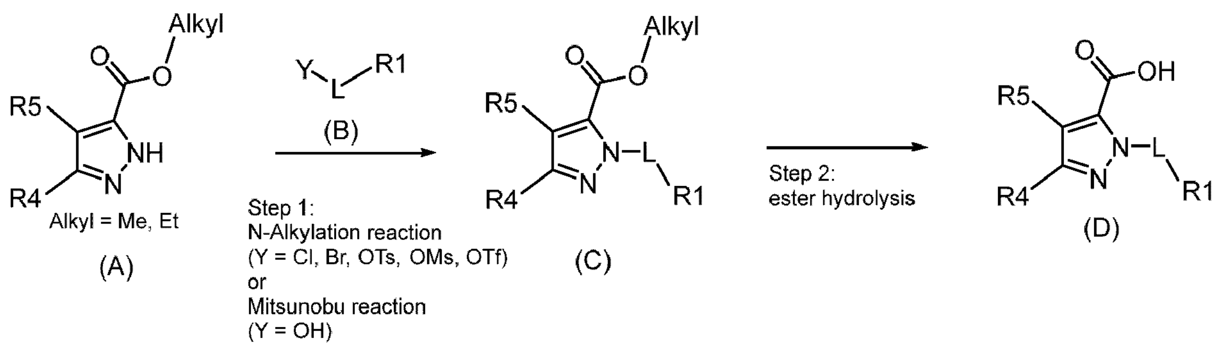


In some embodiments, intermediates need to bear one or more protecting groups, such as SEM (trimethylsilylethoxymethyl) or Bn (benzyl) which are deprotected after N-alkylation using standard deprotection protocols known in the art (T. W. Green, P. G. M. Wuts, Protective Groups in Organic Synthesis, Wiley-Interscience, New York, 1999). In some embodiments functional group interconversions are used such as fluorination using e.g. DAST (Synthesis, 2002, 2561-2578) or difluromethylation using sodium 2-chloro-2,2-difluoroacetate (Chem. Soc. Rev, 2021, 50, 8214-8247).

**Scheme 2:** L, R1, R4, R5 are as defined in claim 1.

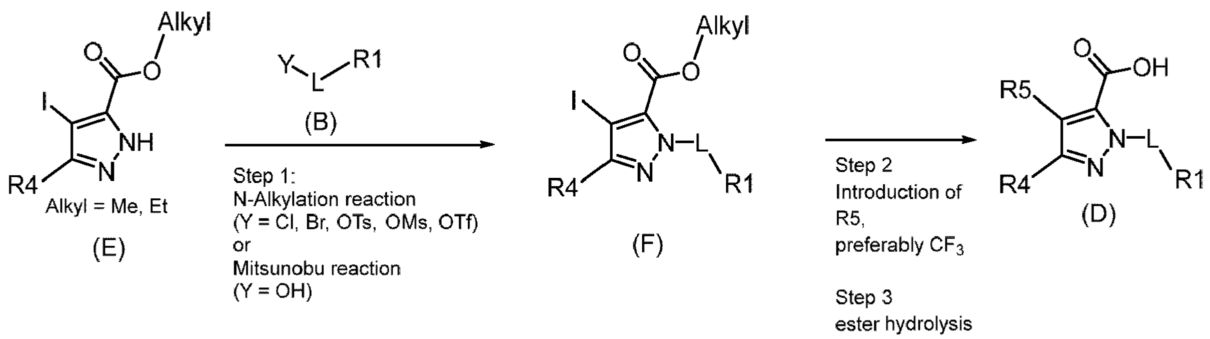


As illustrated in Scheme 2, intermediates of the general formula (D) can also be obtained by *N*-alkylation of alkyl 4-iodo-1*H*-pyrazole-5-carboxylates of the general formula (E) with appropriately functionalized alkylation reagents of the general formula (B) (Y is leaving group, such as Br, Cl, OTs, OMs, OTf) under basic conditions to give intermediates of the general formula (F). Introduction of the substituent R5 e.g. via trifluoromethylation with $TMSCF_3$ in the presence of CuI or Suzuki coupling followed by ester hydrolysis gives intermediates of the general formula (D). Alternatively, a Mitsunobu reaction between alkyl 4-iodo-1*H*-pyrazole-5-carboxylates of the general formula (E) and alcohols of the general formula (B) (where Y is OH) can be used to obtain intermediates of the general formula (F).

**Scheme 3:** R4, R5 are as defined in claim 1.

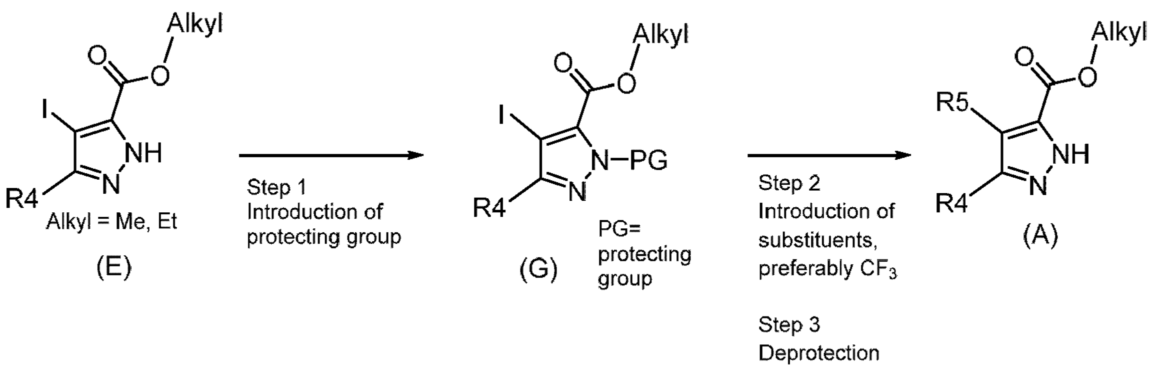


As illustrated in Scheme 3, alkyl 4-iodo-1*H*-pyrazole-5-carboxylates of the general formula (E) can also be used

to obtain alkyl 1*H*-pyrazole-5-carboxylates of the general formula (A) using a sequence consisting of introduction of a protecting group followed by introduction of the substituent R5 e.g. via trifluoromethylation with $TMSCF_3$ in the presence of CuI or Suzuki coupling followed by deprotection. Suitable protecting groups are e.g. SEM (trimethylsilylethoxymethyl). Standard protection/ deprotection protocols are known in the art (T. W. Green, P. G. M. Wuts, Protective Groups in Organic Synthesis, Wiley-Interscience, New York, 1999).

**Scheme 4:** L, X, R1, R2, R3, R4, R5 are as defined in claim 1.

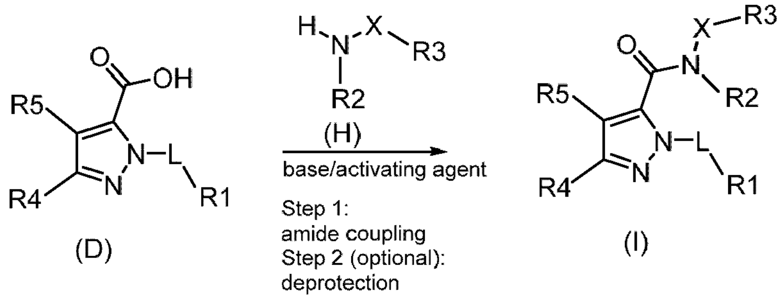


As illustrated in Scheme 4, carboxylic acids of the general formula (D) can be converted into carboxamides of the general formula (I) upon amide coupling with amines of the general formula (H) under coupling conditions well precedented in the literature (eg March's Advanced Organic Chemistry, 2007, 6th Edition, page 1427-1474).

Alternatively, and as illustrated in Scheme 5, carboxylic acids of the general formula (D) can be converted into carboxamides of the general formula (H) upon amide coupling with amines of the general formula (G) under coupling conditions well precedented in the literature (eg March's Advanced Organic Chemistry, 2007, 6th Edition, page 1427-1474). Intermediates of the general formula (H) can be further used in metal-catalyzed C-N coupling reactions with the corresponding aryl halides or heteroaryl halides of the general formula (I), preferably with corresponding aryl bromides, aryl iodides, heteroaryl bromides or heteroaryl iodides, to provide compounds of the general formula (F). Metal catalyzed C-N coupling reactions are generally known in the art (Current Organic Synthesis, 2011, 8, 53). Favorable C-N coupling reactions are palladium and copper catalyzed cross-coupling reactions *(*Chem. Rev. 2016, 116, 12564; Chem. Soc. Rev. 2014, 43, 3525; Chem. Sci. 2010, 1, 13).

**Scheme 5:** L, X, R1, R2, R3, R4, R5 are as defined in claim 1. Z=halogen.

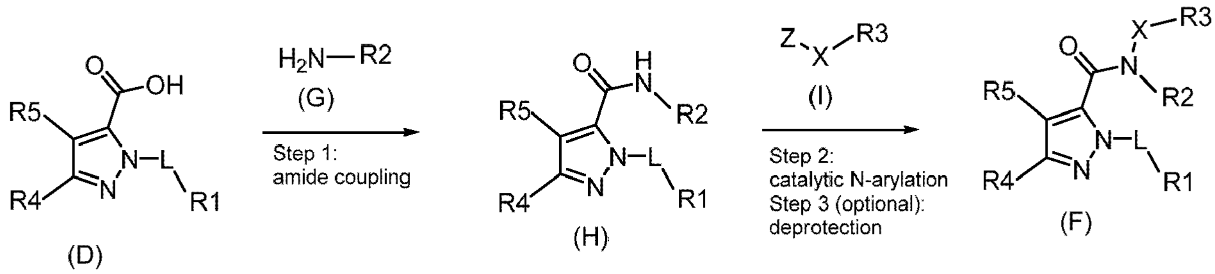


In some embodiments, (hetero)arylamines of the general formula (H) or (hetero)arylhalides of the general formula (I) need to bear one or several protecting groups, such as *tert*-butyloxycarbonyl (Boc) or *tert*-butyl (tBu), which are deprotected after amide bond formation (optional step-3, Schemes 4-5) using standard deprotection protocols known in the art (T. W. Green, P. G. M. Wuts, Protective Groups in Organic Synthesis, Wiley-Interscience, New York, 1999).

Alkyl 1*H*-pyrazole-5-carboxylates of the general formula (A), alkylation reagents of the general formula (B), (hetero)arylamines of the general formula (E), (hetero)arylhalides of the general formula (I) and (hetero)arylamines of the general formula (H) and can be either commercially available or prepared as described in the present invention or synthesized according to the standard procedures known to the person skilled in the art.

In some embodiments, carboxamides of the general formula F need to be converted into target compounds using further protocols known to the person skilled in the art, for example amidation of a carboxylic acid, as exemplified in Example 15.

**HPLC conditions:**

**Basic:**

Method A: Column: YMC Triart C18 (250 x 20.0 mm, 5 μm); Mobile phase: A = 10 mM Ammonium acetate in water, B=MeCN; Flow rate: 16 mL/min.

Method B: Column: Xterra RP18 OBD (250 x 19.0 mm, 10 μm); Mobile phase: A = 20 mM Ammonium bicarbonate in water, B=MeCN; Flow rate: 16 mL/min.

Method C: Column: YMC Triart C18 (250 x 20 mm, 5 μm); Mobile phase: A = 20 mM Ammonium bicarbonate in water, B=MeCN; Flow rate of 16 mL/min.

Method D: Column: LYMC (250 x 20 mm, 5μm); Mobile phase: A = 20 mM Ammonium bicarbonate in water, B=MeCN; Flow rate of 16 mL/min

Method E: Column: YMC Actus Triart C18 (250 x 20 mm, 5 μm); Mobile phase: A = 20 mM Ammonium bicarbonate in water, B=MeCN; Flow rate of 16 mL/min.

Method F: Column: X-SELECT-C18 (150 x 19mm, 5 μm); Mobile phase: A = 10 mM Ammonium bicarbonate in water, B=MeCN; Flow rate of 17 mL/min.

Method G: Column: X-SELECT-C18 (150 x 19mm, 5 μm); Mobile phase: A = 10 mM Ammonium bicarbonate in water, B=MeOH; Flow rate of 17 mL/min.

Method H: Column: XBridge C18 (250 x 19 mm, 5 μm); Mobile phase: A = 20 mM Ammonium bicarbonate in water, B=MeOH; Flow rate of 16 mL/min.

Method I: Column: X-SELECT-C18 (250 x 19mm, 5 μm); Mobile phase: A = 10 mM Ammonium bicarbonate in water, B=MeCN; Flow rate of 17 mL/min.

Method J: Column: X-SELECT-C18 (250 x 19mm, 5 μm); Mobile phase: A = 10 mM Ammonium acetate in water, B=MeCN; Flow rate of 17 mL/min.

**Acidic:**

Method K: Column: YMC Triart C18 (150 x 25 mm, 10 μm); Mobile phase: A = 0.1% Formic Acid in water, B=MeCN; Flow rate: 22 mL/min.

Method L: Column: X-SELECT C18 (250 x 20.0 mm, 5 μm); Mobile phase: A = 0.1% Formic Acid in water, B=MeCN; Flow rate: 17 mL/min.

Method M: Column: X-SELECT C18 (150 x 19mm, 5 μm); Mobile phase: A = 0.1% Formic Acid in water, B=MeCN; Flow rate: 17 mL/min.

Grace chromatography: C18 40 μm irregular column, 40 g (FlashPure (BUCHI)); Max pressure rating 245 psi, eluent: 0.1% formic acid/MeCN

**Intermediate 1**

ethyl 4-iodo-5-methyl-1H-pyrazole-3-carboxylate

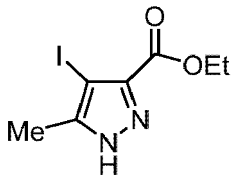

To a solution of ethyl 5-methyl-1H-pyrazole-3-carboxylate (50.0 g, 0.32 mol) in DCM (500 mL) was added NIS (86.4 g, 0.38 mol) portion wise at 0 °C. The resulting reaction mixture was stirred at rt for 16 h. The reaction was quenched with sat. sodium thiosulfate solution (800 mL) at 0°C and extracted with DCM (500 mL). The organic layer was washed with sat. $NaHCO_3$ solution (2 x500 mL) followed by brine (400 mL), dried over $Na_2SO_4$ and concentrated under reduced pressure to afford ethyl 4-iodo-5-methyl-1H-pyrazole-3-carboxylate (75 g, 83% yield). LC-MS: m/z $[M+H]^+$ = 281.

**Intermediate 2**

ethyl 4-iodo-5-methyl-1-((2-(trimethylsilyl)ethoxy)methyl)-1H-pyrazole-3-carboxylate or ethyl 4-iodo-3-methyl-1-((2-(trimethylsilyl)ethoxy)methyl)-1H-pyrazole-5-carboxylate

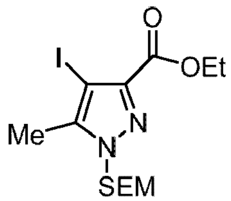

or

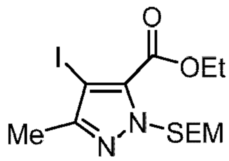

To the solution of ethyl 4-iodo-5-methyl-1H-pyrazole-3-carboxylate (Intermediate 1, 35.0 g, 0.12 mol) in DCM (875 mL) was added DIPEA (64.8 mL, 0.37 mol) at 0 °C and the resulting mixture was stirred for 30 min. SEM-Cl (26.4 mL, 0.14 mol) in DCM (100 mL) was added dropwise at 0 °C. The reaction mixture was stirred at rt for 16 h, then quenched with ice water (1000 mL) and extracted with DCM (1000 mL). The organic layer was washed with brine, dried over $Na_2SO_4$ and concentrated under reduced pressure. The residue was purified by column chromatography (silica gel, 10% EtOAc/Hex) to afford peak 1, ethyl 4-iodo-5-methyl-1-((2-(trimethylsilyl)ethoxy)methyl)-1H-pyrazole-3-carboxylate or ethyl 4-iodo-3-methyl-1-((2-(trimethylsilyl)ethoxy)methyl)-1H-pyrazole-5-carboxylate (35 g, 68%yield). LC-MS: m/z $[M+H]^+$ = 411. Further elution provided peak 2, ethyl 4-iodo-3-methyl-1-((2-(trimethylsilyl)ethoxy)methyl)-1H-pyrazole-3-carboxylate or ethyl 4-iodo-5-methyl-1-((2-(trimethylsilyl)ethoxy)methyl)-1H-pyrazole-5-carboxylate.

**Intermediate 3**

ethyl 5-methyl-4-(trifluoromethyl)-1-((2-(trimethylsilyl)ethoxy)methyl)-1H-pyrazole-3-carboxylate or ethyl 3-methyl-4-(trifluoromethyl)-1-((2-(trimethylsilyl)ethoxy)methyl)-1H-pyrazole-5-carboxylate

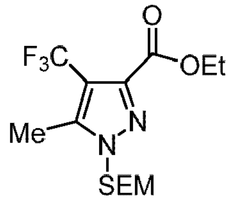

or

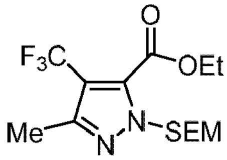

To the solution of ethyl 4-iodo-5-methyl-1-((2-(trimethylsilyl)ethoxy)methyl)-1H-pyrazole-3-carboxylate or ethyl 4-iodo-3-methyl-1-((2-(trimethylsilyl)ethoxy)methyl)-1H-pyrazole-5-carboxylate (Intermediate 2, 42.0 g, 0.1 mol) in DMF (420 mL) were added KF (14.5 g, 0.25 mol) and CuI (28.6 g, 0.15 mol) followed by $TMSCF_3$ (64.0 g, 0.45 mol) at rt. The resulting reaction mixture was heated at 80 °C for 18 h in a sealed tube. The reaction was quenched with ice cold water (1000 mL), diluted with MTBE (1000 mL) and filtered. The organic layer was separated, washed with brine and dried

over $Na_2SO_4$. The solvent was evaporated under reduced pressure to afford a residue which was purified by column chromatography (silica gel, 10% EtOAc/Hex) to afford ethyl 5-methyl-4-(trifluoromethyl)-1-((2-(trimethylsilyl)ethoxy)methyl)-1H-pyrazole-3-carboaxylate or ethyl 3-methyl-4-(trifluoromethyl)-1-((2-(trimethylsilyl)ethoxy)methyl)-1H-pyrazole-5-carboxylate (30 g, 83% yield). LC-MS: m/z $[M+H]^+$ = 353.

**Intermediate 4**

ethyl 5-methyl-4-(trifluoromethyl)-1H-pyrazole-3-carboxylate

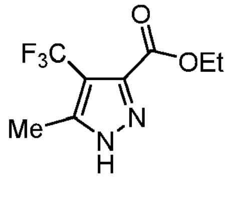

To a solution of ethyl 5-methyl-4-(trifluoromethyl)-1-((2-(trimethylsilyl)ethoxy)methyl)-1H-pyrazole-3-carboxylate or ethyl 3-methyl-4-(trifluoromethyl)-1-((2-(trimethylsilyl)ethoxy)methyl)-1H-pyrazole-5-carboxylate (Intermediate 3, 31.0 g, 87 mmol) in EtOH (310 mL) was added 4M HCl in dioxane (250 mL) dropwise at 0 °C. The resulting reaction mixture was stirred at rt for 16 h, then concentrated under reduced pressure. The residue was diluted with EtOAc (750 mL), washed with sat. $NaHCO_3$ solution (2x300 mL) followed by brine (300 mL) and dried over $Na_2SO_4$. The solvent was evaporated under reduced pressure to afford ethyl 5-methyl-4-(trifluoromethyl)-1H-pyrazole-3 carboxylate (12 g, 61% yield). LC-MS: m/z $[M+H]^+$ = 223.

**Intermediate 5**

ethyl 3-cyclopropyl-4-iodo-1H-pyrazole-5-carboxylate

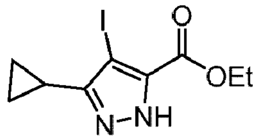

The title compound was prepared (25 g, 90% yield) from ethyl 3-cyclopropyl-1H-pyrazole-5-carboxylate using a similar method to that described for Intermediate 1. $^1$H-NMR (DMSO-$d_6$, 400 MHz) $\delta$ (ppm) = 4.64 (s, 2H), 4.35 (q, 2H), 3.43 (s, 2H), 1.99-1.97 (m, 1H), 1.41-1.35 (m, 4H), 1.30-1.26 (m, 6H), 0.94-0.90 (m, 2H), 0.82-0.80 (m, 2H).

**Intermediate 6**

ethyl 3-cyclopropyl-4-iodo-1-((2-(trimethylsilyl)ethoxy)methyl)-1H-pyrazole-5-carboxylate

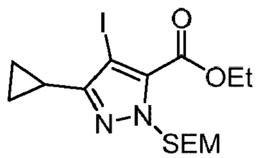

The title compound was prepared (22 g, 90% yield) from ethyl 3-cyclopropyl-4-iodo-1H-pyrazole-5-carboxylate (Intermediate 5) using a similar method to that described for Intermediate 2.

Note: Structures of the isomers were confirmed by NOE experiment and ethyl 3-cyclopropyl-4-iodo-1-((2-(trimethylsilyl)ethoxy)methyl)-1H-pyrazole-5-carboxylate was used for next step.

**Intermediate 7**

ethyl 3-cyclopropyl-4-(trifluoromethyl)-1-((2-(trimethylsilyl)ethoxy)methyl)-1H-pyrazole-5-carboxylate

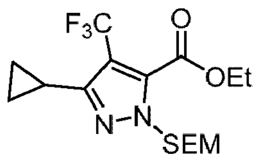

The title compound was prepared (7.8 g, 78% yield) from ethyl 3-cyclopropyl-4-iodo-1-((2-(trimethylsilyl)ethoxy)methyl)-1H-pyrazole-5-carboxylate (Intermediate 6) using a similar method to that described for Intermediate 3.

**Intermediate 8**

ethyl 3-cyclopropyl-4-(trifluoromethyl)-1H-pyrazole-5-carboxylate

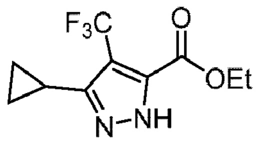

The title compound was prepared (1.8 g, 58% yield) from ethyl 3-cyclopropyl-4-(trifluoromethyl)-1-((2-(trimethylsilyl)ethoxy)methyl)-1H-pyrazole-5-carboxylate (Intermediate 7) using a similar method to that described for Intermediate 4. LC-MS: m/z $[M+H]^+$ = 249.

**Intermediate 9**

ethyl 3-acetyl-4-methyl-1H-pyrazole-5-carboxylate

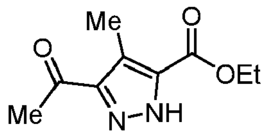

To a stirred solution of KOH (112 g, 2 mol) in EtOH (1.7 L), was added pentane-2,4-dione (200 g, 2 mol) at 80 °C, followed by ethyl diazoacetate (258 g, 2 mol). The resulting reaction mixture was stirred at 80 °C for 1 h. The reaction was concentrated under reduced pressure, diluted with water (4.4 L), the pH adjusted to ~2 with 3N aq. HCl solution at 15 - 20 °C. The precipitate was filtered, washed with excess water and dried under reduced pressure to afford ethyl 3-acetyl-4-methyl-1H-pyrazole-5-carboxylate (150 g, 38% yield). The crude product was taken to next step without purification. LC-MS: m/z $[M+H]^+$ = 197.

**Intermediate 10**

ethyl 3-(1,1-difluoroethyl)-4-methyl-1H-pyrazole-5-carboxylate

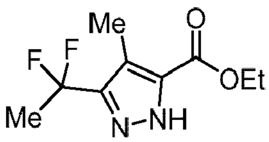

To a stirred solution of ethyl 3-acetyl-4-methyl-1H-pyrazole-5-carboxylate (Intermediate 9, 100 g, 510 mmol) in DCM (1L) was added DAST (164 g, 1.02 mol) at 0 °C under argon atmosphere. The reaction mixture was stirred at rt for 12 h, then quenched with 50% aq. $NaHCO_3$ solution (1.0 L). The aqueous layer was extracted with DCM (1.0 L). The combined organic layers were washed with water (1.0 L), brine (1.0 L), dried over anhydrous $Na_2SO_4$ and concentrated under reduced pressure to get a residue which was purified by column chromatography (silica gel, 10% EtOAc/Hex) to afford ethyl 3-(1,1-difluoroethyl)-4-methyl-1H-pyrazole-5-carboxylate (40 g, 35% yield). $^1$HNMR (400 MHz, DMSO-$d_6$): $\delta$ 13.97 (s, 1H), 4.35 - 4.32 (m, 2H), 2.44 (s, 3H), 2.00 (t, 3H), 1.34 (t, 3H)

**Intermediate 11**

ethyl 4-methyl-5-(trifluoromethyl)-4,5-dihydro-1H-pyrazole-3-carboxylate

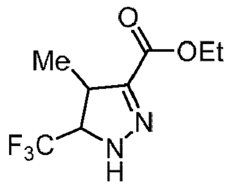

To a solution of ethyl (E)-but-2-enoate (10.0 g, 87.6 mmol) in DCM (80 mL) was added $NaNO_2$ (9.06 g, 131 mmol) in $H_2O$ (40 mL) at 0 °C. After 15 min, 2,2,2 trifluoroethylamine hydrochloride (17.8 g, 131 mmol) was added to the reaction mixture. The reaction was stirred at room temperature for 20 h, then diluted with ice water and extracted with EtOAc (3x500 mL). The combined organic layers were washed with brine, dried over $Na_2SO_4$ and concentrated under reduced pressure to get the crude which was purified by CombiFlash chromatography (silica gel, 0-60% EtOAc/Hex) to yield ethyl 4-methyl-5-(trifluoromethyl)-4,5-dihydro-1H-pyrazole-3-carboxylate (9.7 g, 49% yield). LC-MS: m/z $[M+H]^+$ = 225.

**Intermediate 12**

ethyl 4-methyl-3-(trifluoromethyl)-1H-pyrazole-5-carboxylate

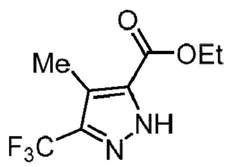

To a solution of ethyl 4-methyl-5-(trifluoromethyl)-4,5-dihydro-1H-pyrazole-3-carboxylate (Intermediate 11, 9.7 g, 43.3 mmol) in $Et_2O$ (150 mL) was added $Br_2$ (8.31 g, 52.0 mmol) at 0 °C. The reaction mixture was stirred for 16 h at rt. The reaction was concentrated and quenched with saturated solution of sodium thiosulfate (500 mL) at 0 °C and extracted with EtOAc (2x500 mL). The combined organic layers were washed with brine (300 mL) and dried over $Na_2SO_4$. The solvent was evaporated under reduced pressure to get a residue which was purified by CombiFlash column chromatography (silica gel, 0-50% EtOAc/Hex) to yield ethyl 4-methyl-3-(trifluoromethyl)-1H-pyrazole-5-carboxylate (7.0 g, 72%yield). LC-MS: m/z $[M+H]^+$ = 223.

**Intermediate 13**

(3-fluorobicyclo[1.1.1]pentan-1-yl)methyl methanesulfonate

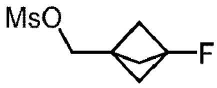

To a solution of (3-fluorobicyclo[1.1.1]pentan-1-yl)methanol (165 mg, 1.44 mmol) in DCM (10 mL) was added TEA (0.6 mL, 4.32 mmol) followed by $MeSO_2Cl$ (0.14 mL, 1.73 mmol) at 0 °C. The reaction mixture was stirred at rt for 1 h. The reaction was diluted with DCM (100 mL), washed with water (2x50 mL) and brine (50 mL). The organic layer was dried over $Na_2SO_4$ and concentrated under reduced pressure to yield (3-fluorobicyclo[1.1.1]pen-tan-1-yl)methyl methanesulfonate which was used in next step without further purification (278 mg).

**Intermediates 14 to 19**

The following Intermediates were synthesised using a similar method as described for Intermediate 13 from the corresponding alcohol.

| Intermediate | Structure and Name | Starting Materials (SM) and Data |
|---|---|---|
| **14** | 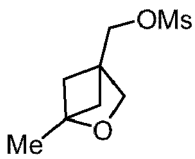 (1-methyl-2-oxabicyclo [2.1.1] hexan-4-yl)methyl methanesulfonate | SM: (1-methyl-2-oxabicyclo[2.1.1]hexan-4-yl)methanol<br>640 mg (crude) |
| **15** | 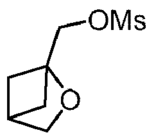 (2-oxabicyclo[2.1.1]hexan-1-yl)methyl methanesulfonate | SM: (2-oxabicyclo [2.1.1]hexan-1-yl)methanol<br>542 mg (crude) |
| **16** | 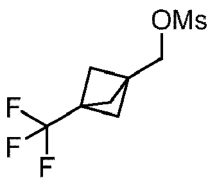 (3-(trifluoromethyl)bicyclo[1.1.1]pentan-1-yl)methyl methanesulfonate | SM: (3-(trifluoromethyl) bicyclo[1.1.1]pen-tan-1-yl)methanol<br>625 mg (crude)<br>$^1$H-NMR ($CDCl_3$, 400 MHz) $\delta$ ppm = 4.23 (s, 2H), 3.01 (s, 3H), 2.01 (s, 6H). |
| **17** | 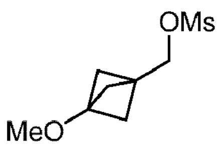 (3-methoxybicyclo[1.1.1]pentan-1-yl)methyl methanesulfonate | SM: (3-methoxybicyclo [1.1.1]pentan-1yl)methanol<br>310 mg (crude) |

(continued)

| Intermediate | Structure and Name | Starting Materials (SM) and Data |
|---|---|---|
| 18 | 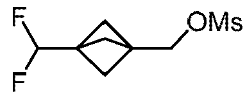 (3-(difluoromethyl)bicyclo[1.1.1]pentan-1-yl)methyl methanesulfonate | SM: (3-(difluoromethyl) bicyclo[1.1.1]pentan-1-yl)methanol<br>380 mg (crude) |
| **19** | 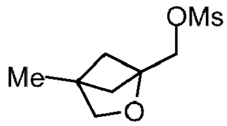 (4-methyl-2-oxabicyclo [2.1.1] hexan-1-yl)methyl methanesulfonate | SM: (4-methyl-2-oxabicyclo[2.1.1]hexan-1-yl)methanol<br>1.0 g (crude) |

**Intermediate 20**

ethyl 2-methyl-4-oxocyclopentane-1-carboxylate

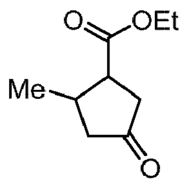

To a solution of ethyl 2-methyl-4-oxocyclopent-2-ene-1-carboxylate (5.0 g, 29.8 mmol) in EtOH (100 mL) was added 10% Pd/C (1.0 g) at rt under argon atmosphere. The resulting reaction mixture was stirred under $H_2$ balloon pressure (40 psi) at rt for 6 h. The reaction mixture was filtered through a Celite® pad and washed with EtOAc (50 mL). The filtrate was dried over anhydrous $Na_2SO_4$ and concentrated under reduced pressure to afford ethyl 2-methyl-4-oxocyclopentane-1-carboxylate (4.7 g, crude) which was used in the next step without further purification.

**Intermediate 21**

ethyl 4,4-difluoro-2-methylcyclopentane-1-carboxylate

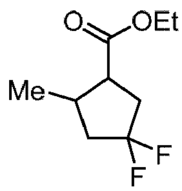

To a solution of ethyl 2-methyl-4-oxocyclopentane-1-carboxylate (Intermediate 20, 4.0 g, 23.5 mmol) in DCM (80 mL) was added DAST (15.2 g, 94.1 mmol) at 0 °C under argon atmosphere. The reaction mixture was stirred at rt for 24 h. The reaction was quenched with sat. $NaHCO_3$ solution (150 mL) and extracted with DCM (2x100 mL). The combined organic layers were washed with $H_2O$ (100 mL), brine (100 mL), dried over anhydrous $Na_2SO_4$ and concentrated under reduced pressure to afford ethyl 4,4-difluoro-2-methylcyclopentane-1-carboxylate (3.1 g, crude) which was used in the next step without further purification.

**Intermediate 22**

ethyl 4,4-difluoro-1,2-dimethylcyclopentane-1-carboxylate

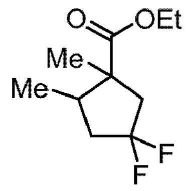

To a solution of ethyl 4,4-difluoro-2-methylcyclopentane-1-carboxylate (Intermediate 21, 3.0 g, 15.6 mmol) in THF (35 mL) was added LiHMDS in THF (1M, 23.4 mL, 23.4 mmol)) at -5 °C. The reaction mixture was stirred at 0 °C for 30 mins under argon atmosphere. To the reaction mixture was added a solution of MeI (3.33 g, 23.4 mmol) in THF (10 mL). The reaction mixture was stirred at 0 °C - rt for 12 h. The reaction was slowly quenched with saturated $NH_4Cl$ solution (75 mL) and extracted with EtOAc (2x70 mL). The combined organic layers were washed with $H_2O$ (75 mL), brine (50 mL), dried over anhydrous $Na_2SO_4$ and concentrated under reduced pressure to afford ethyl 4,4-difluoro-1,2-dimethylcyclopentane-1-carboxylate (3.1 g crude) which was used in the next step without further purification.

**Intermediate 23**

(4,4-difluoro-1,2-dimethylcyclopentyl)methanol

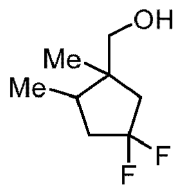

To a solution of ethyl 4,4-difluoro-1,2-dimethylcyclopentane-1-carboxylate (Intermediate 22, 3.0 g, 10.5 mmol) in THF (45 mL) was added LAH in THF (1M, 15.7 mL, 15.7 mmol) at 0 °C under argon atmosphere. The reaction mixture was stirred at 0 °C for 2 h, then slowly poured into crushed ice (150 g). The pH was adjusted with 1N aq. HCl solution to 6. The mixture was extracted with EtOAc (2x100 mL). The combined organic layers were washed with $H_2O$ (75 mL), brine (50 mL), dried over anhydrous $Na_2SO_4$ and concentrated under reduced pressure to give the crude product which was purified by column chromatography (silica gel, 12% EtOAc/Hex) to afford 4,4-difluoro-1,2-dimethylcyclopentyl)methanol (1.15 g, 30% over 4 steps).

**Intermediate 24**

(4,4-difluoro-1,2-dimethylcyclopentyl)methyl trifluoromethanesulfonate

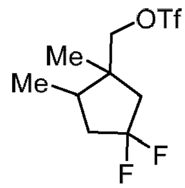

To a solution of (4,4-difluoro-1,2-dimethylcyclopentyl)methanol (Intermediate 23, 1.1 g, 6.70 mmol) in DCM (22 mL) was added pyridine (1.06 g, 13.4 mmol) followed by trifluoromethanesulfonic anhydride (2.46 g, 8.72 mmol) dropwise at 0 °C under argon atmosphere. The reaction mixture was stirred at 0 °C for 2 h, then quenched with $H_2O$ (50 mL) and

extracted with DCM (2x30 mL). The combined organic layers were washed with $H_2O$ (30 mL), brine (30 mL), dried over anhydrous $Na_2SO_4$ and concentrated under reduced pressure to afford (4,4-difluoro-1,2-dimethylcyclopentyl)methyl trifluoromethanesulfonate (2.0 g, crude) which was used without further purification.

**Intermediate 25**

ethyl 1-((3-fluorobicyclo[1.1.1]pentan-1-yl)methyl)-3-methyl-4-(trifluoromethyl)-1H-pyrazole-5-carboxylate

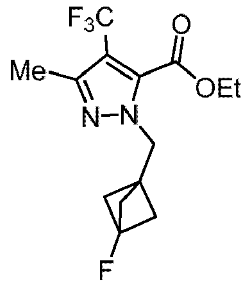

To a solution of ethyl 3-methyl-4-(trifluoromethyl)-1H-pyrazole-5-carboxylate (Intermediate 4, 320 mg, 1.44 mmol) in DMF (10 mL) were added (3-fluorobicyclo[1.1.1]pentan-1-yl)methyl methanesulfonate (Intermediate 13, 280 mg, 1.44 mmol) and $Cs_2CO_3$ (936 mg, 2.88 mmol) at rt. The reaction mixture was heated at 80 °C for 4 h, then quenched with cold $H_2O$ (50 mL) and extracted with EtOAc (3x50 mL). The combined organic layers were washed with cold brine (50 mL) and dried over $Na_2SO_4$. The solvent was evaporated under reduced pressure to give a residue which was purified by CombiFlash chromatography (silica gel, 0-5% EtOAc/Hex) to yield ethyl 1-((3-fluorobicyclo[1.1.1]pentan-1-yl)methyl)-3-methyl-4-(trifluoromethyl)-1H-pyrazole-5-carboxylate (270 mg, 58% yield). LC-MS: m/z $[M+H]^+$ = 321.

**Intermediates 26 to 37**

The following Intermediates were synthesised using a similar method as described for Intermediate 25 from the corresponding pyrazole and mesylate or tosylate. Method A denotes $K_2CO_3$, Method B denotes $Cs_2CO_3$ used as the base.

| Intermediate | Structure and Name | Starting Materials (SM) and Data |
|---|---|---|
| **26** | 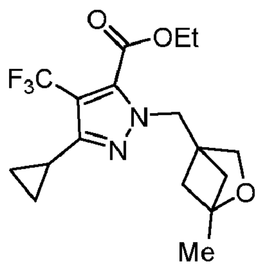 ethyl 3-cyclopropyl-1-((1-methyl-2-oxabicyclo [2.1.1]hexan-4-yl)methyl)-4-(trifluoromethyl)-1H-pyrazole-5-carboxylate | SM: Intermediate 8 and Intermediate 14<br>Method A<br>550 mg, 76% yield. LC-MS: m/z $[M+H]^+$ = 359. |

(continued)

| Intermediate | Structure and Name | Starting Materials (SM) and Data |
|---|---|---|
| **27** | 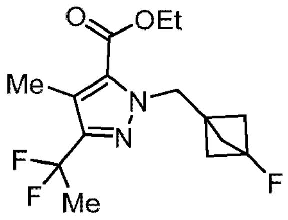 ethyl 3-(1,1-difluoroethyl)-1-((3-fluorobicyclo[1.1.1] pentan-1-yl)methyl)-4-methyl-1H-pyrazole-5-carboxylate | SM: Intermediate 10 and Intermediate 13<br>Method B<br>700 mg, 68% yield. LC-MS: m/z $[M+H]^+$ = 317. |
| **28** | 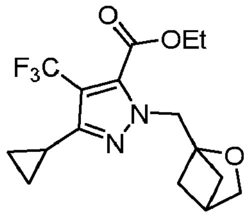 ethyl 1-((2-oxabicyclo[2.1.1]hexan-1-yl)methyl)-3-cyclopropyl-4-(trifluoromethyl)-1H-pyrazole-5-carboxylate | SM: Intermediate 8 and Intermediate 15<br>Method B<br>280 mg, 28% yield. LC-MS: m/z $[M+H]^+$ = 345. |
| **29** | 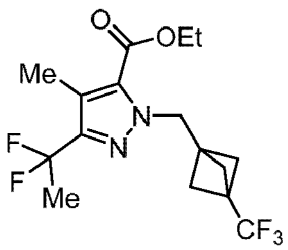 ethyl 3-(1,1-difluoroethyl)-4-methyl-1-((3-(trifluoromethyl)bicyclo [1.1.1]pentan-1-yl) methyl)-1H-pyrazole-5-carboxylate | SM: Intermediate 10 and Intermediate 16<br>Method A<br>1.2 g, 71% yield. LC-MS: m/z $[M+H]^+$ = 367. |
| **30** | 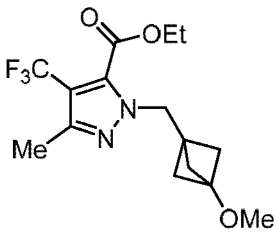 ethyl 1-((3-methoxybicyclo[1.1.1]pentan-1-yl) methyl)-3-methyl-4-(trifluoromethyl)-1H-pyrazole-5-carboxylate | SM: Intermediate 4 and 17<br>Method A<br>140 mg, 37% yield. LC-MS: m/z $[M+H]^+$ = 332. |

(continued)

| Intermediate | Structure and Name | Starting Materials (SM) and Data |
|---|---|---|
| **31** | 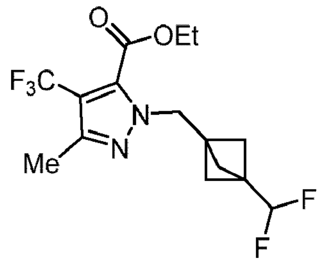 ethyl 1-((3-(difluoromethyl)bicyclo[1.1.1]pentan-1-yl)methyl)-3-methyl-4-(trifluoromethyl)-1H-pyrazole-5-carboxylate | SM: Intermediate 4 and Intermediate 18 Method A 140 mg, 7% yield. LC-MS: m/z $[M+H]^+$ = 353. |
| **32** | 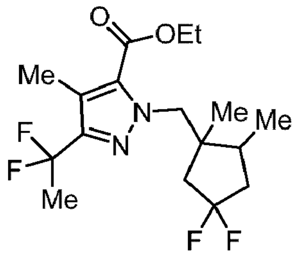 ethyl 1-((4,4-difluoro-1,2-dimethylcyclopentyl) methyl)-3-(1,1-difluoroethyl)-4-methyl-1H-pyrazole-5-carboxylate | SM: Intermediate 10 and Intermediate 24 Method B 1.32 g, 66% yield. $^1$HNMR (400 MHz, DMSO-$d_6$): δ 4.44 (s, 2H), 4.40-4.36 (m, 2H), 2.37 (s, 6H), 2.12-1.91 (m, 5H), 1.12 (d, 3H), 1.02-.096 (m, 6H). |
| **33** | 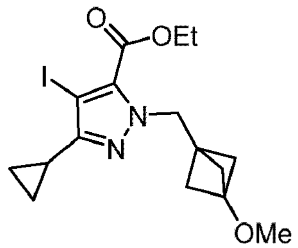 ethyl 3-cyclopropyl-4-iodo-1-((3-methoxybicyclo [1.1.1]pentan-1-yl)methyl)- 1H-pyrazole-5-carboxylate | SM: Intermediate 5 and Intermediate 17 Method A 350 mg, 30% yield over 2 steps. LC-MS: m/z $[M+H]^+$ = 417. |
| **34** | 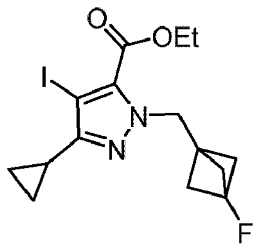 ethyl 3 -cyclopropyl-1-((3 -fluorobicyclo [1.1.1] pentan-1-yl)methyl)-4-iodo-1H-pyrazole-5-carboxylate | SM: Intermediate 5 and Intermediate 13 Method A 0.65 g, 49% yield. LC-MS: m/z $[M+H]^+$ = 405. |

(continued)

| Intermediate | Structure and Name | Starting Materials (SM) and Data |
|---|---|---|
| **35** | 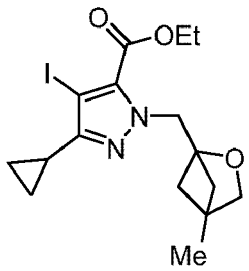 ethyl 3-cyclopropyl-4-iodo-1-((4-methyl-2-oxabicyclo[2.1.1]hexan-1-yl)methyl)-1H-pyrazole-5-carboxylate | SM: Intermediate 5 and Intermediate 19<br>Method A<br>500 mg, 36% yield. LC-MS: m/z $[M+H]^+ = 417$. |
| **36** | 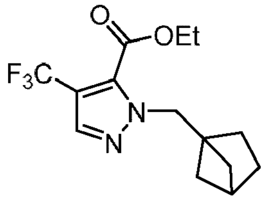 ethyl 1-(bicyclo [2.1.1]hexan-1-ylmethyl)-4-(trifluoromethyl)-1H-pyrazole-5-carboxylate | SM: ethyl 4-(trifluoromethyl)-1H-pyrazole-5-carboxylate and bicyclo[2.1.1]hexan-1-ylmethyl methanesulfonate<br>Method A<br>130 mg, 22%yield. LC-MS: m/z $[M+H]^+ = 303$. |
| **37** | 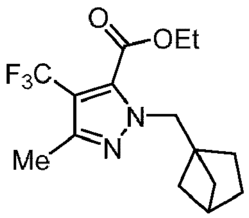 ethyl 1-(bicyclo[2.1.1]hexan-1 -ylmethyl)-3-methyl-4-(trifluoromethyl)-1H-pyrazole-5-carboxylate | SM: Intermediate 4 and bicyclo [2.1.1]hexan-1-ylmethyl methanesulfonate<br>Method A<br>65 mg, 12% yield. LC-MS: m/z $[M+H]^+ = 317$. |

**Intermediate 38**

ethyl 4-methyl-3-(trifluoromethyl)-1-((3-(trifluoromethyl)bicyclo[1.1.1]pentan-1-yl)methyl)-1H-pyrazole-5-car-boxylate

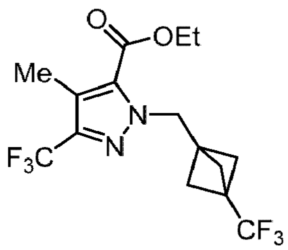

To a solution of ethyl 4-methyl-3-(trifluoromethyl)-1H-pyrazole-5-carboxylate (Intermediate 12, 0.7 g, 3.15 mmol) and (3-(trifluoromethyl)bicyclo[1.1.1]pentan-1-yl)methanol (0.68 g, 4.09 mmol) in THF (70 mL) was added $PPh_3$ (1.23 g, 4.72 mmol) at 0 °C. The reaction mixture was stirred for 5 min followed by addition of DIAD (0.9 mL, 4.72 mmol) at 0 °C. The reaction mixture was stirred at 80 °C for 16 h, then concentrated under reduced pressure to get a crude which was purified by CombiFlash chromatography (silica; 0-20% EtOAc/Hex) to yield ethyl 4-methyl-3-(trifluoromethyl)-1-((3-(trifluoromethyl)bicyclo[1.1.1]pentan-1-yl)methyl)-1H-pyrazole-5-carboxylate (0.8 g, 68% yield). LC-MS: m/z

$[M+H]^+$ = 371.

**Intermediates 39 to 41**

The following Intermediates were synthesised using a similar method as described for Intermediate 38 from the corresponding pyrazole and alcohol.

| Intermediate | Structure and Name | Starting Materials (SM) and Data |
|---|---|---|
| **39** | 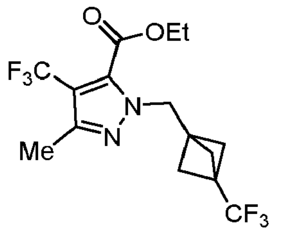 ethyl 3-methyl-4-(trifluoromethyl)-1-((3-(trifluoromethyl) bicyclo[1.1.1]pentan-1-yl)methyl)-1H-pyrazole-5-carboxylate | SM: Intermediate 4 and (3-(trifluoromethyl)bicyclo[1.1.1] pentan-1-yl)metanol<br>285 mg, 85% yield. LC-MS: m/z $[M+H]^+$ = 371. |
| **40** | 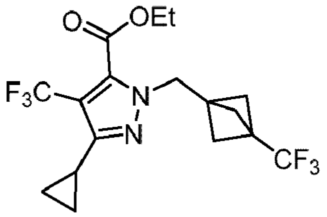 ethyl 3-cyclopropyl-4-(trifluoromethyl)-1-((3-(trifluoromethyl)bicyclo[1.1.1]pentan-1-yl)methyl)-1H-pyrazole-5-carboxylate | SM: Intermediate 8 and (3-(trifluoromethyl)bicyclo[1.1.1] pentan-1-yl)methanol<br>0.85 g, 68% yield. LC-MS: m/z $[M+H]^+$ = 397. |
| **41** | 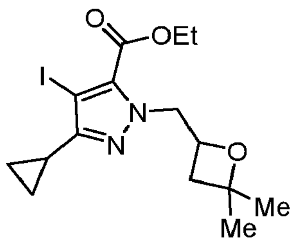 ethyl 3-cyclopropyl-1-((4,4-dimethyloxetan-2-yl)methyl)-4-iodo-1H-pyrazole-5-carboxylate | SM: Intermediate 5 and (4,4-dimethyloxetan-2-yl)methanol<br>1.03 g, 78% yield. LC-MS: m/z $[M+H]^+$ = 405. |

**Intermediate 42**

ethyl 1-((2-oxabicyclo[2.1.1]hexan-1-yl)methyl)-4-(trifluoromethyl)-1H-pyrazole-5-carboxylate

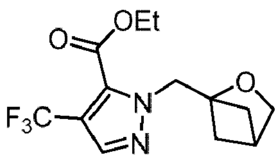

To a solution of ethyl 4-(trifluoromethyl)-1H-pyrazole-5-carboxylate (500 mg, 2.40 mmol) in MeCN (15 mL) was added $K_2CO_3$ (663 mg, 4.80 mmol) and 1-(bromomethyl)-2-oxabicyclo[2.1.1]hexane (510 mg, 2.88 mmol) at rt. The reaction mixture was stirred at 80 $^0C$ under argon atmosphere for 16 h, then filtered. The filtrate was concentrated under

reduced pressure to get a crude which was purified by column chromatography (silica gel, 6% EtOAc/Hex) to afford ethyl 1-((2-oxabicyclo[2.1.1]hexan-1-yl)methyl)-4-(trifluoromethyl)-1H-pyrazole-5-carboxylate (120 mg, 16% yield). LC-MS: m/z $[M+H]^+$ = 305.

**Intermediate 43 to 47**

The following Intermediates were synthesised using a similar method as described for Intermediate 42 from the corresponding pyrazole and bromide.

| Intermediate | Structure and Name | Starting Materials (SM) and Data |
|---|---|---|
| **43** | 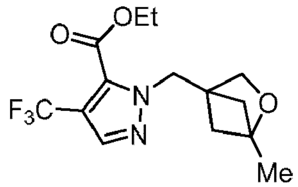 ethyl 1-((1-methyl-2-oxabicyclo[2.1.1]hexan-4-yl)methyl)-4-(trifluoromethyl)-1H-pyrazole-5-carboxylate | SM: ethyl 4-(trifluoromethyl)-1H-pyrazole-5-carboxylate and 4-(bromomethyl)-1-methyl-2-oxabicyclo[2.1.1]hexane<br>150 mg, 20% yield. LC-MS: m/z $[M+H]^+$ = 319. |
| **44** | 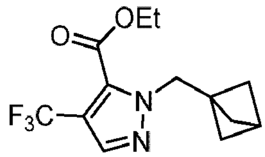 ethyl 1-(bicyclo[1.1.1]pentan-1-ylmethyl)-4-(trifluoromethyl)-1H-pyrazole-5-carboxylate | SM: ethyl 4-(trifluoromethyl)-1H-pyrazole-5-carboxylate and 1-(bromomethyl)bicyclo[1.1.1] pentane<br>170 mg, 31% yield. LC-MS: m/z $[M+H]^+$ = 289. |
| **45** | 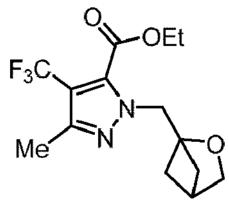 ethyl 1-((2-oxabicyclo[2.1.1]hexan-1-yl)methyl)-3-methyl-4-(trifluoromethyl)-1H-pyrazole-5-carboxylate | SM: Intermediate 4 and 1-(bromomethyl)-2-oxabicyclo[2.1.1]hexane 80 mg, 12% yield. LC-MS: m/z $[M+H]^+$ = 319. |
| **46** | 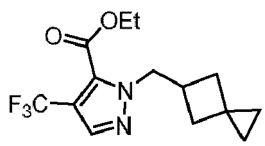 ethyl 1-(spiro [2.3] hexan-5-ylmethyl)-4-(trifluoromethyl)-1H-pyrazole-5-carboxylate | SM: ethyl 4-(trifluoromethyl)-1H-pyrazole-5-carboxylate and 5-(bromomethyl)spiro[2.3] hexane<br>180 mg, 44% yield. LC-MS: m/z $[M+H]^+$ = 303. |
| **47** | 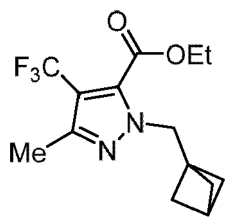 ethyl 1-(bicyclo[1.1.1]pentan-1-ylmethyl)-3-methyl-4-(trifluoromethyl)-1H-pyrazole-5-carboxylate | SM: Intermediate 4 and 1-(bromomethyl)bicyclo [1.1.1]pentane 45 mg, 7% yield.<br>$^1$HNMR (400 MHz, DMSO-$d_6$): δ 4.43 (s, 2H), 4.38-4.34 (q, 2H), 2.29 (s, 3H), 1.61 (s, 6H), 1.57 (s, 1H), 1.30 (t, 3H). |

**Intermediate 48**

ethyl 3-cyclopropyl-1-((3-methoxybicyclo[1.1.1]pentan-1-yl)methyl)-4-(trifluoromethyl)-1H-pyrazole-5-carboxylate

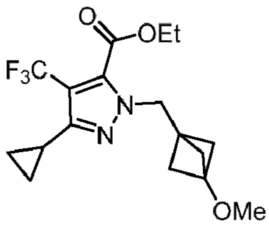

To a stirred solution of ethyl 3-cyclopropyl-4-iodo-1-((3-methoxybicyclo[1.1.1]pentan-1-yl)methyl)-1H-pyrazole-5-carboxylate (Intermediate 33, 150 mg, 0.347 mmol) in DMF (10 mL) were added CuI (98.8 mg, 0.521 mmol) and KF (24.2 mg, 0.416 mmol) at rt. The reaction mixture was degassed with argon for 10 mins, then $TMSCF_3$ (259 mg, 1.74 mmol) was added. The reaction mixture was heated to 80 °C and stirred for 16 h. Water was added (50 mL), the mixture was extracted with $Et_2O$ (2×100 mL), dried over $Na_2SO_4$ and concentrated under reduced pressure to afford ethyl 3-cyclopropyl-1-((3-methoxybicyclo[1.1.1]pentan-1-yl)methyl)-4-(trifluoromethyl)-1H-pyrazole-5-carboxylate (175 mg, crude). LC-MS: m/z $[M+H]^+$ = 359.

**Intermediate 49 to 51**

The following Intermediates were synthesised using a similar method as described for Intermediate 48 from the corresponding iodo pyrazole and $TMSCF_3$.

| Intermediate | Structure and Name | Starting Materials (SM) and Data |
|---|---|---|
| **49** | 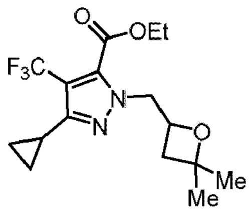 ethyl 3-cyclopropyl-1-((4,4-dimethyloxetan-2-yl)methyl)-4-(trifluoromethyl)-1H-pyrazole-5-carboxylate | SM: Intermediate 41<br>750 mg, 88% yield. LC-MS: m/z $[M+H]^+$ = 347. |
| **50** | 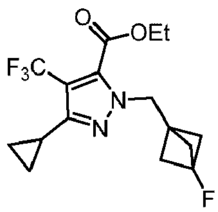 ethyl 3-cyclopropyl-1-((3-fluorobicyclo[1.1.1]pentan-1-yl)methyl)-4-(trifluoromethyl)-1H-pyrazole-5-carboxylate | SM: Intermediate 34<br>250 mg, crude. LC-MS: m/z $[M+H]^+$ = 347. |

(continued)

| Intermediate | Structure and Name | Starting Materials (SM) and Data |
|---|---|---|
| **51** | 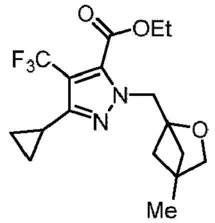 ethyl 3-cyclopropyl-1-((4-methyl-2-oxabicyclo[2.1.1]hexan-1-yl) methyl)-4-(trifluoromethyl)-1H-pyrazole-5-carboxylate | SM: Intermediate 35<br>200 mg, crude. LC-MS: m/z $[M+H]^+$ = 359. |

**Intermediate 52**

1-((3-fluorobicyclo[1.1.1]pentan-1-yl)methyl)-3-methyl-4-(trifluoromethyl)-1H-pyrazole-5-carboxylic acid

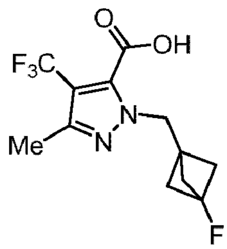

To a solution of ethyl 1-((3-fluorobicyclo[1.1.1]pentan-1 yl)methyl)-3-methyl-4-(trifluoromethyl)-1H-pyrazole-5-carboxylate (Intermediate 25, 400 mg, 1.25 mmol) in MeOH (16 mL) was added 2N NaOH (4 mL). The reaction mixture was heated at 70 °C for 1 h. The mixture was concentrated under reduced pressure and diluted with water (25 mL), acidified with sat. $NaHSO_4$ solution up to pH~3-4 and extracted with DCM (3x50 mL). The organic layer was dried over $Na_2SO_4$ and concentrated under reduced pressure to get 1-((3-fluorobicyclo[1.1.1]pentan-1-yl)methyl)-3-methyl-4-(trifluoromethyl)-1H-pyrazole-5-carboxylic acid (330 mg, 90% yield). LC-MS: m/z $[M+H]^+$ = 293.

**Intermediates 53 to 74**

The following Intermediates were synthesised using a similar method as described for Intermediate 52 from the corresponding ester. Method A denotes $LiOH{\cdot}H_2O$ in THF, Method B: NaOH in MeOH, Method C: $LiOH{\cdot}H_2O$ in THF/MeOH 2:1, Method D: $LiOH{\cdot}H_2O$ in THF/EtOH/$H_2O$ 1:1:1, Method E: $LiOH{\cdot}H_2O$ in THF/MEOH/$H_2O$ 1:1:1, Method F: NaOH in THF/MeOH/$H_2O$ 1:1:1 as reagents.

| Intermediate | Structure and Name | Starting Materials (SM) and Data |
|---|---|---|
| **53** | 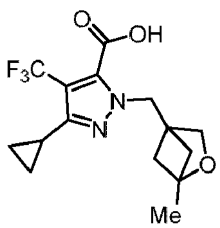 3-cyclopropyl-1-((1-methyl-2-oxabicyclo[2.1.1] hexan-4-yl)methyl)-4-(trifluoromethyl)-1H-pyrazole-5-carboxylic acid. | SM: Intermediate 26<br>Method A<br>400 mg, 87% yield. LC-MS: m/z $[M+H]^+$ = 331. |

(continued)

| Intermediate | Structure and Name | Starting Materials (SM) and Data |
|---|---|---|
| **54** | 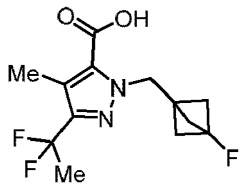 3-(1,1-difluoroethyl)-1-((3-fluorobicyclo[1.1.1] pentan-1-yl)methyl)-4-methyl-1H-pyrazole-5-carboxylic acid. | SM: Intermediate 27<br>Method B<br>700 mg, 91% yield. LC-MS: m/z $[M+H]^+$ = 313. |
| **55** | 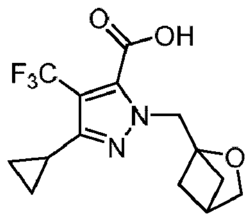 1-((2-oxabicyclo [2.1.1]hexan-1-yl)methyl)-3-cyclopropyl-4-(trifluoromethyl)-1H-pyrazole-5-carboxylic acid. | SM: Intermediate 28<br>Method B<br>270 mg, 83% yield. LC-MS: m/z $[M+H]^+$ = 316. |
| **56** | 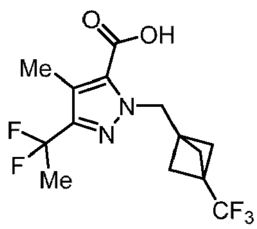 3-(1,1-difluoroethyl)-4-methyl-1-((3-(trifluoromethyl) bicyclo[1.1.1]pentan-1-yl)methyl)-1H-pyrazole-5-carboxylic acid. | SM: Intermediate 29<br>Method A<br>1.1 g, 99%yield. LC-MS: m/z $[M+H]^+$ = 339. |
| **57** | 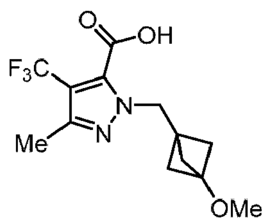 1-((3-methoxybicyclo[1.1.1]pentan-1-yl)methyl)-3-methyl-4-(trifluoromethyl)-1H-pyrazole-5-carboxylic acid. | SM: Intermediate 30<br>Method A<br>110 mg, 90% yield (crude). LC-MS: m/z $[M+H]^+$ = 305. |
| **58** | 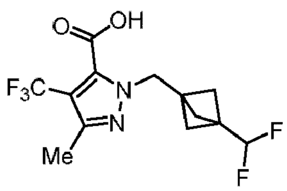 1-((3-(difluoromethyl)bicyclo[1.1.1]pentan-1-yl) methyl)-3-methyl-4-(trifluoromethyl)-1H-pyrazole-5-carboxylic acid. | SM: Intermediate 31<br>Method A<br>120 mg, 93% yield. LC-MS: m/z $[M+H]^+$ = 325. |

(continued)

| Intermediate | Structure and Name | Starting Materials (SM) and Data |
|---|---|---|
| **59** | 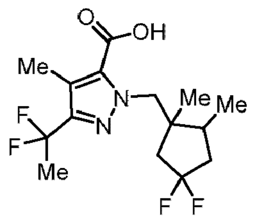 1-((4,4-difluoro-1,2-dimethylcyclopentyl)methyl)-3-(1,1-difluoroethyl)-4-methyl-1H-pyrazole-5-carboxylic acid. | SM: Intermediate 32<br>Method C<br>1.11 g, 92% yield.<br>$^1$HNMR (400 MHz, DMSO-$d_6$): δ 13.8 (brs, 1H), 4.71 (d, 1H), 4.40-4.30 (m, Hz, 1H), 2.35-2.24 (m, 5H), 2.05-1.90 (m, 6H), 1.06 (s, 3H), 0.87 (d, 3H). |
| **60** | 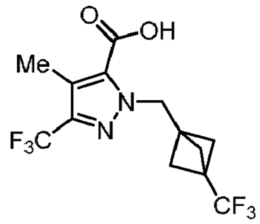 4-methyl-3-(trifluoromethyl)-1-((3-(trifluoromethyl) bicyclo[1.1.1]pentan-1-yl)methyl)-1H-pyrazole-5-carboxylic acid | SM: Intermediate 38<br>Method D<br>0.79 g, 71% yield. LC-MS: m/z $[M+H]^+$ = 343. |
| **61** | 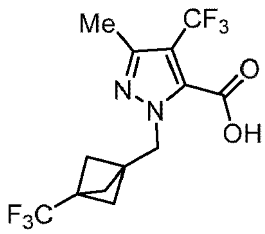 3 -methyl-4-(trifluoromethyl)-1 -((3-(trifluoromethyl) bicyclo[1.1.1]pentan-1-yl)methyl)-1H-pyrazole-5-carboxylic acid | SM: Intermediate 39<br>Method E<br>0.23 g, 87% yield. LC-MS: m/z $[M+H]^+$ = 343. |
| **62** | 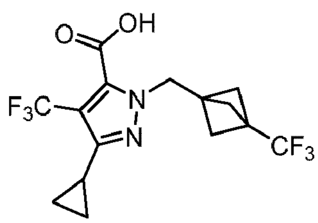 3-cyclopropyl-4-(trifluoromethyl)-1-((3-(trifluoromethyl)bicyclo[1.1.1]pentan-1-yl) methyl)-1H-pyrazole-5-carboxylic acid | SM: Intermediate 40<br>Method D<br>1.08 g, 77% yield. LC-MS: m/z $[M+H]^+$ = 369. |
| **63** | 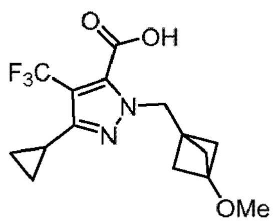 3-cyclopropyl-1-((3-methoxybicyclo[1.1.1]pentan-1-yl)methyl)-4-(trifluoromethyl)-1H-pyrazole-5-carboxylic acid | SM: Intermediate 48<br>Method E<br>300 mg, crude. LC-MS: m/z $[M+H]^+$ = 331. |

(continued)

| Intermediate | Structure and Name | Starting Materials (SM) and Data |
|---|---|---|
| **64** | 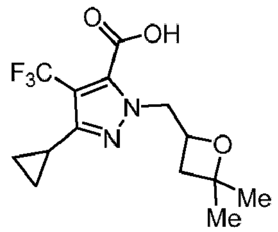 3-cyclopropyl-1-((4,4-dimethyloxetan-2-yl)methyl)-4-(trifluoromethyl)-1H-pyrazole-5-carboxylic acid | SM: Intermediate 49<br>Method E<br>950 mg, crude. LC-MS: m/z $[M+H]^+$ = 319. |
| **65** | 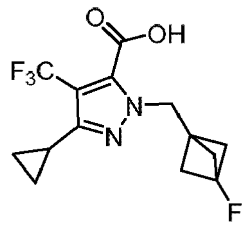 3-cyclopropyl-1-((3-fluorobicyclo[1.1.1]pentan-1-yl) methyl)-4-(trifluoromethyl)-1H-pyrazole-5-carboxylic acid | SM: Intermediate 50<br>Method E<br>450 mg, crude. LC-MS: m/z $[M+H]^+$ = 319. |
| **66** | 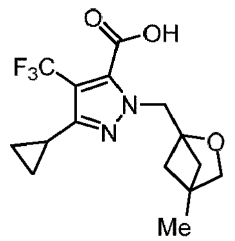 3-cyclopropyl-1-((4-methyl-2-oxabicyclo[2.1.1] hexan-1-yl)methyl)-4-(trifluoromethyl)-1H-pyrazole-5-carboxylic acid | SM: Intermediate 51<br>Method E<br>150 mg, 81 % yield over 2 steps. LC-MS: m/z $[M+H]^+$ = 331. |
| **67** | 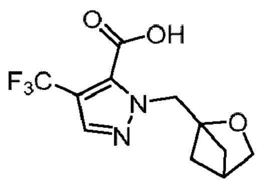 1-((2-oxabicyclo [2.1.1] hexan-1-yl)methyl)-4-(trifluoromethyl)-1H-pyrazole-5-carboxylic acid | SM: Intermediate 42<br>Method E<br>100 mg, 93% yield. LC-MS: m/z $[M+H]^+$ = 277. |
| **68** | 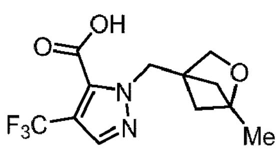 1-((1-methyl-2-oxabicyclo[2.1.1]hexan-4-yl)methyl)-4-(trifluoromethyl)-1H-pyrazole-5-carboxylic acid | SM: Intermediate 43<br>Method E<br>120 mg, 94% yield. LC-MS: m/z $[M+H]^+$ = 291. |

(continued)

| Intermediate | Structure and Name | Starting Materials (SM) and Data |
|---|---|---|
| **69** | 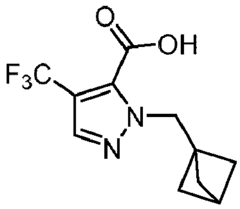 1-(bicyclo[1.1.1]pentan-1-ylmethyl)-4-(trifluoromethyl)-1H-pyrazole-5-carboxylic acid | SM: Intermediate 44<br>Method F<br>130 mg, crude. LC-MS: m/z $[M+H]^+$ = 261. |
| **70** | 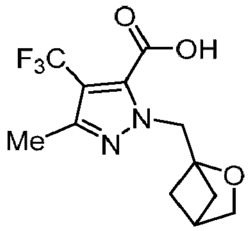 1-((2-oxabicyclo [2.1.1] hexan-1-yl)methyl)-3-methyl-4-(trifluoromethyl)-1H-pyrazole-5-carboxylic acid | SM: Intermediate 45<br>Method C<br>75 mg, crude. LC-MS: m/z $[M+H]^+$ = 291. |
| **71** | 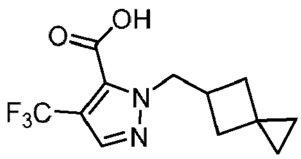 1-(spiro[2.3]hexan-5-ylmethyl)-4-(trifluoromethyl)-1H-pyrazole-5-carboxylic acid | SM: Intermediate 46<br>Method F<br>175 mg, crude. LC-MS: m/z $[M+H]^+$ = 275. |
| **72** | 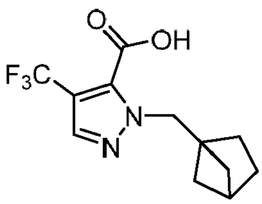 1-(bicyclo[2.1.1]hexan-1-ylmethyl)-4-(trifluoromethyl)-1H-pyrazole-5-carboxylic acid | SM: Intermediate 36<br>Method F<br>100 mg, crude. LC-MS: m/z $[M+H]^+$ = 275. |
| **73** | 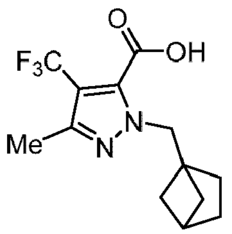 1-(bicyclo [2.1.1] hexan-1-ylmethyl)-3-methyl-4-(trifluoromethyl)-1H-pyrazole-5-carboxylic acid | SM: Intermediate 37<br>Method C<br>62 mg, crude. LC-MS: m/z $[M+H]^+$ = 289. |

(continued)

| Intermediate | Structure and Name | Starting Materials (SM) and Data |
|---|---|---|
| **74** | 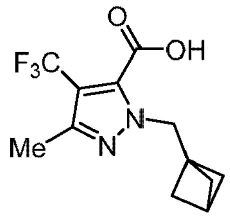 1-(bicyclo[1.1.1]pentan-1-ylmethyl)-3-methyl-4-(trifluoromethyl)-1H-pyrazole-5-carboxylic acid | SM: Intermediate 47<br>Method C<br>40 mg, crude. LC-MS: m/z $[M+H]^+$ = 275. |

**Intermediate 75**

1-((3-fluorobicyclo[1.1.1]pentan-1-yl)methyl)-3-methyl-4-(trifluoromethyl)-1H-pyrazole-5-carboxamide

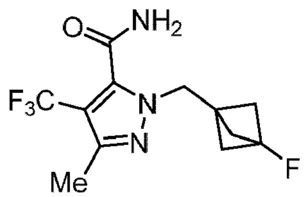

To a solution of 1-((3-fluorobicyclo[1.1.1]pentan-1-yl)methyl)-3-methyl-4-(trifluoromethyl)-1H-pyrazole-5-carboxylic acid (Intermediate 52, 330 mg, 1.13 mmol) in DMF (20 mL) were added HATU (858 mg, 2.26 mmol), DIPEA (0.6 mL, 3.26 mmol), $NH_4Cl$ (600 mg, 11.3 mmol) and the reaction mixture was stirred at rt for 16 h. The reaction was quenched with water (50 mL) and extracted with EtOAc (3x50 mL). The organic layers were washed with cold brine (50 mL) and dried over $Na_2SO_4$. The solvent was evaporated under reduced pressure to get the crude product which was purified by CombiFlash chromatography (silica gel, 0-2% MeOH/DCM) to yield 1-((3-fluorobicyclo[1.1.1]pentan-1-yl)methyl)-3-methyl-4-(trifluoromethyl)-1H-pyrazole-5-carboxamide (250 mg, 76% yield). LC-MS: m/z $[M+H]^+$ = 292.

**Intermediate 76 to 88**

The following Intermediates were synthesised using a similar method as described for Intermediate 75 from the corresponding acid.

| Intermediate | Structure and Name | Starting Materials (SM) and Data |
|---|---|---|
| **76** | 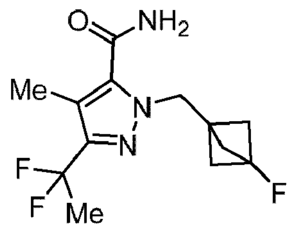 3-(1,1-difluoroethyl)-1-((3-fluorobicyclo[1.1.1]pentan-1-yl)methyl)-4-methyl-1H-pyrazole-5-carboxamide | SM: Intermediate 54<br>380 mg, 94%yield. LC-MS: m/z $[M+H]^+$ = 288. |

(continued)

| Intermediate | Structure and Name | Starting Materials (SM) and Data |
|---|---|---|
| **77** | 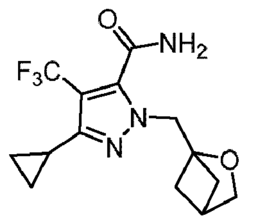 1-((2-oxabicyclo[2.1.1]hexan-1-yl)methyl)-3-cyclopropyl-4-(trifluoromethyl)-1H-pyrazole-5-carboxamide | SM: Intermediate 55 240 mg, 90% yield. LC-MS: m/z $[M+H]^+$ = 316. |
| **78** | 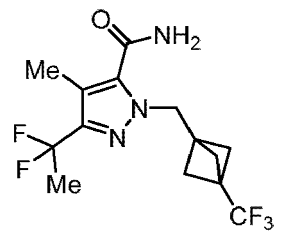 3-(1,1-difluoroethyl)-4-methyl-1-((3-(trifluoromethyl)bicyclo[1.1.1]pentan-1-yl)methyl)-1H-pyrazole-5-carboxamide | SM: Intermediate 56 400 mg, 81% yield. LC-MS: m/z $[M+H]^+$ = 338. |
| **79** | 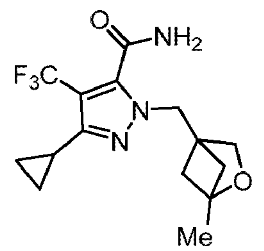 3-cyclopropyl-1-((1-methyl-2-oxabicyclo[2.1.1]hexan-4-yl)methyl)-4-(trifluoromethyl)-1H-pyrazole-5-carboxamide | SM: Intermediate 53 110 mg, 92% yield. LC-MS: m/z $[M+H]^+$ = 330. |
| **80** | 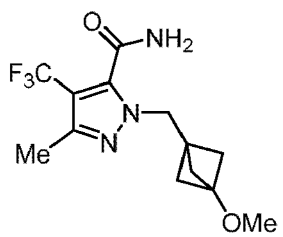 1-((3-methoxybicyclo[1.1.1]pentan-1-yl)methyl)-3-methyl-4-(trifluoromethyl)-1H-pyrazole-5-carboxamide | SM: Intermediate 57 100 mg, 90% yield. LC-MS: m/z $[M+H]^+$ = 304. |
| **81** | 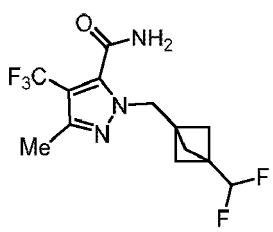 1-((4,4-difluoro-1,2-dimethylcyclopentyl)methyl)-3-(1,1-difluoroethyl)-4-methyl-1H-pyrazole-5-carboxamide | SM: Intermediate 58 85 mg, 71% yield. LC-MS: m/z $[M+H]^+$ = 324. |

(continued)

| Intermediate | Structure and Name | Starting Materials (SM) and Data |
|---|---|---|
| **82** | 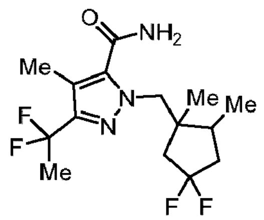 1-((4,4-difluoro-1,2-dimethylcyclopentyl)methyl)-3-(1,1-difluoroethyl)-4-methyl-1H-pyrazole-5-carboxamide | SM: Intermediate 59 1.0 g, 91% yield. LC-MS: m/z $[M+H]^+$ = 336. |
| **83** | 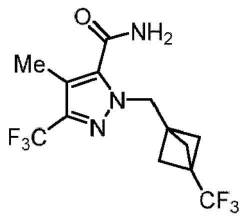 4-methyl-3-(trifluoromethyl)-1-((3-(trifluoromethyl)bicyclo[1.1.1] pentan-1-yl)methyl)-1H-pyrazole-5-carboxamide | SM: Intermediate 60 0.42 g, 70% yield. LC-MS: m/z $[M+H]^+$ = 342. |
| **84** | 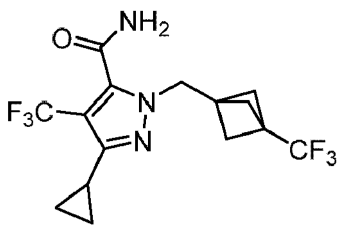 3-cyclopropyl-4-(trifluoromethyl)-1-((3-(trifluoromethyl)bicyclo [1.1.1]pentan-1-yl)methyl)-1H-pyrazole-5-carboxamide | SM: Intermediate 62 350 mg, 78% yield. LC-MS: m/z $[M+H]^+$ = 368. |
| **85** | 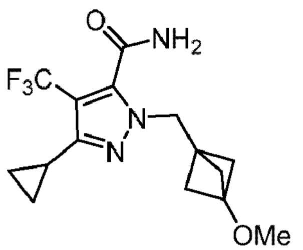 3-cyclopropyl-1-((3-methoxybicyclo[1.1.1]pentan-1-yl)methyl)-4-(trifluoromethyl)-1H-pyrazole-5-carboxamide | SM: Intermediate 63 250 mg, crude. LC-MS: m/z $[M+H]^+$ = 330. |
| **86** | 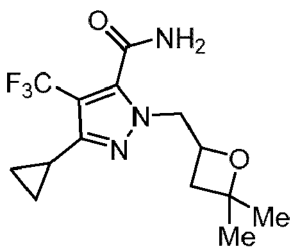 3-cyclopropyl-1-((4,4-dimethyloxetan-2-yl)methyl)-4-(trifluoromethyl)-1H-pyrazole-5-carboxamide | SM: Intermediate 64 810 mg, crude. LC-MS: m/z $[M+H]^+$ = 318. |

(continued)

| Intermediate | Structure and Name | Starting Materials (SM) and Data |
|---|---|---|
| **87** | 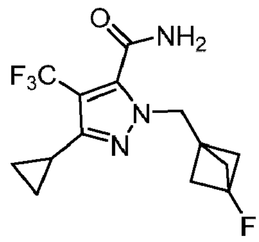<br>3-cyclopropyl-1-((3-fluorobicyclo[1.1.1]pentan-1-yl)methyl)-4-(trifluoromethyl)-1H-pyrazole-5-carboxamide | SM: Intermediate 65<br>250 mg, crude. LC-MS: m/z $[M+H]^+$ = 318. |
| **88** | 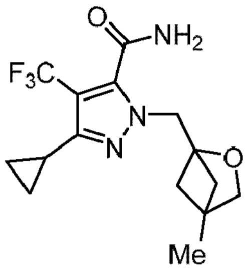<br>3-cyclopropyl-1-((4-methyl-2-oxabicyclo[2.1.1]hexan-1-yl)methyl)-4-(trifluoromethyl)-1H-pyrazole-5-carboxamide | SM: Intermediate 66<br>105 mg, 75% yield. LC-MS: m/z $[M+H]^+$ = 330. |

**Intermediate 89**

4-(3-methyl-4-(trifluoromethyl)-1-((3-(trifluoromethyl)bicyclo[1.1.1]pentan-1-yl)methyl)-1H-pyrazole-5-carboxamido)picolinic acid

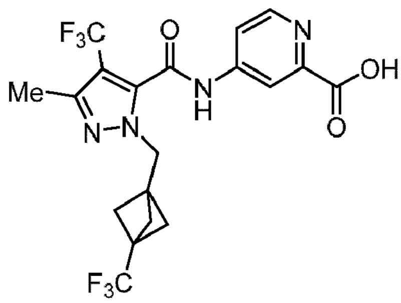

Step A: To a solution of 3-methyl-4-(trifluoromethyl)-1-((3-(trifluoromethyl)bicyclo[1.1.1]pentan-1-yl)methyl)-1H-pyrazole-5-carboxylic acid (Intermediate 61, 0.13 g, 0.38 mmol) in pyridine (3 mL) were added $POCl_3$ (0.06 mL, 0.57 mmol) and methyl 4-aminopyridine-2-carboxylate (0.09 g, 0.57 mmol) at 0 °C and the reaction mixture was stirred at rt for 2 h. The reaction mixture was concentrated under reduced pressure, diluted with ice water and extracted with EtOAc (3x50 mL). The combined organic layers were washed with brine and dried over $Na_2SO_4$. The organic layer was concentrated under reduced pressure to get a mixture of methyl (Z)-4-((chloro(3-methyl-4-(trifluoromethyl)-1-((3-(trifluoromethyl)bicyclo[1.1.1]pentan-1-yl)methyl)-1H-pyrazol-5-yl)methylene)amino)picolinate and methyl 4-(3-methyl-4-(trifluoromethyl)-1-((3-(trifluoromethyl)-bicyclo[1.1.1]pentan-1-yl)methyl)-1H-pyrazole-5-carboxamido)picolinate.

Step B: To the mixture of Step A (0.175 g, 0351 mmol) in THF (10 mL) was added NaOH (1M, 5 mL) at 0 °C and the reaction mixture was stirred at rt for 16 h. The reaction mixture was concentrated under reduced pressure, acidified with sat. $NaHSO_4$ solution to pH 2 and extracted with EtOAc (3x60 mL). The combined organic layers were washed with brine and dried over $Na_2SO_4$, then concentrated under reduced pressure to yield 4-(3-methyl-4-(trifluoromethyl)-1-((3-(trifluoromethyl)bicyclo[1.1.1]pentan-1-yl)methyl)-1H-pyrazole-5-carboxamido)picolinic acid which was directly used to next step without further purification (0.18 g, crude). LC-MS: m/z $[M+H]^+$ = 463.

## Examples

### Example 1

4-(1-((3-fluorobicyclo[1.1.1]pentan-1-yl)methyl)-3-methyl-4-(trifluoromethyl)-1H-pyrazole-5-carboxamido)picolinamide

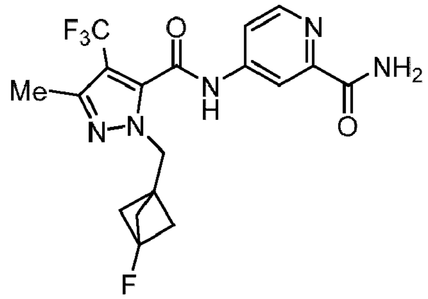

A mixture of 1-((3-fluorobicyclo[1.1.1]pentan-1-yl)methyl)-3-methyl-4-(trifluoromethyl)-1H-pyrazole-5-carboxamide (Intermediate 75, 275 mg, 0.94 mmol), 4-bromopicolinamide (283 mg, 27.9 mmol) and $Cs_2CO_3$ (916 mg, 2.82 mmol) in dioxane (20 mL) was degassed with argon for 15 min. CuI (54 mg, 0.28 mmol) and trans N,N'-dimethylcyclohexane-1,2-diamine (34 mg, 0.28 mmol) were added and the reaction mixture was stirred at 100 °C for 16 h. The reaction mixture was cooled to rt, filtered through Celite®, the Celite® pad was washed with EtOAc (25 mL). The filtrate was diluted with water (50 mL) and extracted with EtOAc (3x50 mL). The organic layers were washed with brine (50 mL), dried over $Na_2SO_4$ and concentrated under reduced pressure to get a residue which was purified by prep HPLC (Method A) to get 4-(1-((3-fluorobicyclo[1.1.1]pentan-1-yl)methyl)-3-methyl-4-(trifluoromethyl)-1H-pyrazole-5-carboxamido)picolinamide (80 mg, 20% yield). LC-MS: m/z $[M+H]^+$ = 412. $^1$H-NMR (400 MHz, DMSO-$d_6$) δ (ppm) = 11.45 (s, 1H), 8.57 (d, 1H), 8.31 (s, 1H), 8.13 (s, 1H), 7.79 (d, 1H), 7.70 (s, 1H), 4.50 (s, 2H,), 2.31 (s, 3H), 1.95-1.91 (m, 6H).

### Examples 2 to 9

The following Examples were prepared using a similar method as described for Example 1 from 4-bromopicolinamide and the corresponding amide and purified using HPLC methods indicated.

| Example No | Structure, Name and Starting Materials (SM) | Data |
|---|---|---|
| 2 | 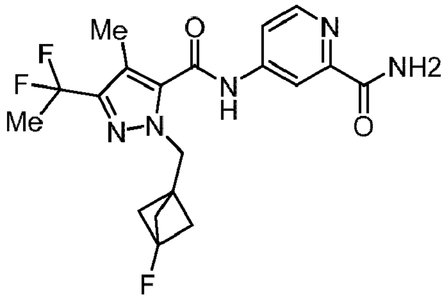<br>4-(3-(1,1-difluoroethyl)-1-((3-fluorobicyclo[1.1.1] pentan-1-yl)methyl)-4-methyl-1H-pyrazole-5-carboxamido)picolinamide<br>SM: Intermediate 76<br>HPLC: Method B | 130 mg, 21% yield. LC-MS: m/z $[M+H]^+$ = 407. $^1$H-NMR (400 MHz, DMSO-$d_6$) δ (ppm) = 11.00 (s, 1H), 8.56 (d, 1H), 8.38 (d, 1H), 8.10 (s, 1H), 7.85-7.83 (m, 1H), 7.66 (s, 1H), 4.63 (s, 2H), 2.26 (s, 3H), 2.08-1.98 (m, 3H), 1.92-1.91 (m, 6H). |

(continued)

| Example No | Structure, Name and Starting Materials (SM) | Data |
|---|---|---|
| **3** | 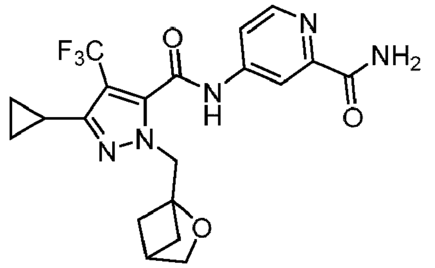 1-((2-oxabicyclo[2.1.1]hexan-1-yl)methyl)-3-cyclopropyl-4-(trifluoromethyl)-1H-pyrazole-5-carboxamide<br>SM: Intermediate 77<br>HPLC: Method C | 60 mg, 13%yield. LC-MS: m/z $[M+H]^+$ = 436. $^1$H-NMR (400 MHz, DMSO-$d_6$) δ (ppm) = 11.42 (s, 1H), 8.57 (d, 1H), 8.33 (d, 1H), 8.12 (d, 1H), 7.79-7.77 (m, 1H), 7.68 (d, 1H), 4.46 (m, 2H), 3.58 (s, 2H), 2.83-2.82 (m, 1H), 1.97-1.90 (m, 1H), 1.71-1.68 (m, 2H), 1.30-1.27 (m, 2H), 0.99-0.94 (m, 2H), 0.85-0.83 (m, 2H). |
| **4** | 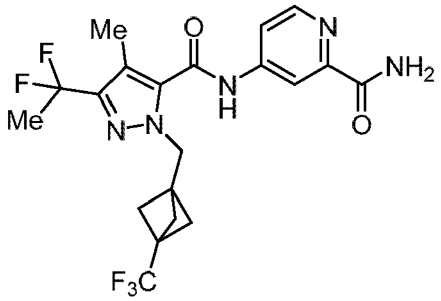 3-(1,1-4-(3-(1,1-difluoroethyl)-4-methyl-1-((3-(trifluoromethyl)bicyclo[1.1.1]pentan-1-yl) methyl)-1H-pyrazole-5-carboxamido)picolinamide<br>SM: Intermediate 78<br>HPLC: Method C | 120 mg, 29% yield. LC-MS: m/z $[M+H]^+$ = 458. $^1$H-NMR (400 MHz, DMSO-$d_6$) δ (ppm) = 10.99 (s, 1H), 8.56 (d, 1H), 8.38 (d, 1H), 8.10 (s, 1H), 7.85-7.83 (m, 1H), 7.66 (s, 1H), 4.48 (s, 2H), 2.25 (s, 3H), 2.03 (t, 3H), 1.83 (s, 6H). |
| **5** | 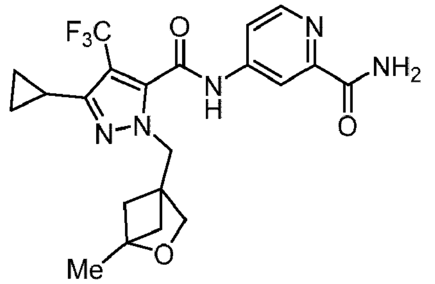 4-(3-cyclopropyl-1-((1-methyl-2-oxabicyclo[2.1.1] hexan-4-yl)methyl)-4-(trifluoromethyl)-1H-pyrazole-5-carboxamido)picolinamide<br>SM: Intermediate 79<br>HPLC: Method C | 60 mg, 26% yield. LC-MS: m/z $[M+H]^+$ = 450. $^1$H-NMR (400 MHz, DMSO-$d_6$) δ (ppm) = 11.46 (s, 1H), 8.58 (d, 1H), 8.32 (s, 1H), 8.11 (s, 1H), 7.79-7.77 (m, 1H), 7.68 (s, 1H), 4.44 (s, 2H), 3.42 (s, 2H), 1.95-1.91 (m, 1H), 1.52-1.48 (m, 2H), 1.40-1.34 (m, 2H), 1.26 (s, 3H), 1.00-0.95 (m, 2H), 0.85-0.83 (m, 2H). |

(continued)

| Example No | Structure, Name and Starting Materials (SM) | Data |
|---|---|---|
| **6** | 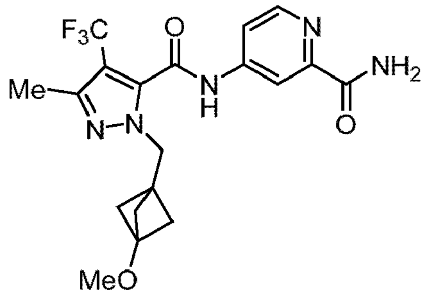 4-(1-((3-methoxybicyclo[1.1.1]pentan-1-yl)methyl)-3-methyl-4-(trifluoromethyl)-1H-pyrazole-5-carboxamido)picolinamide<br>SM: Intermediate 80<br>HPLC: Method D | 10 mg, 7% yield. LC-MS: m/z $[M+H]^+$ = 424. $^1H$-NMR (400 MHz, DMSO-$d_6$) δ (ppm) = 11.44 (s, 1H), 8.58 (d, 1H), 8.31 (s, 1H), 8.13 (s, 1H), 7.79 (d, 1H), 7.69 (s, 1H), 4.41 (s, 2H), 3.11 (s, 3H), 2.32 (s, 3H), 2.32 (s, 3H), 1.69 (s, 6H) |
| **7** | 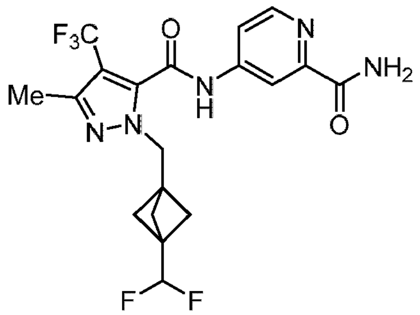 4-(1-((3-(difluoromethyl)bicyclo[1.1.1]pentan-1-yl) methyl)-3-methyl-4-(trifluoromethyl)-1H-pyrazole-5-carboxamido)picolinamide<br>SM: Intermediate 81<br>HPLC: Method C | 12 mg, 8% yield. LC-MS: m/z $[M+H]^+$ = 444. $^1H$-NMR (400 MHz, DMSO-$d_6$) δ (ppm) = 11.44 (bs, 1H), 8.58 (d, 1H), 8.32 (s, 1H), 8.13 (s, 1H), 7.80-7.78 (m, 1H), 7.69 (s, 1H), 6.11 (t, 1H), 4.32 (s, 2H), 2.31 (s, 3H), 1.69 (s, 6H). |
| **8** | 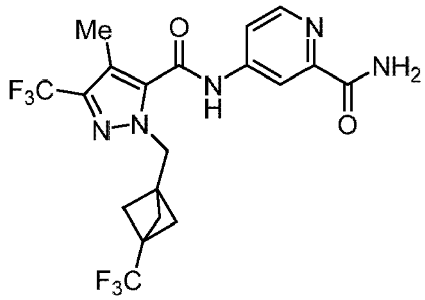 4-(4-methyl-3-(trifluoromethyl)-1-((3-(trifluoromethyl) bicyclo[1.1.1]pentan-1-yl)methyl)-1H-pyrazole-5-carboxamido)picolinamide<br>SM: Intermediate 83<br>HPLC: Method E | 125 mg, 22% yield. LC-MS: m/z $[M+H]^+$ = 462. $^1H$ NMR (400 MHz, DMSO-$d_6$): δ (ppm) = 11.07 (s, 1H), 8.57 (d, 1H), 8.36 (d, 1H), 8.11 (s, 1H), 7.84-7.82 (m, 1H), 7.66 (s, 1H), 4.57 (s, 2H), 2.26 (s, 3H), 1.40 (s, 6H). |

(continued)

| Example No | Structure, Name and Starting Materials (SM) | Data |
|---|---|---|
| **9** | 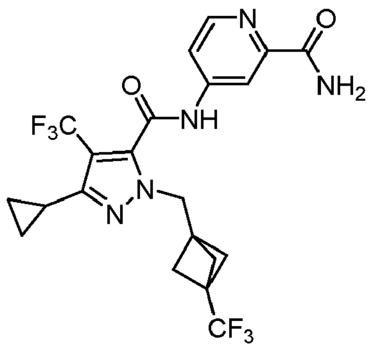<br>4-(3-cyclopropyl-4-(trifluoromethyl)-1-((3-(trifluoromethyl)bicyclo[1.1.1]pentan-1-yl)methyl)-1H-pyrazole-5-carboxamido)picolinamide<br>SM: Intermediate 84<br>HPLC: Method E | 0.20 g, 46% yield. LC-MS: m/z $[M+H]^+$ = 488. $^1H$ NMR (400 MHz, DMSO-$d_6$): δ (ppm) = 11.46 (s, 1H), 8.59 (d, 1H), 8.32 (s, 1H), 8.12 (s, 1H), 7.79-7.77 (m, 1H), 7.68 (s, 1H), 4.30 (s, 2H), 1.96-1.93 (m, 1H), 1.84 (s, 6H), 1.00-0.96 (m, 2H), 0.86-0.83 (m, 2H). |

**Example 10**

4-(1-((4,4-difluoro-1,2-dimethylcyclopentyl)methyl)-3-(1,1-difluoroethyl)-4-methyl-1H-pyrazole-5-carboxamido)picolinamide, synthesized in the forms of:

4-(1-((4,4-difluoro-1,2-dimethylcyclopentyl)methyl)-3-(1,1-difluoroethyl)-4-methyl-1H-pyrazole-5-carboxamido)picolinamide, isomer 1 (10a)

4-(1-((4,4-difluoro-1,2-dimethylcyclopentyl)methyl)-3-(1,1-difluoroethyl)-4-methyl-1H-pyrazole-5-carboxamido)picolinamide, isomer 2 (10b)

4-(1-((4,4-difluoro-1,2-dimethylcyclopentyl)methyl)-3-(1,1-difluoroethyl)-4-methyl-1H-pyrazole-5-carboxamido)picolinamide, isomer 3 (10c)

4-(1-((4,4-difluoro-1,2-dimethylcyclopentyl)methyl)-3-(1,1-difluoroethyl)-4-methyl-1H-pyrazole-5-carboxamido)picolinamide, isomer 4 (10d)

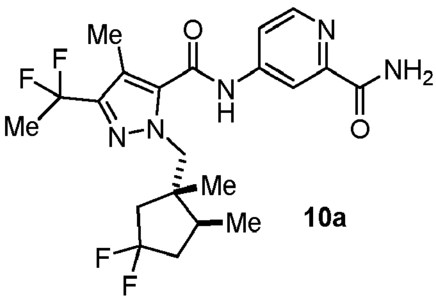

**10a**

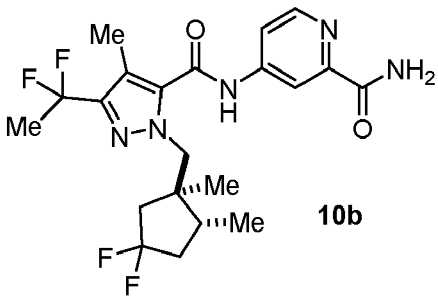

**10b**

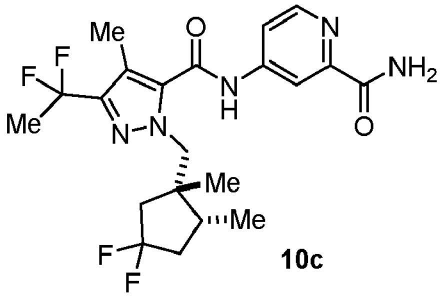

10c

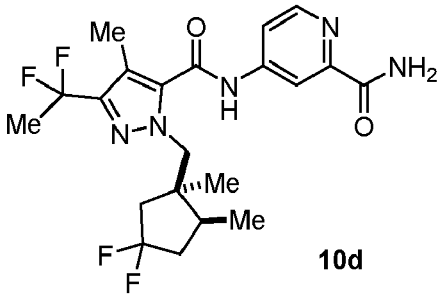

10d

Step A: To a solution of 1-((4,4-difluoro-1,2-dimethylcyclopentyl)methyl)-3-(1,1-difluoroethyl)-4-methyl-1H-pyrazole-5-carboxamide (Intermediate 82, 450 mg, 1.34 mmol) in dioxane (9 mL) in a sealed tube was added 4-bromopicolinamide (324 mg, 1.61 mmol) and $Cs_2CO_3$ (1.09 g, 3.36 mmol) at rt. The reaction mixture was degassed with argon for 10 min followed by addition of trans N,N-dimethyl 1,2-diamino cyclohexane (57.2 mg, 0.40 mmol) and Copper (I) trifluoromethane sulfonate benzene complex (169 mg, 0.33 mmol) at rt. The reaction mixture was stirred at 85 °C for 16 h. The reaction mixture was cooled to rt, filtered over Celite® which was washed with EtOAc (30 mL). The filtrate was concentrated under reduced pressure to get a residue which was purified by Grace chromatography (see under HPLC conditions) to afford 4-(1-((4,4-difluoro-1,2-dimethylcyclopentyl)methyl)-3-(1,1-difluoroethyl)-4-methyl-1H-pyrazole-5-carboxamido)picolinamide (240 mg, 39%).

Step B: This mixture was separated by prep-SFC using a Chiralcel OJ-H (250×30mm, 5 μm) column, eluting with 10% MeOH isocratic gradient, at 60 g/min, to give 105 mg of diastereomer 1 and 85 mg of diastereomer 2.

Diastereomer 1 was separated into its enantiomers by prep-SFC using a Chiralpak IG (250×30mm, 5 μm) column, eluting with 10% (0.5% Diethylamine in MeOH) isocratic gradient, at 60 g/min, (Method 1), to give 27 and 20 mg of enantiomers Peak 1, Example 10a and Peak 2, Example 10b, respectively.

Diastereomer 2 was separated into its enantiomers by prep-HPLC using a Lux Amylose-3 (250×30mm, 5 μm) column, eluting with n-Hex: IPA (95:05), at 42.0 mL/min. (Method 2), to give 27 and 25 mg of enantiomers Peak 3, Example 10c and Peak 4, Example 10d, respectively.

| Example | Data: |
|---|---|
| **10** | 27 mg, chiral purity: 99.9%. Retention time: 1.86 min (Method 1).<br>LC-MS: m/z $[M+H]^+$ = 456. $^1H$ NMR (400 MHz, DMSO-$d_6$): δ (ppm) = 11.12 (s, 1H), 8.56 (d, 1H), 8.37 (d, 1H), 8.10 (d, 1H), 7.85-7.83 (dd, 1H), 7.66 (d, 1H), 4.26 (s, 2H), 2.36-2.19 (m, 5H), 2.09-1.85 (m, 6H), 0.82 (s, 3H), 0.78 (d, 3H). |
| **11** | 20 mg, chiral purity: 98.1%. Retention time: 2.20 min (Method 1).<br>LC-MS: m/z $[M+H]^+$ =: 456. $^1H$ NMR (400 MHz, DMSO-$d_6$): δ (ppm) = 11.07 (s, 1H), 8.56 (d, 1H), 8.35 (d, 1H), 8.09 (d, 1H), 7.85-7.83 (dd, 1H), 7.65 (d, 1H), 4.32-4.22 (q, 2H), 2.51 (m, 1H), 2.41-2.30 (m, 1H), 2.35 (s, 3H), 2.10-1.93 (m, 5H), 1.83-1.70 (m, 1H), 1.0 (d, 3H), 0.85 (s, 3H). |
| **12** | 27 mg, chiral purity: 99.9%. Retention time: 15.83 min (Method 2).<br>LC-MS: m/z $[M+H]^+$ = 456. $^1H$ NMR (400 MHz, DMSO-$d_6$): δ (ppm) = 11.13 (s, 1H), 8.56 (d, 1H), 8.37 (d, 1H), 8.10 (d, 1H), 7.85-7.83 (dd, 1H), 7.66 (d, 1H), 4.26 (s, 2H), 2.36-2.19 (m, 5H), 2.09-1.85 (m, 6H), 0.82 (s, 3H), 0.78 (d, 3H). |
| **13** | 25 mg, chiral purity: 98.6%. Retention time: 19.57 min (Method 2).<br>LC-MS: m/z $[M+H]^+$ = 456. $^1H$ NMR (400 MHz, DMSO-$d_6$): δ (ppm) = 11.07 (s, 1H), 8.56 (d, 1H), 8.35 (d, 1H), 8.09 (d, 1H), 7.85-7.83 (dd, 1H), 7.65 (d, 1H), 4.32-4.22 (q, 2H), 2.51 (m, 1H), 2.41-2.30 (m, 1H), 2.35 (s, 3H), 2.10-1.93 (m, 5H), 1.83-1.70 (m, 1H), 1.0 (d, 3H), 0.85 (s, 3H). |

**Examples 14 to 17**

The following Examples were prepared using a similar method as described for Example 10-13 step A, from 4-bromopicolinamide and the corresponding amide.

| Example | Structure, Name and Starting Materials (SM) | Data |
|---|---|---|
| **14** | 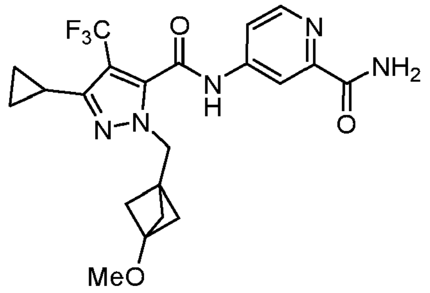<br>4-(3-cyclopropyl-1-((3-methoxybicyclo[1.1.1]pentan-1-yl)methyl)-4-(trifluoromethyl)-1H-pyrazole-5-carboxamido)picolinamide<br>SM: Intermediate 85 | 52 mg. LC-MS: m/z $[M+H]^+$ = 450. $^1$H-NMR (400 MHz, DMSO-$d_6$): δ (ppm) = 11.46 (s, 1H), 8.57 (d, 1H), 8.32 (s, 1H), 8.11 (s, 1H), 7.78 (dd, 1H), 7.68 (s, 1H), 4.36 (s, 2H), 3.11 (s, 3H), 1.97-1.90 (m, 1H), 1.67 (s, 6H), 1.00-0.95 (m, 2H), 0.87-0.82 (m, 2H). |
| **15** | 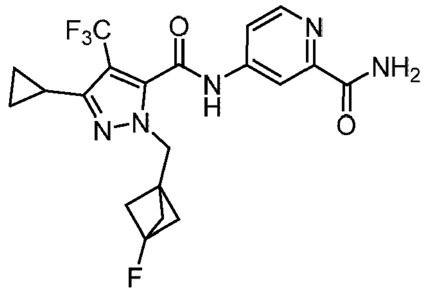<br>4-(3-cyclopropyl-1-((3-fluorobicyclo[1.1.1]pentan-1-yl) methyl)-4-(trifluoromethyl)-1H-pyrazole-5-carboxamido)picolinamide<br>SM: Intermediate 87 | 50 mg. LC-MS: m/z $[M+H]^+$ = 438. $^1$H-NMR (500 MHz, DMSO-$d_6$): δ (ppm) = 11.46 (s, 1H), 8.58 (d, 1H), 8.3 1 (s, 1H), 8.12 (s, 1H), 7.78 (d, 1H), 7.68 (s, 1H), 4.44 (s, 2H), 1.96-1.90 (m, 7H), 1.01-0.95 (m, 2H), 0.87-0.82 (m, 2H). |
| **16** | 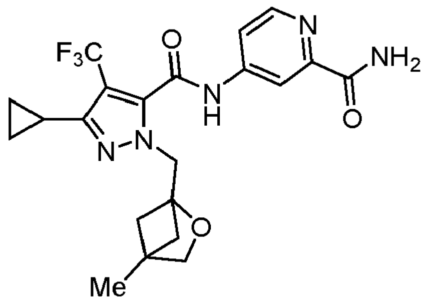<br>4-(3-cyclopropyl-1-((4-methyl-2-oxabicyclo[2.1.1] hexan-1-yl)methyl)-4-(trifluoromethyl)-1H-pyrazole-5-carboxamido)picolinamide<br>SM: Intermediate 88<br>HPLC: Method G | 15 mg, 22% yield. LC-MS: m/z $[M+H]^+$ = 450. $^1$HNMR (400 MHz, DMSO-$d_6$): δ (ppm) = 11.40 (s, 1H), 8.55 (d, 1H), 8.30 (bs, 1H), 8.06 (bs, 1H), 7.80 - 7.50 (m, 2H), 4.45 (s, 2H), 3.38 (s, 2H), 1.96 - 1.89 (s, 1H), 1.54 - 1.44 (m, 2H), 1.36 - 1.33 (m, 2H), 1.24 - 1.20 (m, 3H), 0.98 - 0.88 (s, 2H), 0.86 - 0.80 (m, 2H). |

**Example 17**

4-(3-cyclopropyl-1-((4,4-dimethyloxetan-2-yl)methyl)-4-(trifluoromethyl)-1H-pyrazole-5-carboxamido)picolinamide, synthesized in the form of:

4-(3-cyclopropyl-1-((4,4-dimethyloxetan-2-yl)methyl)-4-(trifluoromethyl)-1H-pyrazole-5-carboxamido)picolinamide, enantiomer 1 (17a)

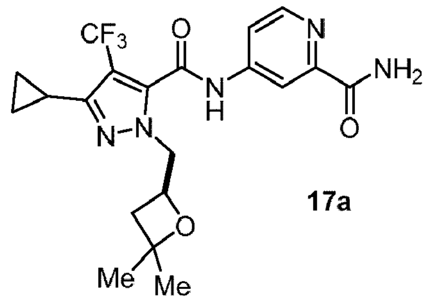

**17a**

4-(3-Cyclopropyl-1-((4,4-dimethyloxetan-2-yl)methyl)-4-(trifluoromethyl)-1H-pyrazole-5-carboxamido)picolinamide was prepared using a similar method to that described for Example 10 step A, from 4-bromopicolinamide and 3-cyclopropyl-1-((4,4-dimethyloxetan-2-yl)methyl)-4-(trifluoromethyl)-1H-pyrazole-5-carboxamide (Intermediate 86). This racemate was further purified by SFC (Lux Cellulose-4 (250 × 30 mm, 5 μm) column, eluting 10% MeOH isocratic gradient, to give Peak 1, enantiomer 1, 90 mg of 4-(3-cyclopropyl-1-((4,4-dimethyloxetan-2-yl)methyl)-4-(trifluoromethyl)-1H-pyrazole-5-carboxamido)picolinamide (17a).

Analytical HPLC: X-Bridge C18 (150 × 4.6mm, 3.5 μm) column, Mobile phase: A = 10 mM $NH_4OAc$, B = 100% MeCN, Flow rate of 1.0 mL/min, $R_t$ = 10.33 min.

LC-MS: m/z $[M+H]^+$ = 438. $^1$H-NMR (500 MHz, DMSO-$d_6$): δ (ppm) = 11.48 (s, 1H), 8.55 (bs, 1H), 8.31 (bs, 1H), 8.11 (bs, 1H), 7.79 (bs, 1H), 7.67 (bs, 1H), 4.71-4.66 (m, 1H), 4.37-4.30 (m, 2H), 2.39-2.35 (m, 1H), 2.18-2.16 (m, 1H), 1.97-1.92 (m, 1H), 1.29 (s, 3H), 1.02 (s, 3H), 0.97-0.95 (m, 2H), 0.85-0.83 (m, 2H).

**Example 18**

4-(3-methyl-4-(trifluoromethyl)-1-((3-(trifluoromethyl)bicyclo[1.1.1]pentan-1-yl)methyl)-1H-pyrazole-5-carboxamido)picolinamide

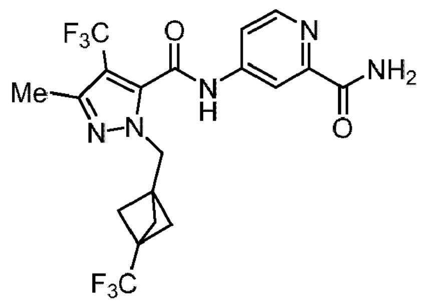

To a solution of 4-(3-methyl-4-(trifluoromethyl)-1-((3-(trifluoromethyl)bicyclo[1.1.1]pentan-1-yl)methyl)-1H-pyrazole-5-carboxamido)picolinic acid (Intermediate 89, 0.34 g, 0.39 mmol) in DMF (3 mL) were added TEA (0.16 mL, 1.17 mmol) and ethyl chloroformate (0.05 mL, 0.58 mmol) at -10 °C. The reaction mixture was stirred for 2 h. $NH_4OH$ (5 mL) was added at 0 °C and the reaction mixture was stirred at rt for 16 h, then diluted with ice water and extracted with EtOAc (3x50 mL). The combined organic layer was washed with brine (30 mL), dried over $Na_2SO_4$, concentrated under reduced pressure, then purified by HPLC (Method E) to obtain 4-(3-methyl-4-(trifluoromethyl)-1-((3-(trifluoromethyl)bicyclo[1.1.1]pentan-1-yl)methyl)-1H-pyrazole-5-carboxamido)picolinamide (52 mg, 29%). LC-MS: m/z $[M+H]^+$ = 462. $^1$H NMR (400 MHz, DMSO-$d_6$): δ (ppm) = 11.43 (s,1H), 8.59-8.58 (m, 1H), 8.32 (s, 1H), 8.11 (s, 1H), 7.80-7.78 (m, 1H), 7.67 (s, 1H), 4.35 (s, 2H), 2.31 (s, 3H), 1.87 (s, 6H).

**Examples 19 to 21**

The following Examples were prepared using a similar method as described for Example 10, step A, from 4-bromo-2-(methylsulfonyl)pyridine and the corresponding amide.

| Example | Structure, Name and Starting Materials (SM) | Data |
|---|---|---|
| **19** | 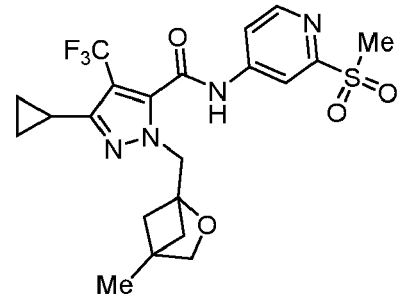<br>3-cyclopropyl-1-((4-methyl-2-oxabicyclo[2.1.1]hexan-1-yl)methyl)-N-(2-(methylsulfonyl)pyridin-4-yl)-4-(trifluoromethyl)-1H-pyrazole-5 -carboxamide<br>SM: Intermediate 88<br>HPLC: Method G | 18 mg; 24% yield. LC-MS: m/z $[M+H]^+$ = 485. $^1$HNMR (400 MHz, DMSO-$d_6$): $\delta$ (ppm) = 11.66 (s, 1H), 8.71 (d, 1H), 8.37 (s, 1H), 7.84 (d, 1H), 4.44 (s, 2H), 3.38 (s, 2H), 3.29 (s, 3H), 1.98 - 1.91 (m, 1H), 1.54 - 1.50 (m, 2H), 1.38 - 1.32 (m, 2H), 1.22 (s, 3H), 0.99 - 0.93 (m, 2H), 0.85 - 0.81 (m, 2H). |
| **20** | 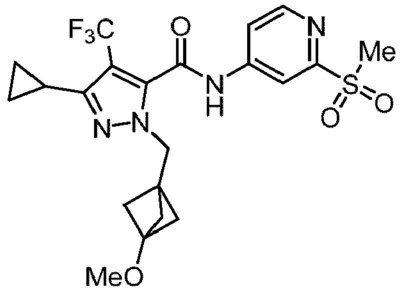<br>3-cyclopropyl-1-((3-methoxybicyclo[1.1.1]pentan-1-yl) methyl)-N-(2-(methylsulfonyl)pyridin-4-yl)-4-(trifluoromethyl)-1H-pyrazole-5-carboxamide<br>SM: Intermediate 85 | 72 mg; 48% yield. LC-MS: m/z $[M+H]^+$ = 485. $^1$H-NMR (400 MHz, DMSO-$d_6$): $\delta$ (ppm) = 11.72 (s, 1H), 8.73 (d, 1H), 8.36 (s, 1H), 7.85 (s, 1H), 4.38 (s, 2H), 3.29 (s, 3H), 3.11 (s, 3H), 1.98-1.91 (m, 1H), 1.67 (s, 6H), 1.00-0.95 (m, 2H), 0.87-0.82 (m, 2H). |
| **21** | 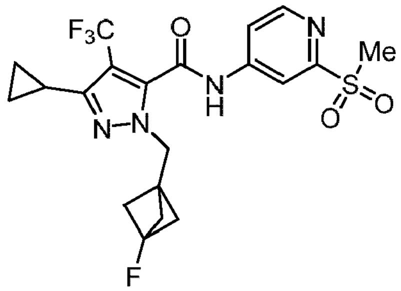<br>3-cyclopropyl-1-((3-fluorobicyclo[1.1.1]pentan-1-yl) methyl)-N-(2-(methylsulfonyl)pyridin-4-yl)-4-(trifluoromethyl)-1H-pyrazole-5-carboxamide<br>SM: Intermediate 87 | 49 mg; 33% yield. LC-MS: m/z $[M+H]^+$ = 473. $^1$H-NMR (500 MHz, DMSO-$d_6$): $\delta$ (ppm) = 11.73 (s, 1H), 8.73 (d, 1H), 8.36 (s, 1H), 7.85 (s, 1H), 4.46 (s, 2H), 3.29 (s, 3H), 1.96-1.89 (m, 7H), 1.01-0.95 (m, 2H), 0.87-0.82 (m, 2H). |

**Example 22**

3-cyclopropyl-1-((4,4-dimethyloxetan-2-yl)methyl)-N-(2-(methylsulfonyl)pyridin-4-yl)-4-(trifluoromethyl)-1H-pvrazole-5-carboxamide, synthesized in the form of:

3-cyclopropyl-1-((4,4-dimethyloxetan-2-yl)methyl)-N-(2-(methylsulfonyl)pyridin-4-yl)-4-(trifluoromethyl)-1H-pvrazole-5-carboxamide, enantiomer 1, (22a)

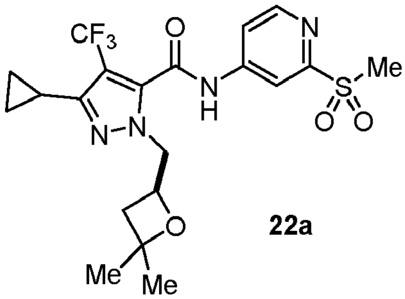

**22a**

3-Cyclopropyl-1-((4,4-dimethyloxetan-2-yl)methyl)-N-(2-(methylsulfonyl)pyridin-4-yl)-4-(trifluoromethyl)-1H-pyrazole-5-carboxamide was prepared using a similar method as described for Example 10, step A, from 4-bromo-2-(methylsulfonyl)pyridine and 3-cyclopropyl-1-((4,4-dimethyloxetan-2-yl)methyl)-4-(trifluoromethyl)-1H-pyrazole-5-carboxamide (Intermediate 86), 380 mg racemate, 51% yield. This was purified by Prep SFC (Lux Cellulose-4 (250 × 30 mm, 5 μm) column, eluting 25% MeOH isocratic gradient, at 60 g/min, to give Peak 1, enantiomer 1, 135 mg of 3-cyclopropyl-1-((4,4-dimethyloxetan-2-yl)methyl)-N-(2-(methylsulfonyl)pyridin-4-yl)-4-(trifluoromethyl)-1H-pyrazole-5-carboxamide (22a).

Analytical HPLC: X-Bridge C18 (150 × 4.6mm, 3.5μm) column, Mobile phase: A = 0.05% TFA (aq), B = 100% MeCN, Flow rate of 1.0 mL/min, Retention time: 11.06 min.

LC-MS: m/z $[M+H]^+$ = 473. $^1$H-NMR (500 MHz, DMSO-$d_6$): δ (ppm) = 11.74 (s, 1H), 8.70 (s, 1H), 8.37 (s, 1H), 7.84 (s, 1H), 4.69-4.64 (m, 1H), 4.39-4.35 (m, 2H), 3.28 (s, 3H), 2.39-2.35 (m, 1H), 2.18-2.14 (m, 1H), 1.96-1.94 (m, 1H), 1.30 (s, 3H), 1.02 (s, 3H), 0.97-0.95 (m, 2H), 0.85-0.83 (m, 2H).

**Example 23**

3-cyclopropyl-1-((1-methyl-2-oxabicyclo[2.1.1]hexan-4-yl)methyl)-N-(2-(methylsulfonyl)pyridin-4-yl)-4-(trifluoromethyl)-1H-pyrazole-5-carboxamide

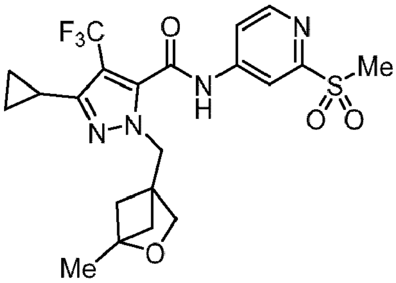

To a solution of 3-cyclopropyl-1-((1-methyl-2-oxabicyclo[2.1.1]hexan-4-yl)methyl)-4-(trifluoromethyl)-1H-pyrazole-5-carboxylic acid (Intermediate 53, 180 mg, 0.54 mmol) in pyridine (10 mL) was added 2-(methylsulfonyl)pyridin-4-amine (102 mg, 0.82 mmol) followed by $POCl_3$ (152 μL, 1.63 mmol) at 0 °C. The reaction mixture was stirred at rt for 16 h. The reaction mixture was diluted with EtOAc (250 mL), washed with sat. Na-$HCO_3$ solution (50 mL), water (50 mL), brine (50 mL) and dried over $Na_2SO_4$. The organic layer was concentrated under reduced pressure to get a residue which was purified by HPLC (Method K) to yield 3-cyclopropyl-1-((1-methyl-2-oxabicyclo[2.1.1]hexan-4-yl)methyl)-N-(2-(methylsulfonyl)pyridin-4-yl)-4-(trifluoromethyl)-1H-pyrazole-5-carboxamide. The reaction was repeated twice at 50 mg scale and the total yield across these experiments was 36 mg, 11%yield. LC-MS: m/z $[M+H]^+$ = 485. $^1$H-NMR (400 MHz, DMSO-$d_6$) δ (ppm) = 11.74 (s, 1H), 8.73 (d, 1H), 8.36 (s, 1H), 7.85 (d, 1H), 4.45 (s, 2H), 3.43 (s, 2H), 3.23 (s, 3H), 1.96-1.93 (m, 1H), 1.49 (d, 2H), 1.38 (d, 2H), 1.27 (s, 3H), 1.00-0.95 (m, 2H), 0.84-0.83 (m, 2H).

**Example 24**

1-((2-oxabicyclo[2.1.1]hexan-1-yl)methyl)-3-cyclopropyl-N-(2-(methylsulfonyl)pyridin-4-yl)-4-(trifluoromethyl)-1H-pyrazole-5-carboxamide

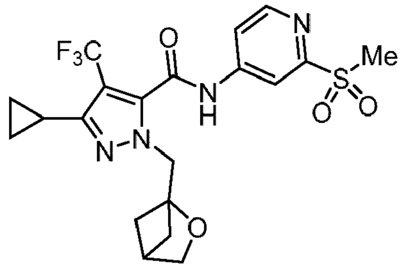

To a solution of 1-((2-oxabicyclo[2.1.1]hexan-1-yl)methyl)-3-cyclopropyl-4-(trifluoromethyl)-1H-pyrazole-5-carboxylic acid (Intermediate 55, 240 mg, 0.97 mmol) in pyridine (10 mL) was added $POCl_3$ (0.302 mL, 2.90 mmol) dropwise at 0 °C followed by 2-(methanesulfonyl)pyridin-4-amine (248 mg, 1.45 mmol). The reaction mixture was stirred at rt for 2 h, then quenched with water (50 mL) and extracted with EtOAc (3 × 100 mL). The combined organic layers were washed with cold brine (50 mL) and dried over $Na_2SO_4$. The organic layer was concentrated to get a residue which was dissolved in a mixture of THF (10 mL) and 2(N) NaOH (2N, 10 mL). The reaction mixture was stirred at rt for 16h, then concentrated under reduced pressure, and extracted with EtOAc (3 × 100 mL). The combined organic layers were washed with cold brine and dried over $Na_2SO_4$, then concentrated to get a residue which was purified by reverse phase chromatography (Column: YMC Trial C18 (250 × 20.0 mm, 5μ), flow rate of 16 mL/min. Mobile phase: A = 20 mM $NH_4CO_3$ in water, B=MeCN; Gradient: 30% B to 55% B in 3 min, then 85% B in 22 min, to yield 1-((2-oxabicyclo[2.1.1]hexan-1-yl)methyl)-3-cyclopropyl-N-(2-(methylsulfonyl)pyridin-4-yl)-4-(trifluoromethyl)-1H-pyrazole-5-carboxamide (23 mg, 5% yield). LC-MS: m/z $[M+H]^+$ = 471. $^1$H-NMR (400 MHz, DMSO-$d_6$) δ (ppm) = 11.67 (s, 1H), 8.71 (d, 1H), 8.38 (s, 1H), 8.09 (s, 1H), 7.85(d, 1H), 4.47 (s, 2H), 3.58 (s, 2H), 2.83 (s, 1H), 1.94 (s, 1H), 1.72 (m, 2H), 1.27-1.24 (m, 2H), 0.98-0.96 (m, 2H), 0.84-0.83 (m, 2H).

**Examples 25 to 29**

The following Examples were prepared using a similar method as described for Example 24 from 4-bromo-2-(methylsulfonyl)pyridine and the corresponding acid.

| Example | Structure, Name and Starting Materials (SM) | Data |
|---|---|---|
| 25 | 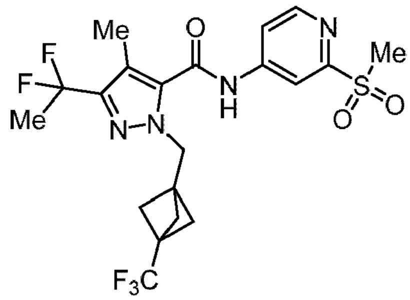 3-(1,1-difluoroethyl)-4-methyl-N-(2-(methylsulfonyl) pyridin-4-yl)-1 -((3 -(trifluoromethyl)bicyclo[1.1.1] pentan-1-yl)methyl)-1H-pyrazole-5-carboxamide SM: Intermediate 56 | HPLC: Method C<br>184 mg, 21% yield. LC-MS: m/z $[M+H]^+$ = 493. $^1$H-NMR (400 MHz, DMSO-$d_6$) δ (ppm) = 11.24 (s, 1H), 8.70 (d, 1H), 8.24 (s, 1H), 7.90 (d, 1H), 4.46 (s, 2H), 3.29 (s, 3H), 2.26 (s, 3H), 2.03 (t, 3H), 1.83 (s, 6H). |

(continued)

| Example | Structure, Name and Starting Materials (SM) | Data |
|---|---|---|
| **26** | 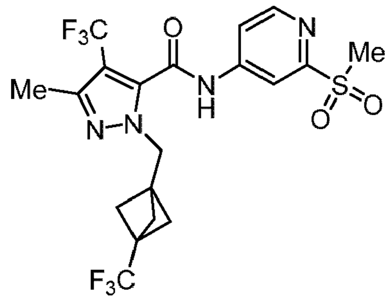 3-methyl-N-(2-(methylsulfonyl)pyridin-4-yl)-4-(trifluoromethyl)-1-((3-(trifluoromethyl)bicyclo [1.1.1]pentan-1-yl)methyl)-1H-pyrazole-5-carboxamide<br>SM: Intermediate 61 | HPLC: Method E<br>0.05 g, 34% yield. LC-MS: m/z $[M+H]^+$ = 497. 1H NMR (400 MHz, DMSO-$d_6$): $\delta$ (ppm) = 11.70 (s, 1H), 8.74-8.72 (m, 1H), 8.37-8.36 (m, 1H), 7.86-7.84 (m, 1H), 4.37 (s, 2H), 3.30 (s, 3H), 2.32 (s, 3H), 1.87 (s, 6H). |
| **27** | 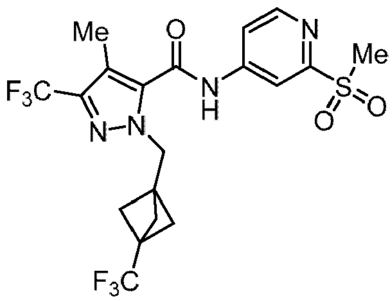 4-methyl-N-(2-(methylsulfonyl)pyridin-4-yl)-3-(trifluoromethyl)-1-((3-(trifluoromethyl)bicyclo [1.1.1]pentan-1-yl)methyl)-1H-pyrazole-5-carboxamide<br>SM: Intermediate 60 | Purification: CombiFlash chromatography (silica gel, 0-80% EtOAc/Hex)<br>52 mg, 64% yield. LC-MS: m/z $[M+H]^+$ = 497. $^1$H NMR (400 MHz, DMSO-$d_6$): $\delta$ (ppm) = 11.31 (s, 1H), 8.73 (d, 1H), 8.42 (d, 1H), 7.91-7.89 (m, 1H), 4.57 (s, 2H), 3.31 (s, 3H), 2.27 (s, 3H), 1.84 (s, 6H). |
| **28** | 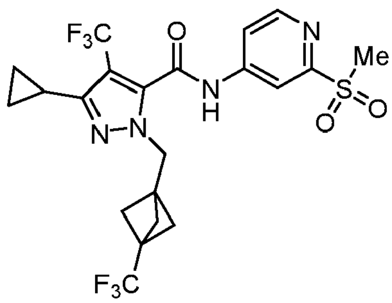 3-cyclopropyl-N-(2-(methylsulfonyl)pyridin-4-yl)-4-(trifluoromethyl)-1-((3-(trifluoromethyl)bicyclo [1.1.1]pentan-1-yl)methyl)-1H-pyrazole-5-carboxamide<br>SM: Intermediate 62 | HPLC: Method H<br>18 g, 36% yield. LC-MS: m/z $[M+H]^+$ = 523. $^1$H NMR (400 MHz, DMSO-$d_6$): $\delta$ (ppm) = 11.71 (s, 1H), 8.73 (d, 1H), 8.37 (s, 1H), 7.85 (d, 1H), 4.32 (s, 2H), 3.28 (s, 3H), 1.99-1.95 (m, 1H), 1.85 (s, 6H), 0.99-0.97 (m, 2H), 0.92-0.84 (m, 2H). |

(continued)

| Example | Structure, Name and Starting Materials (SM) | Data |
|---|---|---|
| **29** | 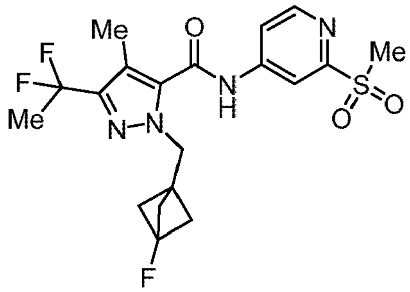 3-(1,1-difluoroethyl)-1-((3-fluorobicyclo[1.1.1] pentan-1-yl)methyl)-4-methyl-N-(2-(methylsulfonyl) pyridin-4-yl)-1H-pyrazole-5-carboxamide<br>SM: Intermediate 54 | HPLC: Method C<br>120 mg, 37% yield. LC-MS: m/z $[M+H]^+$ = 443. $^1$H-NMR (400 MHz, DMSO-$d_6$): δ (ppm) = 11.26 (s, 1H), 8.71 (d, 1H), 8.42 (s, 1H), 7.92-7.90 (m, 1H), 4.63 (s, 2H), 3.29 (s, 3H), 2.26 (s, 3H), 2.06-1.98 (m, 3H), 1.92-1.91 (m, 6H). |

**Example 30**

1-((2-oxabicyclo[2.1.1]hexan-1-yl)methyl)-N-(3-sulfamoylphenyl)-4-(trifluoromethyl)-1H-pyrazole-5-carboxamide

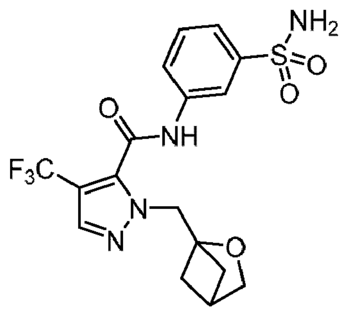

To a solution of 1-((2-oxabicyclo[2.1.1]hexan-1-yl)methyl)-4-(trifluoromethyl)-1H-pyrazole-5-carboxylic acid (Intermediate 67, 30 mg, 0.10 mmol) and 3-aminobenzenesulfonamide (22.4 mg, 0.13 mmol) in DMF (5 mL) was added HATU (82.6 mg, 0.21 mmol) followed by N-methylmorpholine (54.9 mg, 0.54 mmol) at rt. The reaction mixture was stirred for 2 h, then diluted with EtOAc (15 mL). The organic layer was washed with water (15 mL), brine (15 mL), dried over $Na_2SO_4$ and concentrated under reduced pressure to get a residue which was purified by HPLC (Method F) to afford 1-((2-oxabicyclo[2.1.1]hexan-1-yl)methyl)-N-(3-sulfamoylphenyl)-4-(trifluoromethyl)-1H-pyrazole-5-carboxamide (17 mg, 37% yield). LC-MS: m/z $[M+H]^+$ = 431. $^1$H-NMR (400 MHz, DMSO-$d_6$): δ (ppm) = 11.13 (bs, 1H), 8.26 (s, 1H), 8.02 (s, 1H), 7.74 (d, 1H), 7.67 - 7.52 (m, 2H), 7.41 (bs, 2H), 4.64 (s, 2H), 3.62 (s, 2H), 2.88 - 2.84 (m, 1H), 1.74 (s, 2H), 1.32 (dd, 2H).

**Example 31 to 35**

The following Examples were prepared using a similar method as described for Example 30 from 3-aminobenzenesulfonamide and the corresponding acid.

| Example | Structure, Name and Starting Materials (SM) | Data |
|---|---|---|
| **31** | 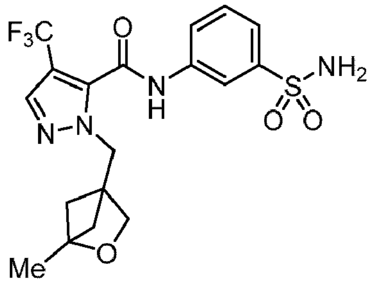 1-((1-methyl-2-oxabicyclo[2.1.1]hexan-4-yl) methyl)-N-(3-sulfamoylphenyl)-4-(trifluoromethyl)-1H-pyrazole-5-carboxamide<br>SM: Intermediate 68 | Grace chromatography<br>26 mg, 49% yield. LC-MS: m/z $[M+H]^+$ = 443. $^1$HNMR (400 MHz, DMSO-$d_6$): δ (ppm) = 11.18 (s, 1H), 8.24 (s, 1H), 8.06 (s, 1H), 7.77 (d, 1H), 7.67 - 7.57 (m, 2H), 7.44 (s, 2H), 4.60 (s, 2H), 3.46 (s, 2H), 1.52 (d, 1H), 1.42 (d, 1H), 1.27 (s, 3H). |
| **32** | 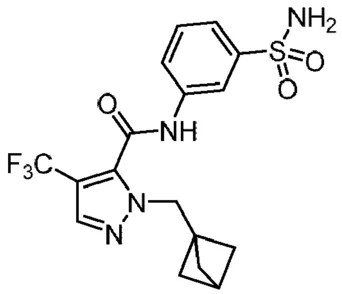 1-(bicyclo[1.1.1]pentan-1-ylmethyl)-N-(3-sulfamoylphenyl)-4-(trifluoromethyl)-1H-pyrazole-5-carboxamide<br>SM: Intermediate 69 | HPLC: Method I<br>11 mg, 20% yield. LC-MS: m/z $[M+H]^+$ = 413. $^1$HNMR (400 MHz, DMSO-$d_6$): δ (ppm) = 11.15 (s, 1H), 8.28 - 8.22 (m, 1H), 8.04 (s, 1H), 7.78 (d, 1H), 7.66 - 7.58 (m, 2H), 7.44 (s, 2H), 4.32 (s, 2H), 2.44 (s, 1H), 1.65 (s, 6H). |
| **33** | 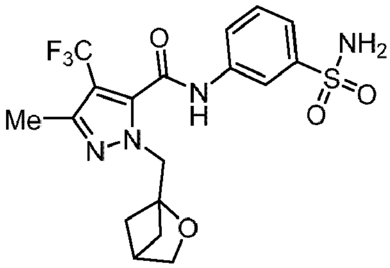 1-((2-oxabicyclo[2.1.1]hexan-1-yl)methyl)-3-methyl-N-(3-sulfamoylphenyl)-4-(trifluoromethyl)-1H-pyrazole-5-carboxamide<br>SM: Intermediate 70 | HPLC: Method L<br>6 mg, 8% yield. LC-MS: m/z $[M+H]^+$ = 445. $^1$HNMR (400 MHz, DMSO-$d_6$): δ (ppm) = 11.14 (s, 1H), 8.27 (s, 1H), 7.76 - 7.73 (m, 1H), 7.63 - 7.54 (m, 2H), 7.43 (s, 2H), 4.49 (s, 2H), 3.61 (s, 2H), 2.86 - 2.82 (m, 1H), 2.30 (s, 3H), 1.74 - 1.72 (m, 2H), 1.33 - 1.31 (m, 2H). |
| **34** | 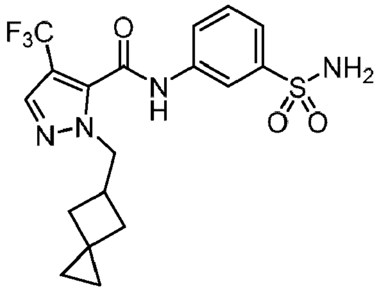 1-(spiro[2.3]hexan-5-ylmethyl)-N-(3-sulfamoylphenyl)-4-(trifluoromethyl)-1H-pyrazole-5-carboxamide<br>SM: Intermediate 71 | HPLC: Method I<br>11 mg, 14% yield. LC-MS: m/z $[M+H]^+$ = 427. $^1$HNMR (400 MHz, DMSO-$d_6$): δ (ppm) = 11.30 (s, 1H), 8.28 (s, 1H), 8.03 (s, 1H), 7.78 (d, 1H), 7.66 - 7.58 (m, 2H), 7.44 (s, 2H), 4.36 (d, 2H), 2.89 - 2.83 (m, 1H), 2.14 - 2.06 (m, 2H), 1.94 - 1.87 (m, 2H), 0.38 - 0.30 (m, 4H). |

(continued)

| Example | Structure, Name and Starting Materials (SM) | Data |
|---|---|---|
| **35** | 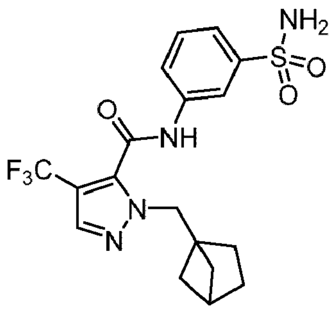<br>1-(bicyclo[2.1.1]hexan-1-ylmethyl)-N-(3-sulfamoylphenyl)-4-(trifluoromethyl)-1H-pyrazole-5-carboxamide<br>SM: Intermediate 72 | HPLC: Method I<br>7 mg, 14%yield. LC-MS: m/z $[M+H]^+$ = 429. $^1$HNMR (400 MHz, DMSO-$d_6$): δ (ppm) = 8.20 - 8.10 (m, 1H), 8.00 -7.80 (m, 1H), 7.75 - 7.65 (m, 1H), 7.60 - 7.17 (m, 3H), 4.56 (s, 2H), 2.31 (s, 1H), 1.60 - 1.52 (m, 2H), 1.40 - 1.32 (m, 4H), 1.31 (s, 2H), 0.99 - 0.93 (m, 2H). |

**Example 36 to 42**

The following Examples were prepared using a similar method as described for Example 30 from 5-amino-2-fluorobenzamide and the corresponding acid.

| Example | Structure, Name and Starting Materials (SM) | Data |
|---|---|---|
| **36** | 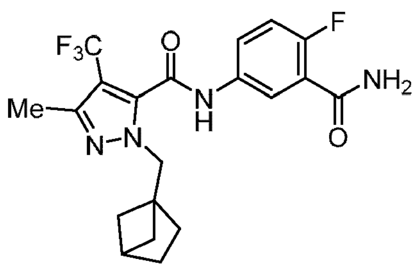<br>1-(bicyclo[2.1.1]hexan-1-ylmethyl)-N-(3-carbamoyl-4-fluorophenyl)-3-methyl-4-(trifluoromethyl)-1H-pyrazole-5-carboxamide<br>SM: Intermediate 73 | HPLC: Method M<br>9 mg, 22% yield. LC-MS: m/z $[M+H]^+$ = 425. $^1$HNMR (400 MHz, DMSO-$d_6$): δ (ppm) = 11.01 (s, 1H), 7.94 (dd, 1H), 7.77 - 7.69 (m, 3H), 7.30 (t, 1H), 4.32 (s, 2H), 2.35 - 2.29 (m, 4H), 1.59 - 1.55 (m, 2H), 1.40 - 1.32 (m, 4H), 0.99 - 0.96 (m, 2H). |
| **37** | 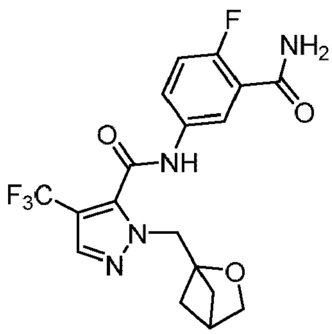<br>1-((2-oxabicyclo[2.1.1]hexan-1-yl)methyl)-N-(3-carbamoyl-4-fluorophenyl)-4-(trifluoromethyl)-1H-pyrazole-5 -carboxamide<br>SM: Intermediate 67 | HPLC: Method F<br>20 mg, 45% yield. LC-MS: m/z $[M+H]^+$ = 411. $^1$HNMR (400 MHz, DMSO-$d_6$): δ (ppm) = 10.97 (ss, 1H), 8.03 (s, 1H), 7.96 (dd, 1H), 7.78 - 7.69 (m, 3H), 7.31 (t, 1H), 4.61(s, 2H), 3.61 (s, 2H), 2.85 (t, 1H), 1.74 (s, 2H), 1.32 (dd, 2H). |

(continued)

| Example | Structure, Name and Starting Materials (SM) | Data |
|---|---|---|
| **38** | 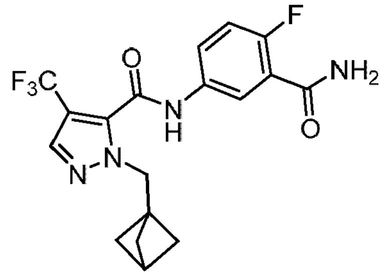 1-(bicyclo[1.1.1]pentan-1-ylmethyl)-N-(3-carbamoyl-4-fluorophenyl)-4-(trifluoromethyl)-1H-pyrazole-5-carboxamide SM: Intermediate 69 | HPLC: Method F 20 mg, 44% yield. LC-MS: m/z $[M+H]^+$ = 395. $^1$HNMR (400 MHz, DMSO-$d_6$): δ (ppm) = 11.00 (s, 1H), 8.03 (s, 1H), 7.96 (dd, 1H), 7.79 - 7.70 (m, 3H), 7.32 (t, 1H), 4.31 (s, 2H), 2.44 (s, 1H), 1.65 (s, 6H). |
| **39** | 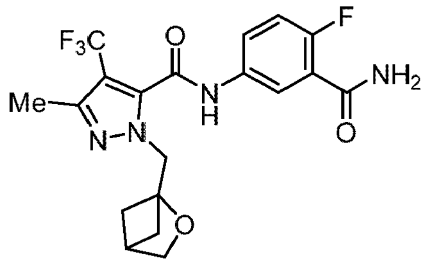 1-((2-oxabicyclo[2.1.1]hexan-1-yl)methyl)-N-(3-carbamoyl-4-fluorophenyl)-3-methyl-4-(trifluoromethyl)-1H-pyrazole-5-carboxamide SM: Intermediate 70 | HPLC: Method F 12 mg, 27% yield. LC-MS: m/z $[M+H]^+$ = 427. $^1$HNMR (500 MHz, DMSO-$d_6$): δ (ppm) = 10.97 (s, 1H), 7.96 (dd, 1H), 7.77 - 7.68 (m, 3H), 7.30 (t, 1H), 4.48 (s, 2H), 3.61 (s, 2H), 2.85 - 2.83 (m, 1H), 2.29 (s, 3H), 1.73 - 1.71 (m, 2H), 1.32 - 1.31 (m, 2H). |
| **40** | 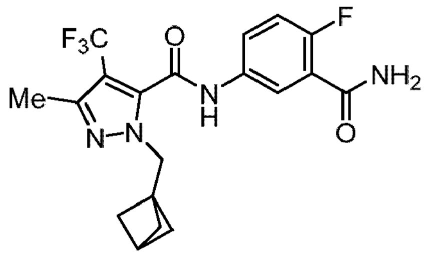 1-(bicyclo[1.1.1]pentan-1-ylmethyl)-N-(3-carbamoyl-4-fluorophenyl)-3-methyl-4-(trifluoromethyl)-1H-pyrazole-5-carboxamide SM: Intermediate 74 | HPLC: Method F 10 mg, 17% yield. LC-MS: m/z $[M+H]^+$ = 409. $^1$HNMR (400 MHz, DMSO-$d_6$): δ (ppm) = 10.99 (s, 1H), 7.92 (s, 1H), 7.71 (br s, 3H), 7.28 (br s, 1H), 4.19 (s, 2H), 2.43 (s, 1H), 2.29 (s, 3H), 1.64 (s, 6H). |
| **41** | 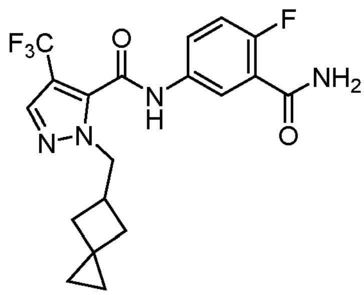 N-(3-carbamoyl-4-fluorophenyl)-1-(spiro[2.3]hexan-5-ylmethyl)-4-(trifluoromethyl)-1H-pyrazole-5-carboxamide SM: Intermediate 71 | HPLC: Method I 11 mg, 25%yield. LC-MS: m/z $[M+H]^+$ = 409. $^1$HNMR (400 MHz, DMSO-$d_6$): δ (ppm) = 11.15 (s, 1H), 8.02 (s, 1H), 7.99 - 7.96 (m, 1H), 7.79 - 7.71 (m, 3H), 7.32 (t, 1H), 4.35 (d, 2H), 2.89 - 2.81 (m, 1H), 2.14 - 2.06 (m, 2H), 1.93 - 1.86 (m, 2H), 0.36 - 0.32 (m, 4H). |

(continued)

| Example | Structure, Name and Starting Materials (SM) | Data |
|---|---|---|
| **42** | 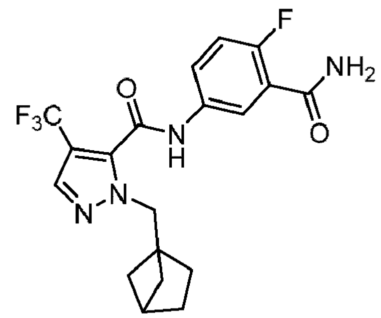 1-(bicyclo[2.1.1]hexan-1-ylmethyl)-N-(3-carbamoyl-4-fluorophenyl)-4-(trifluoromethyl)-1H-pyrazole-5-carboxamide<br>SM: Intermediate 72 | HPLC: Method J<br>20 mg, 44% yield. LC-MS: m/z $[M+H]^+$ = 409.<br>$^1$HNMR (400 MHz, DMSO-$d_6$): δ (ppm) = 11.01 (s, 1H), 8.02 (s, 1H), 7.95 (dd, 1H), 7.78 - 7.70 (m, 3H), 7.31 (t, 1H), 4.45 (s, 2H), 2.33 (s, 1H), 1.61 - 1.54 (m, 2H), 1.40 - 1.32 (m, 4H), 1.00 - 0.96 (m, 2H). |

**Example 43a/43b/43c/43d**

1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide, synthesized in the forms of:

1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide, isomer 1 (43a)

1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide, isomer 2 (43b)

1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide, isomer 3 (43c)

1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide, isomer 4 (43d)

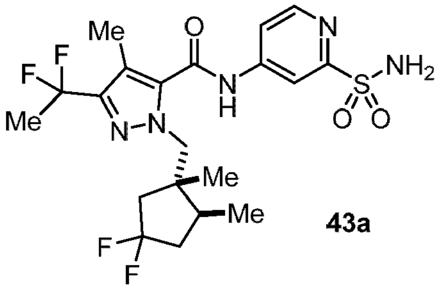
43a

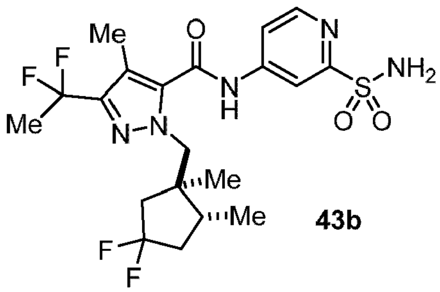
43b

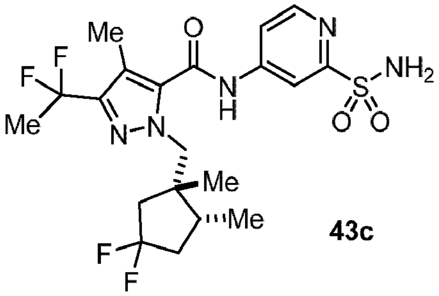
43c

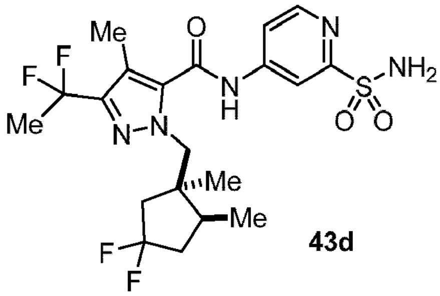
43d

Step A: To a stirred solution of 1-((4,4-difluoro-1,2-dimethylcyclopentyl)methyl)-3-(1,1-difluoroethyl)-4-methyl-1H-pyrazole-5-carboxamide (Intermediate 82, 450 mg, 1.34 mmol) in dioxane (10 mL) in a sealed tube were added tert-butyl ((4-bromopyridin-2-yl)sulfonyl)(tert-butyl)carbamate (633.5 mg, 1.61 mmol), $Cs_2CO_3$ (1.09 g, 3.35 mmol) at rt, and the reaction mixture was degassed with argon for 10 min. To the reaction mixture was added trans N,N-dimethyl 1,2-diamino cyclohexane (57.2 mg, 0.40 mmol) and copper (I) trifluoromethane sulfonate benzene complex (168.9 mg, 0.335 mmol) at rt. The resulting reaction mixture was stirred at 85 °C for 16 h. The reaction mixture was cooled to rt, filtered over Celite®, washing through with ethyl acetate (50 mL). The filtrate was concentrated under reduced pressure to afford tert-butyl tert-butyl((4-(1-((4,4-difluoro-1,2-dimethylcyclopentyl)methyl)-3-(1,1-difluoroethyl)-4-methyl-1H-pyrazole-5-carboxamido)pyridin-2-yl)sulfonyl)carbamate 760 mg, crude). The crude product was directly used in the next step without further purification.

Step B: To the mixture of Step A (750 mg, 1.16 mmol) in 2,2,2-trifluoroethanol (7.5 mL) was added TMSCl (2.2 mL) at 0 °C and the resulting reaction mixture was stirred at rt for 3 h. The reaction mixture was quenched with cold water (30 mL), followed by saturated $NaHCO_3$ solution (50 mL) at 0 °C and extracted with EtOAc(2×50 mL). The combined organic layers were washed with brine (50 mL), dried over anhydrous $Na_2SO_4$ and concentrated under reduced pressure to give the crude product. The crude product was purified by Grace chromatography (see under HPLC conditions) to afford 1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide (495 mg, 75% over two steps).

This mixture (490 mg) was separated by prep-SFC using a Chiralcel OJ-H (250×30mm, 5 μm) column, eluting with 10% MeOH isocratic gradient, at 70 g/min, to give 175 mg of isomers Peak 1 and Peak 2 as a mixture, 73 mg of isomer Peak 3, Example 43c, and 115 mg of isomer Peak 4, Example 43d.

Retention time Peak 3: 3.69 min; Peak 4: 5.54 min.

Isomers Peak 1 and Peak 2 were separated by prep-HPLC using a Chiralpak AD-H (250×30mm, 5 μm) column, eluting with n-Hex : EtOH 95:5, at 36.0 mL//min, to give 60 mg and 40 mg of isomers Peak 1, Example 43a and Peak 2, Example 43b, respectively.

Retention time Peak 1: 22.28 min; Peak 2: 27.26 min.

| Example | Data: |
|---|---|
| **43a** | 60 mg, chiral purity: 97.9%<br>LC-MS: m/z $[M+H]^+$ = 492. $^1$HNMR (400 MHz, DMSO-$d_6$): δ (ppm) = 11.27 (s, 1H), 8.63 (d, 1H), 8.30 (s, 1H), 7.83 (s, 1H), 7.46 (s, 2H), 4.27 (s, 2H), 2.38-2.19 (m, 5H), 2.09-1.85 (m, 6H), 0.82 (s, 3H), 0.78 (d, 3H). |
| **43b** | 40 mg, chiral purity: 96.6%<br>LC-MS: m/z $[M+H]^+$ = 492. $^1$HNMR (400 MHz, DMSO-$d_6$): δ (ppm) = 11.24 (s, 1H), 8.58 (bs, 1H), 8.25 (s, 1H), 7.79 (bs, 1H), 7.42 (bs, 2H), 4.35-4.24 (m, 2H), 2.51 (m, 1H), 2.41-2.29 (m, 1H), 2.26 (s, 3H), 2.11-1.98 (m, 5H), 1.82-1.69 (m, 1H), 1.05 (d, 3H), 0.86 (s, 3H). |
| **43c** | 73 mg, chiral purity: 99.9%<br>LC-MS: m/z $[M+H]^+$ = 492. $^1$HNMR (400 MHz, DMSO-$d_6$): δ (ppm) = 11.22 (s, 1H), 8.64 (d, 1H), 8.29 (s, 1H), 7.84 (d, 1H), 7.47 (s, 2H), 4.27 (q, 2H), 2.43 (m, 1H), 2.38-2.12 (m, 1H), 2.25 (s, 3H), 2.09-1.98 (m, 5H), 1.80-1.74 (m, 1H), 1.01 (d, 3H), 0.92 (s, 3H). |
| **43d** | 115 mg, chiral purity: 99.4%<br>LC-MS: m/z $[M+H]^+$ = 492. $^1$HNMR (400 MHz, DMSO-$d_6$): δ (ppm) = 11.27 (s, 1H), 8.64 (d, 1H), 8.30 (s, 1H), 7.84 (d, 1H), 7.45 (s, 2H), 4.26 (s, 2H), 2.37-2.19 (m, 5H), 2.09-1.85 (m, 7H), 0.82 (s, 3H), 0.78 (d, 3H). |

**Example 47 to 49**

The following Examples were prepared using a similar method as described for Example 30 from 1-(methylsulfonyl)-1H-pyrazol-4-amine and the corresponding acid.

| Example | Structure, Name and Starting Materials (SM) | Data |
|---|---|---|
| **47** | 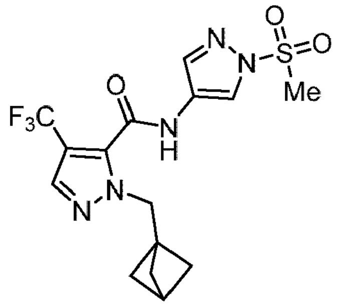 1-(bicyclo[1.1.1]pentan-1-ylmethyl)-N-(1-(methylsulfonyl)-1H-pyrazol-4-yl)-4-(trifluoromethyl)-1H-pyrazole-5-carboxamide SM: Intermediate 69 | HPLC: Method F<br>41 mg, 23%yield. LC-MS: m/z $[M+H]^+$ = 404. $^1$HNMR (400 MHz, DMSO-$d_6$): δ (ppm) = 11.34 (s, 1H), 8.45 (s, 1H), 8.04 (d, 2H), 4.30 (s, 2H), 3.59 (s, 3H), 2.43 (s, 1H), 1.63 (s, 6H). |
| **48** | 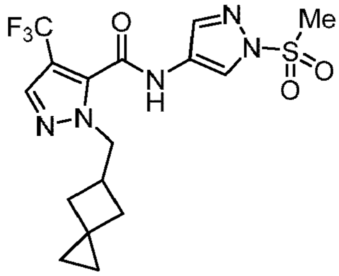 N-(1-(methylsulfonyl)-1H-pyrazol-4-yl)-1-(spiro[2.3]hexan-5-ylmethyl)-4-(trifluoromethyl)-1H-pyrazole-5-carboxamide<br>SM: Intermediate 71 | HPLC: Method I<br>42 mg, 55% yield. LC-MS: m/z $[M+H]^+$ = 418. $^1$HNMR (400 MHz, DMSO-$d_6$): δ (ppm) = 11.48 (s, 1H), 8.47 (s, 1H), 8.03 (s, 2H), 4.33 (d, 2H), 3.59 (s, 3H), 2.88 - 2.79 (m, 1H), 2.11-2.04 (m, 2H), 1.91 - 1.85 (m, 2H), 0.36 - 0.31 (m, 4H). |
| **49** | 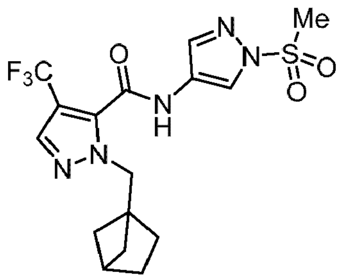 1-(bicyclo[2.1.1]hexan-1-ylmethyl)-N-(1-(methylsulfonyl)-1H-pyrazol-4-yl)-4-(trifluoromethyl)-1H-pyrazole-5-carboxamide<br>SM: Intermediate 72 | HPLC: Method F<br>21 mg, 46% yield. LC-MS: m/z $[M+H]^+$ = 418. $^1$HNMR (400 MHz, DMSO-$d_6$): δ (ppm) = 11.35 (s, 1H), 8.44 (s, 1H), 8.02 (d, 2H), 4.44 (s, 2H), 3.59 (s, 3H), 2.32 (s, 1H), 1.60 - 1.53 (m, 2H), 1.39 - 1.30 (m, 4H), 0.96 - 0.94 (m, 2H). |

The following prophetic examples could be synthesized according to the general schemes and by analogy to the synthetic procedures described above:

| Structure | Prophetic example number | Chemical name |
|---|---|---|
| 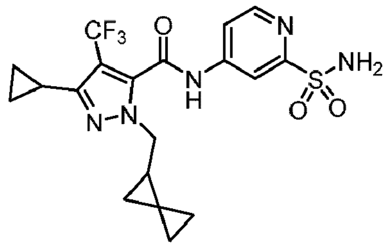 | 50 | 3-cyclopropyl-1-(spiro[2.2]pentan-1-ylmethyl)-N-(2-sulfamoylpyridin-4-yl)-4-(trifluoromethyl)-1H-pyrazole-5-carboxamide |
| 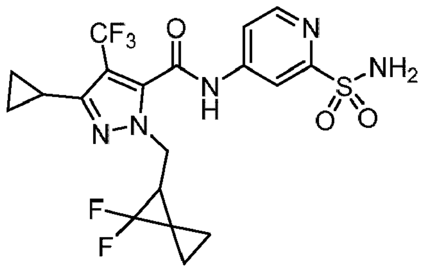 | 51 | 3-cyclopropyl-1-((2,2-difluorospiro[2.2]pentan-1-yl) methyl)-N-(2-sulfamoylpyridin-4-yl)-4-(trifluoromethyl)-1H-pyrazole-5-carboxamide |
| 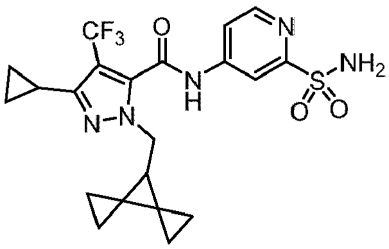 | 52 | 3-cyclopropyl-1-(dispiro[$2.0.2^{4}.1^{3}$]heptan-7-ylmethyl)-N-(2-sulfamoylpyridin-4-yl)-4-(trifluoromethyl)-1H-pyrazole-5-carboxamide |
| 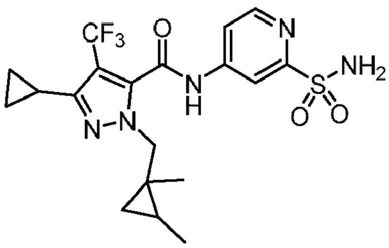 | 53 | 3-cyclopropyl-1-((1,2-dimethylcyclopropyl) methyl)-N-(2-sulfamoylpyridin-4-yl)-4-(trifluoromethyl)-1H-pyrazole-5-carboxamide |
| 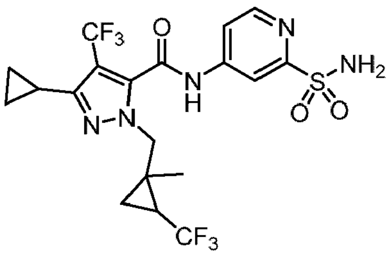 | 54 | 3 -cyclopropyl-1 -((1 -methyl-2-(trifluoromethyl) cyclopropyl)methyl)-N-(2-sulfamoylpyridin-4-yl)-4-(trifluoromethyl)-1H-pyrazole-5-carboxamide |
| 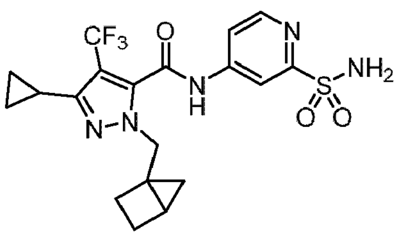 | 55 | 1-(bicyclo [2.1.0]pentan-1-ylmethyl)-3-cyclopropyl-N-(2-sulfamoylpyridin-4-yl)-4-(trifluoromethyl)-1H-pyrazole-5-carboxamide |

(continued)

| Structure | Prophetic example number | Chemical name |
|---|---|---|
| 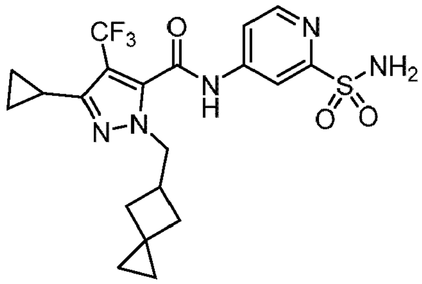 | 56 | 3-cyclopropyl-1-(spiro[2.3]hexan-5-ylmethyl)-N-(2-sulfamoylpyridin-4-yl)-4-(trifluoromethyl)-1H-pyrazole-5-carboxamide |
| 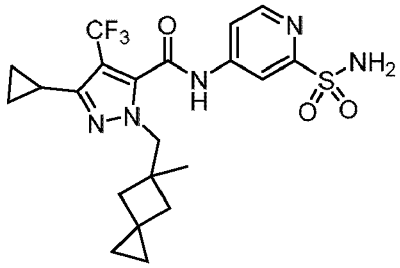 | 57 | 3-cyclopropyl-1-((5-methyl-spiro[2.3]hexan-5-yl) methyl)-N-(2-sulfamoylpyridin-4-yl)-4-(trifluoromethyl)-1H-pyrazole-5-carboxamide |
| 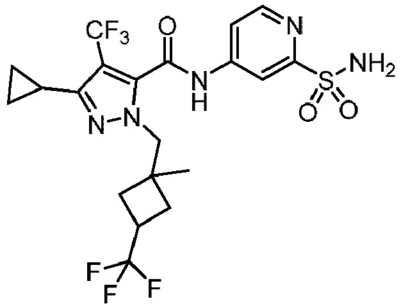 | 58 | 3-cyclopropyl-1-((1-methyl-3-(trifluoromethyl) cyclobutyl)methyl)-N-(2-sulfamoylpyridin-4-yl)-4-(trifluoromethyl)-1H-pyrazole-5-carboxamide |
| 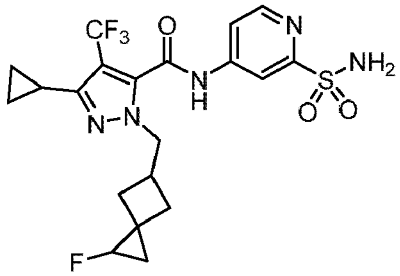 | 59 | 3-cyclopropyl-1-((1-fluoro-spiro[2.3]hexan-5-yl) methyl)-N-(2-sulfamoylpyridin-4-yl)-4-(trifluoromethyl)-1H-pyrazole-5-carboxamide |
| 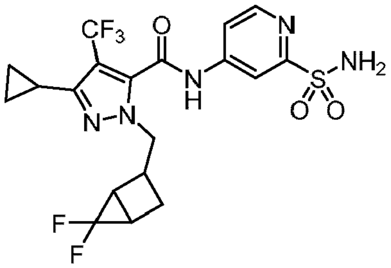 | 60 | 3-cyclopropyl-1-((5,5-difluorobicyclo[2.1.0]pentan-2-yl)methyl)-N-(2-sulfamoylpyridin-4-yl)-4-(trifluoromethyl)-1H-pyrazole-5-carboxamide |

(continued)

| Structure | Prophetic example number | Chemical name |
|---|---|---|
| 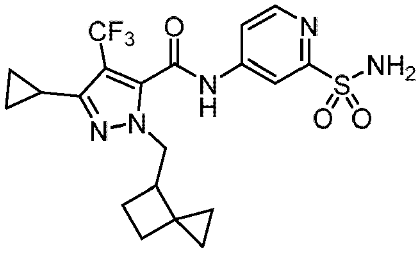 | 61 | 3-cyclopropyl-1-(spiro[2.3]hexan-4-ylmethyl)-N-(2-sulfamoylpyridin-4-yl)-4-(trifluoromethyl)-1H-pyrazole-5-carboxamide |
| 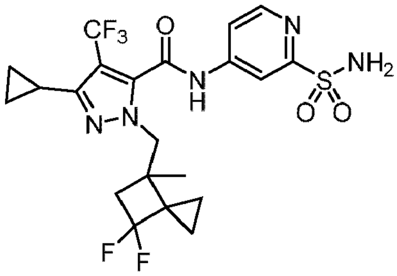 | 62 | 3-cyclopropyl-1-((6,6-difluoro-4-methyl-spiro[2.3] hexan-4-yl)methyl)-N-(2-sulfamoylpyridin-4-yl)-4-(trifluoromethyl)-1H-pyrazole-5-carboxamide |
| 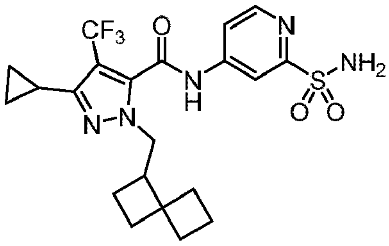 | 63 | 3-cyclopropyl-1-(spiro[3.3]heptan-1-ylmethyl)-N-(2-sulfamoylpyridin-4-yl)-4-(trifluoromethyl)-1H-pyrazole-5-carboxamide |
| 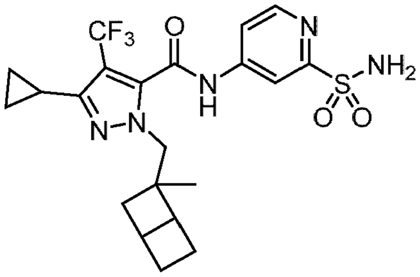 | 64 | 3-cyclopropyl-1-((2-methylbicyclo[2.2.0]hexan-2-yl) methyl)-N-(2-sulfamoylpyridin-4-yl)-4-(trifluoromethyl)-1H-pyrazole-5-carboxamide |
| 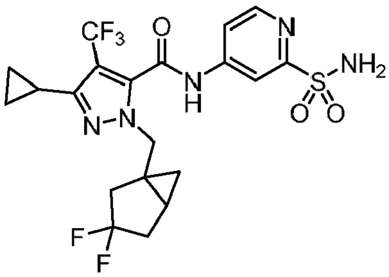 | 65 | 3-cyclopropyl-1-((3,3-difluorobicyclo[3.1.0]hexan-1-yl)methyl)-N-(2-sulfamoylpyridin-4-yl)-4-(trifluoromethyl)-1H-pyrazole-5-carboxamide |

(continued)

| Structure | Prophetic example number | Chemical name |
|---|---|---|
| 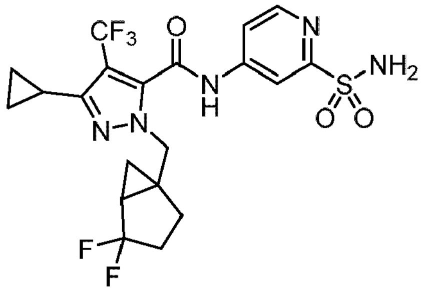 | 66 | 3-cyclopropyl-1-((4,4-difluorobicyclo[3.1.0]hexan-1-yl)methyl)-N-(2-sulfamoylpyridin-4-yl)-4-(trifluoromethyl)-1H-pyrazole-5-carboxamide |
| 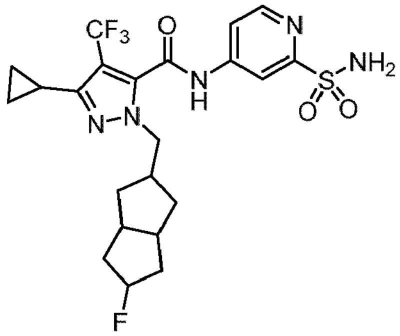 | 67 | 3-cyclopropyl-1-((5-fluorooctahydropentalen-2-yl) methyl)-N-(2-sulfamoylpyridin-4-yl)-4-(trifluoromethyl)-1H-pyrazole-5 -carboxamide |
| 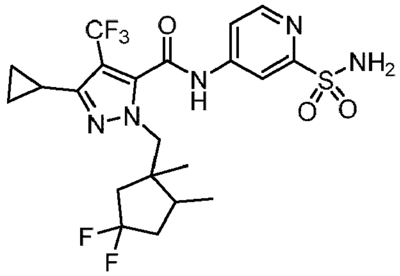 | 68 | 3-cyclopropyl-1-((4,4-difluoro-1,2-dimethylcyclopentyl)methyl)-N-(2-sulfamoylpyridin-4-yl)-4-(trifluoromethyl)-1H-pyrazole-5-carboxamide |
| 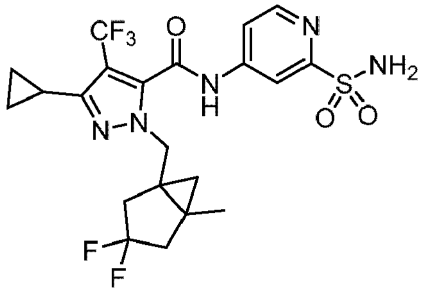 | 69 | 3-cyclopropyl-1-((3,3-difluoro-5-methylbicyclo[3.1.0] hexan-1-yl)methyl)-N-(2-sulfamoylpyridin-4-yl)-4-(trifluoromethyl)-1H-pyrazole-5-carboxamide |
| 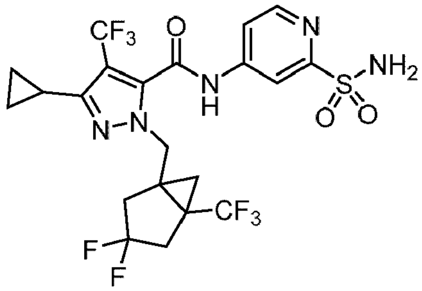 | 70 | 3-cyclopropyl-1-((3,3-difluoro-5-(trifluoromethyl) bicyclo[3.1.0]hexan-1-yl)methyl)-N-(2-sulfamoylpyridin-4-yl)-4-(trifluoromethyl)-1H-pyrazole-5-carboxamide |

(continued)

| Structure | Prophetic example number | Chemical name |
|---|---|---|
| 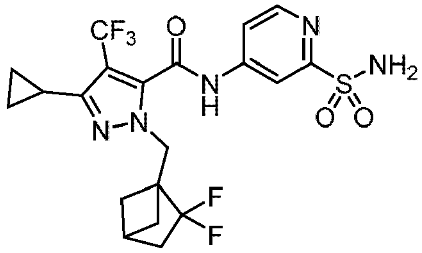 | 71 | 3-cyclopropyl-1-((2,2-difluorobicyclo[2.1.1]hexan-1-yl)methyl)-N-(2-sulfamoylpyridin-4-yl)-4-(trifluoromethyl)-1H-pyrazole-5-carboxamide |
| 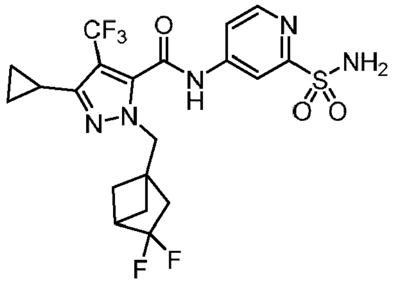 | 72 | 3-cyclopropyl-1-((3,3-difluorobicyclo[2.1.1]hexan-1-yl)methyl)-N-(2-sulfamoylpyridin-4-yl)-4-(trifluoromethyl)-1H-pyrazole-5-carboxamide |
| 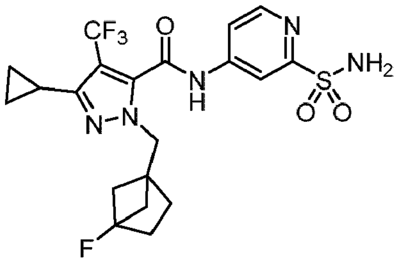 | 73 | 3-cyclopropyl-1-((4-fluorobicyclo[2.1.1]hexan-1-yl) methyl)-N-(2-sulfamoylpyridin-4-yl)-4-(trifluoromethyl)-1H-pyrazole-5-carboxamide |
| 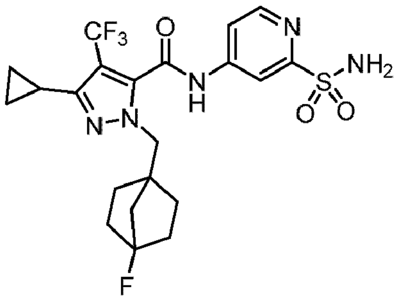 | 74 | 3 -cyclopropyl- 1-((4-fluorobicyclo[2.1.1]hexan-1-yl) methyl)-N-(2-sulfamoylpyridin-4-yl)-4-(trifluoromethyl)-1H-pyrazole-5-carboxamide |
| 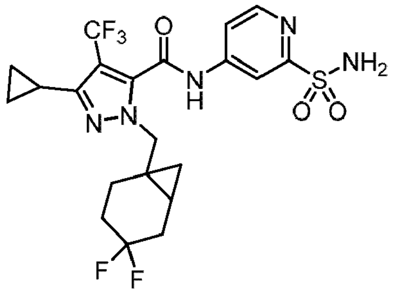 | 75 | 3-cyclopropyl-1-((4,4-difluorobicyclo[4.1.0]heptan-1-yl)methyl)-N-(2-sulfamoylpyridin-4-yl)-4-(trifluoromethyl)-1H-pyrazole-5-carboxamide |

(continued)

| Structure | Prophetic example number | Chemical name |
|---|---|---|
| 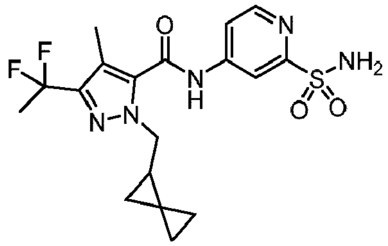 | 76 | 3-(1,1-difluoroethyl)-4-methyl-1-(spiro[2.2]pentan-1-ylmethyl)-N-(2-sulfamoylpyridin-4-yl)-1H-pyrazole-5-carboxamide |
| 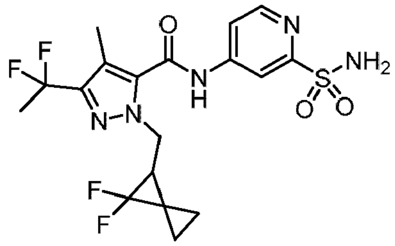 | 77 | 3-(1,1-difluoroethyl)-1-((2,2-difluoro-spiro[2.2] pentan-1-yl)methyl)-4-methyl-N-(2-sulfamoylpyridin-4-yl)-1H-pyrazole-5 -carboxamide |
| 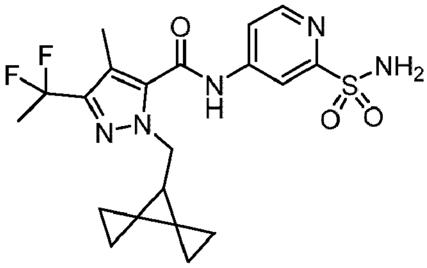 | 78 | 3-(1,1-difluoroethyl)-1-(dispiro[$2.0.2^{4}.1^{3}$]heptan-7-ylmethyl)-4-methyl-N-(2-sulfamoylpyridin-4-yl)-1H-pyrazole-5-carboxamide |
| 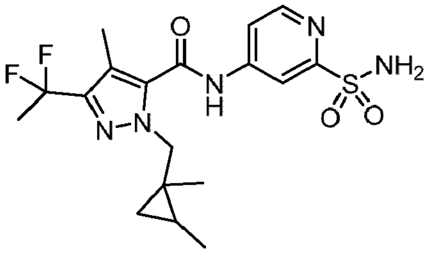 | 79 | 3-(1,1-difluoroethyl)-1-((1,2-dimethylcyclopropyl) methyl)-4-methyl-N-(2-sulfamoylpyridin-4-yl)-1H-pyrazole-5-carboxamide |
| 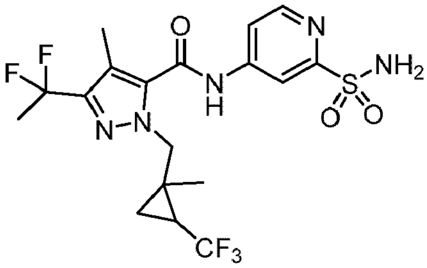 | 80 | 3 -(1,1 -difluoroethyl)-4-methyl-1 -((1 -methyl-2-(trifluoromethyl)cyclopropyl)methyl)-N-(2-sulfamoylpyridin-4-yl)-1H-pyrazole-5-carboxamide |

(continued)

| Structure | Prophetic example number | Chemical name |
|---|---|---|
| 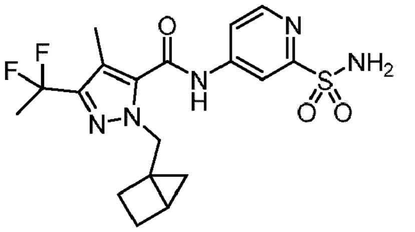 | 81 | 1-(bicyclo [2.1.0]pentan-1-ylmethyl)-3-(1,1-difluoroethyl)-4-methyl-N-(2-sulfamoylpyridin-4-yl)-1H-pyrazole-5-carboxamide |
| 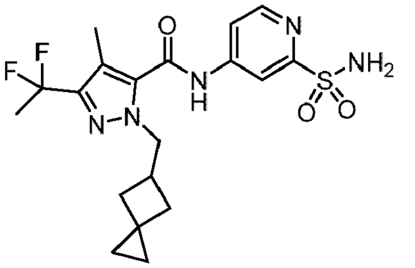 | 82 | 3-(1,1-difluoroethyl)-4-methyl-1-(spiro[2.3]hexan-5-ylmethyl)-N-(2-sulfamoylpyridin-4-yl)-1H-pyrazole-5-carboxamide |
| 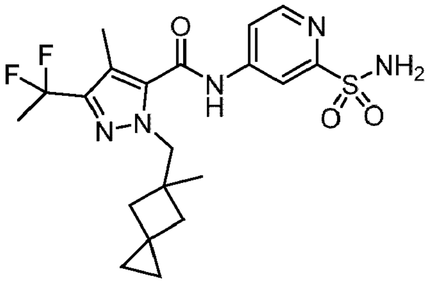 | 83 | 3-(1,1-difluoroethyl)-4-methyl-1-((5-methylspiro[2.3] hexan-5-yl)methyl)-N-(2-sulfamoylpyridin-4-yl)-1H-pyrazole-5-carboxamide |
| 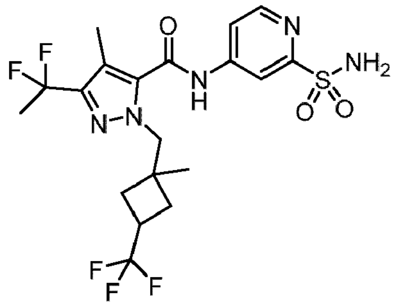 | 84 | 3-(1,1-difluoroethyl)-4-methyl-1-((1-methyl-3-(trifluoromethyl)cyclobutyl)methyl)-N-(2-sulfamoylpyridin-4-yl)-1H-pyrazole-5-carboxamide |
| 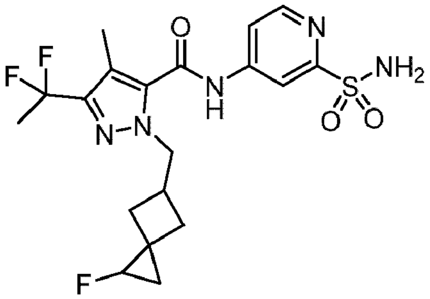 | 85 | 3-(1,1-difluoroethyl)-1-((1-fluoro-spiro[2.3]hexan-5-yl)methyl)-4-methyl-N-(2-sulfamoylpyridin-4-yl)-1H-pyrazole-5 -carboxamide |

(continued)

| Structure | Prophetic example number | Chemical name |
|---|---|---|
| 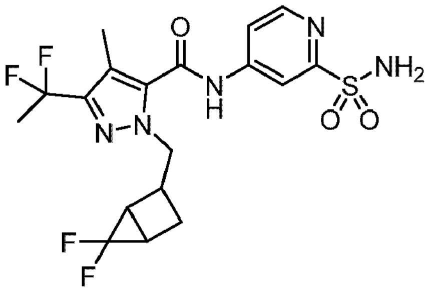 | 86 | 1-((5,5-difluorobicyclo[2.1.0]pentan-2-yl)methyl)-3 -(1,1 -difluoroethyl)-4-methyl-N-(2-sulfamoylpyridin-4-yl)-1H-pyrazole-5-carboxamide |
| 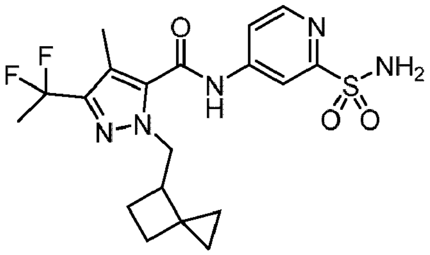 | 87 | 3-(1,1-difluoroethyl)-4-methyl-1-(spiro[2.3]hexan-4-ylmethyl)-N-(2-sulfamoylpyridin-4-yl)-1H-pyrazole-5-carboxamide |
| 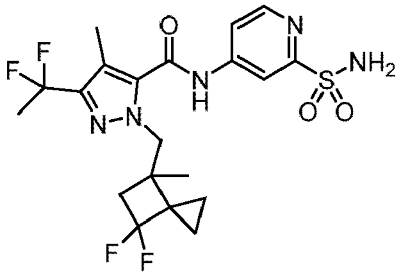 | 88 | 1-((6,6-difluoro-4-methyl-spiro[2.3]hexan-4-yl) methyl)-3-(1,1-difluoroethyl)-4-methyl-N-(2-sulfamoylpyridin-4-yl)-1H-pyrazole-5-carboxamide |
| 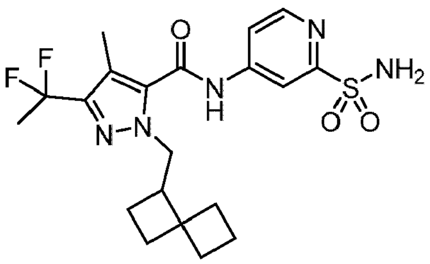 | 89 | 3-(1,1-difluoroethyl)-4-methyl-1-(spiro[3.3]heptan-1-ylmethyl)-N-(2-sulfamoylpyridin-4-yl)-1H-pyrazole-5-carboxamide |
| 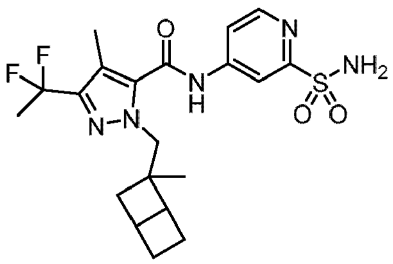 | 90 | 3-(1,1-difluoroethyl)-4-methyl-1-((2-methylbicyclo [2.2.0]hexan-2-yl)methyl)-N-(2-sulfamoylpyridin-4-yl)-1H-pyrazole-5 -carboxamide |

(continued)

| Structure | Prophetic example number | Chemical name |
|---|---|---|
| 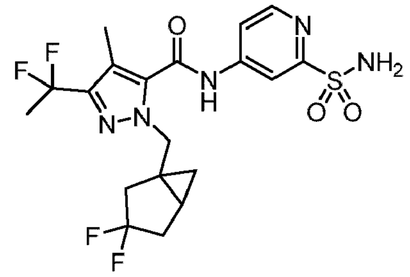 | 91 | 1-((3,3-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide |
| 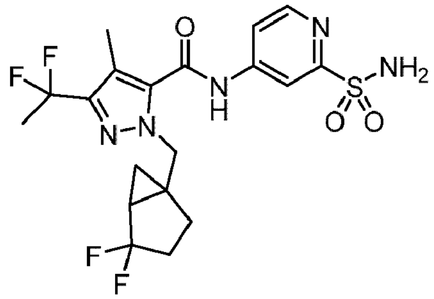 | 92 | 1-((4,4-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide |
| 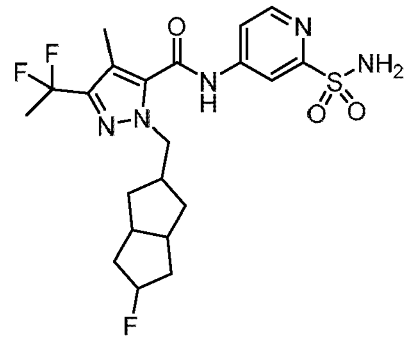 | 93 | 3-(1,1-difluoroethyl)-1-((5 -fluorooctahydropentalen-2-yl)methyl)-4-methyl-N-(2-sulfamoylpyridin-4-yl)-1H-pyrazole-5-carboxamide |
| 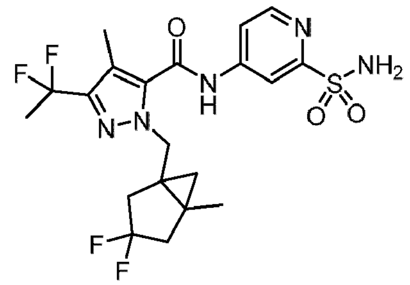 | 94 | 1-((3,3-difluoro-5-methylbicyclo[3. 1.0]hexan-1-yl) methyl)-3 -(1, 1-difluoroethyl)-4-methyl-N-(2-sulfamoylpyridin-4-yl)-1H-pyrazole-5-carboxamide |
| 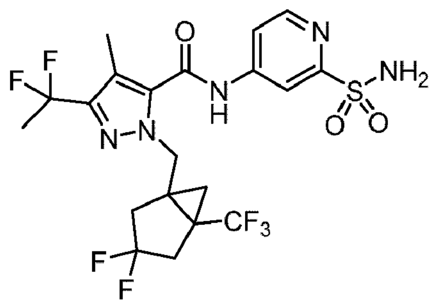 | 95 | 1-((3,3-difluoro-5-(trifluoromethyl)bicyclo[3.1.0] hexan-1-yl)methyl)-3-(1,1-difluoroethyl)-4-methyl-N-(2-sulfamoylpyridin-4-yl)-1H-pyrazole-5-carboxamide |

(continued)

| Structure | Prophetic example number | Chemical name |
|---|---|---|
| 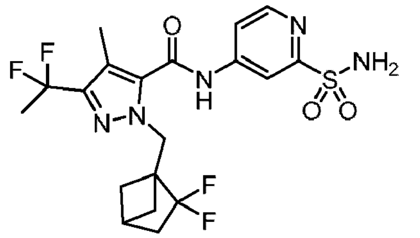 | 96 | 1-((2,2-difluorobicyclo[2.1.1]hexan-1-yl)methyl)-3 -(1,1 -difluoroethyl)-4-methyl-N-(2-sulfamoylpyridin-4-yl)-1H-pyrazole-5-carboxamide |
| 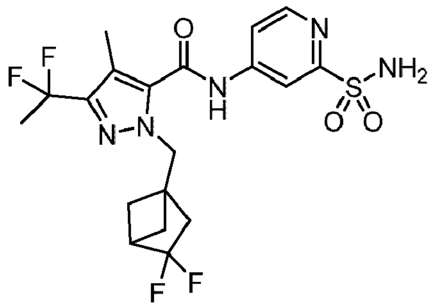 | 97 | 1-((3,3-difluorobicyclo[2.1.1]hexan-1-yl)methyl)-3-(1,1-difluoroethyl)-4-methyl-N-(2-sulfamoylpyridin-4-yl)-1H-pyrazole-5-carboxamide |
| 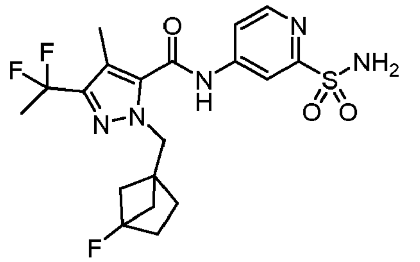 | 98 | 3-(1,1-difluoroethyl)-1-((4-fluorobicyclo[2.1.1]hexan-1-yl)methyl)-4-methyl-N-(2-sulfamoylpyridin-4-yl)-1H-pyrazole-5-carboxamide |
| 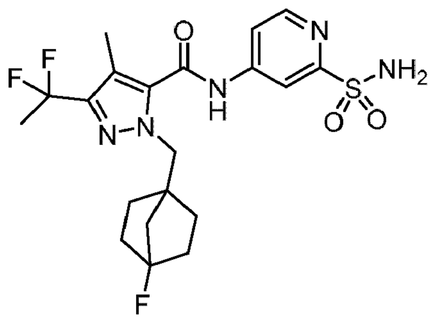 | 99 | 3-(1,1-difluoroethyl)-1-((4-fluorobicyclo[2.2.1] heptan-1-yl)methyl)-4-methyl-N-(2-sulfamoylpyridin-4-yl)-1H-pyrazole-5 -carboxamide |
| 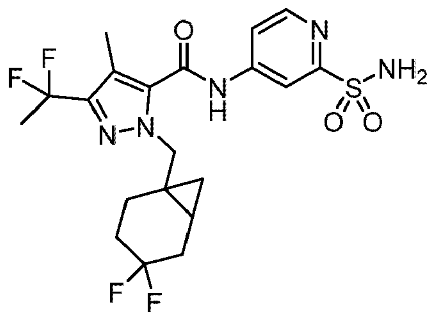 | 100 | 1-((4,4-difluorobicyclo [4.1.0] heptan-1-yl)methyl)-3 -(1,1 -difluoroethyl)-4-methyl-N-(2-sulfamoylpyridin-4-yl)-1H-pyrazole-5-carboxamide |

(continued)

| Structure | Prophetic example number | Chemical name |
|---|---|---|
| 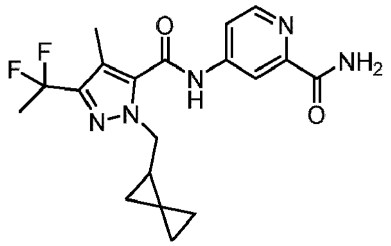 | 101 | 4-(3-(1,1-difluoroethyl)-4-methyl-1-(spiro[2.2] pentan-1-ylmethyl)-1H-pyrazole-5 -carboxamido) picolinamide |
| 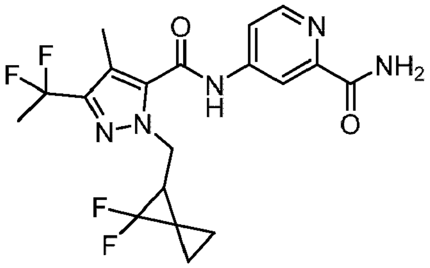 | 102 | 4-(3-(1,1-difluoroethyl)-1-((2,2-difluoro-spiro[2.2] pentan-1-yl)methyl)-4-methyl-1H-pyrazole-5-carboxamido)picolinamide |
| 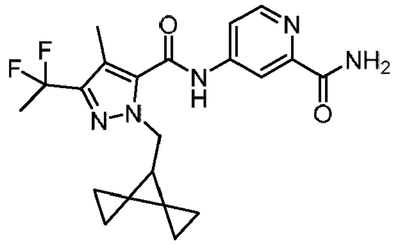 | 103 | 4-(3-(1,1-difluoroethyl)-1-(dispiro[2.0.2⁴.1³]heptan-7-ylmethyl)-4-methyl-1H-pyrazole-5-carboxamido) picolinamide |
| 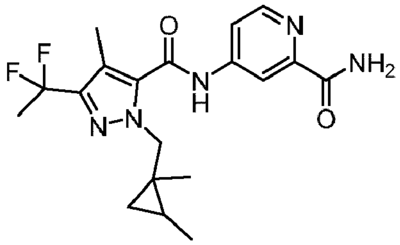 | 104 | 4-(3-(1,1-difluoroethyl)-1-((1,2-dimethylcyclopropyl) methyl)-4-methyl-1H-pyrazole-5-carboxamido) picolinamide |
| 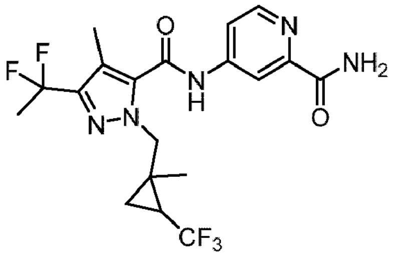 | 105 | 4-(3-(1,1-difluoroethyl)-4-methyl-1-((1-methyl-2-(trifluoromethyl)cyclopropyl)methyl)-1H-pyrazole-5-carboxamido)picolinamide |

(continued)

| Structure | Prophetic example number | Chemical name |
|---|---|---|
| 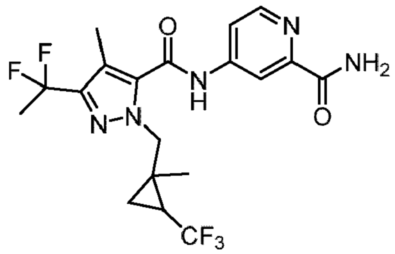 | 106 | 4-(3-(1,1-difluoroethyl)-4-methyl-1-((1-methyl-2-(trifluoromethyl)cyclopropyl)methyl)-1H-pyrazole-5-carboxamido)picolinamide |
| 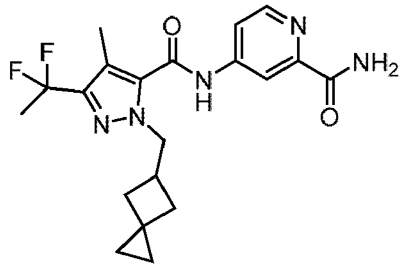 | 107 | 4-(3-(1,1-difluoroethyl)-4-methyl-1-(spiro[2.3]hexan-5-ylmethyl)-1H-pyrazole-5-carboxamido) picolinamide |
| 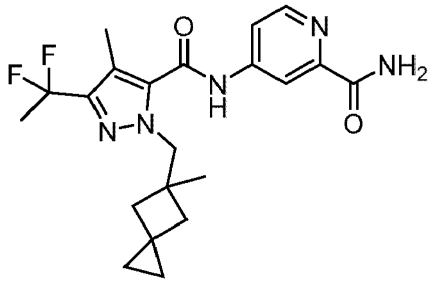 | 108 | 4-(3-(1,1-difluoroethyl)-4-methyl-1-((5-methylspiro [2.3]hexan-5-yl)methyl)-1H-pyrazole-5-carboxamido)picolinamide |
| 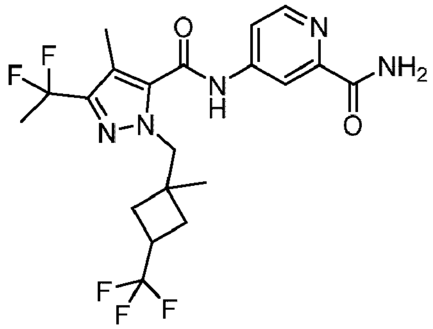 | 109 | 4-(3-(1,1-difluoroethyl)-4-methyl-1-((1-methyl-3-(trifluoromethyl)cyclobutyl)methyl)-1H-pyrazole-5-carboxamido)picolinamide |
| 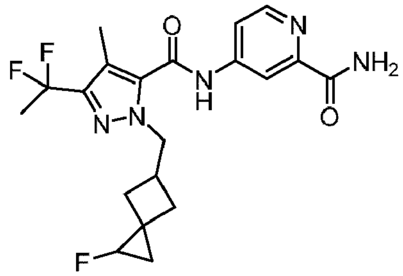 | 110 | 4-(3-(1,1-difluoroethyl)-1-((1-fluoro-spiro[2.3]hexan-5-yl)methyl)-4-methyl-1H-pyrazole-5-carboxamido) picolinamide |

(continued)

| Structure | Prophetic example number | Chemical name |
|---|---|---|
| 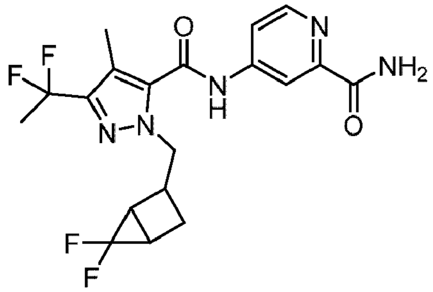 | 111 | 4-(1-((5,5-difluorobicyclo[2.1.0]pentan-2-yl)methyl)-3-(1,1-difluoroethyl)-4-methyl-1H-pyrazole-5-carboxamido)picolinamide |
| 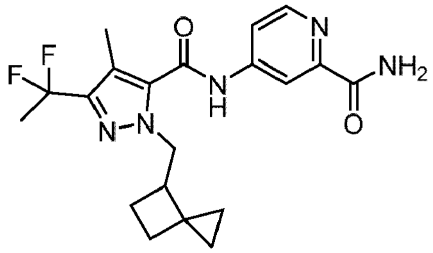 | 112 | 4-(3-(1,1-difluoroethyl)-4-methyl-1-(spiro[2.3]hexan-4-ylmethyl)-1H-pyrazole-5-carboxamido) picolinamide |
| 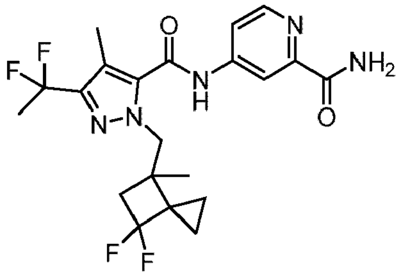 | 113 | 4-(1-((6,6-difluoro-4-methyl-spiro[2.3]hexan-4-yl) methyl)-3-(1,1-difluoroethyl)-4-methyl-1H-pyrazole-5-carboxamido)picolinamide |
| 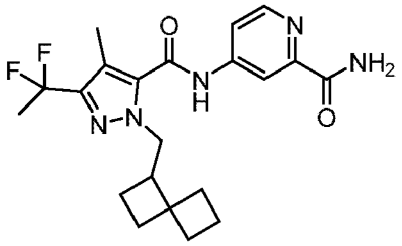 | 114 | 4-(3-(1,1-difluoroethyl)-4-methyl-1-(spiro[3.3] heptan-1-ylmethyl)-1H-pyrazole-5 -carboxamido) picolinamide |
| 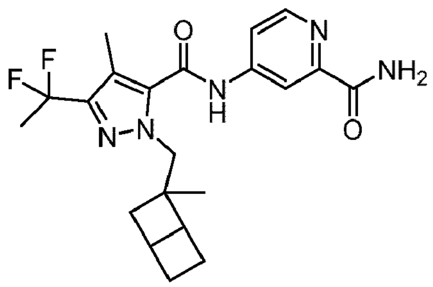 | 115 | 4-(3-(1,1-difluoroethyl)-4-methyl-1-((2-methylbicyclo [2.2.0]hexan-2-yl)methyl)-1H-pyrazole-5-carboxamido)picolinamide |

(continued)

| Structure | Prophetic example number | Chemical name |
|---|---|---|
| 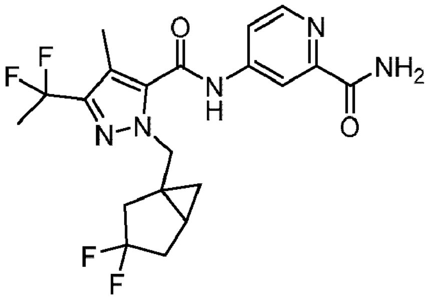 | 116 | 4-(1-((3,3-difluorobicyclo[3.1.0]hexan-1-yl)methyl)-3-(1,1-difluoroethyl)-4-methyl-1H-pyrazole-5-carboxamido)picolinamide |
| 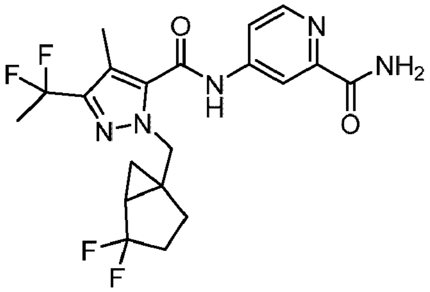 | 117 | 4-(1-((4,4-difluorobicyclo [3.1.0] hexan-1-yl)methyl)-3-(1,1-difluoroethyl)-4-methyl-1H-pyrazole-5-carboxamido)picolinamide |
| 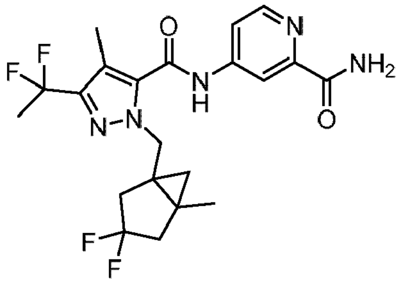 | 118 | 4-(1-((3,3-difluoro-5-methylbicyclo[3.1.0]hexan-1-yl)methyl)-3-(1,1-difluoroethyl)-4-methyl-1H-pyrazole-5-carboxamido)picolinamide |
| 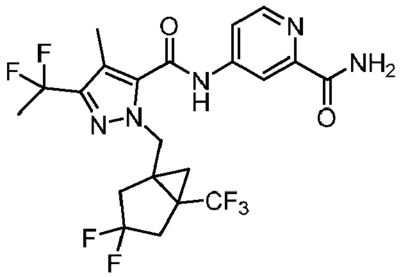 | 119 | 4-(1-((3,3-difluoro-5-(trifluoromethyl)bicyclo[3.1.0]hexan-1-yl)methyl)-3-(1,1-difluoroethyl)-4-methyl-1H-pyrazole-5-carboxamido)picolinamide |
| 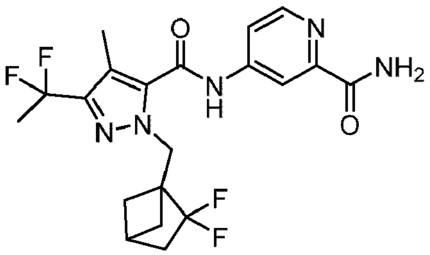 | 120 | 4-(1-((2,2-difluorobicyclo[2.1.1]hexan-1-yl)methyl)-3-(1,1-difluoroethyl)-4-methyl-1H-pyrazole-5-carboxamido)picolinamide |

(continued)

| Structure | Prophetic example number | Chemical name |
|---|---|---|
| 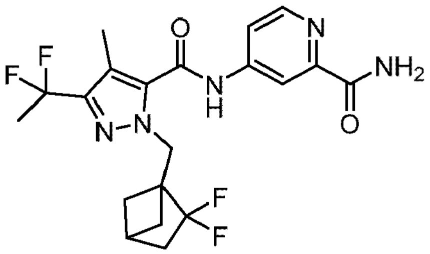 | 121 | 4-(1-((2,2-difluorobicyclo[2.1.1]hexan-1-yl)methyl)-3-(1,1-difluoroethyl)-4-methyl-1H-pyrazole-5-carboxamido)picolinamide |
| 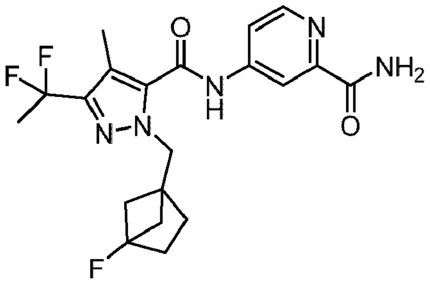 | 122 | 4-(3-(1,1-difluoroethyl)-1-((4-fluorobicyclo[2.1.1]hexan-1-yl)methyl)-4-methyl-1H-pyrazole-5-carboxamido)picolinamide |
| 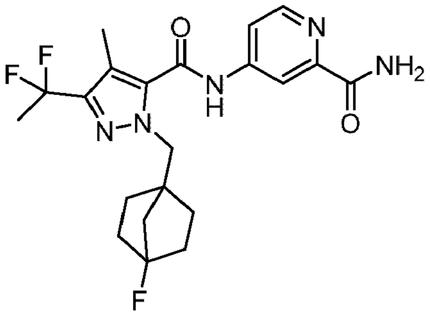 | 123 | 4-(3-(1,1-difluoroethyl)-1-((4-fluorobicyclo [22.1]heptan-1-yl)methyl)-4-methyl-1H-pyrazole-5-carboxamido)picolinamide |
| 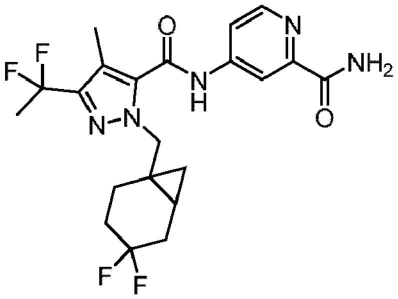 | 124 | 4-(1-((4,4-difluorobicyclo[4.1.0]heptan-1-yl)methyl)-3-(1,1-difluoroethyl)-4-methyl-1H-pyrazole-5-carboxamido)picolinamide |

**Electrophysiology: Voltage-Clamp recordings**

The following $Na_V$1.8 recombinant cell line was used for recordings: HEK-$Na_V$1.8 (NM 006514.1) with b3 (NM_018400.3).

Sodium currents were measured using the patch clamp technique in the whole-cell configuration using the Qube384 (Sophion A/S, Copenhagen, Denmark) automated voltage clamp platform. "Multi hole" plates were used for the cell line expressing $Na_V$1.8 while "single hole" plates were used for the recombinant cell lines expressing the other subtypes. Appropriate filters (for minimum seal resistance and minimum current size) and series resistance compensation (for high quality sodium channel recordings) were applied. Data was collected at ambient room temperature.

The recording intracellular solution contained (in mM): NaCl 145 mM, KCl 4 mM, $CaCl_2$ 2 mM, $MgCl_2$ 1 mM, HEPES 10 mM, Glucose 10 mM, pH 7.4 (NaOH). The extracellular recording solution contained (in mM): CsF 120 mM,

CsCl 20 mM, NaCl 10 mM, EGTA 10 mM, HEPES 10 mM, pH 7.2 (CsOH). Currents were recorded at 25 kHz sampling frequency and filtered at 5 kHz. Series resistance compensation was applied at 65%.

Vehicle (VEH) is the control condition where cells are exposed to 0.3 % DMSO without compound. All runs include VEH controls being exposed to the same voltage protocols to assess non-compound related phenomena such as run down and then used to isolate compound dependent effects on currents.

To check for *state-dependence inhibition,* the following voltage-sequence was applied every 20 seconds: From a resting membrane potential of-120 mV, the first test pulse (P1; 20 ms to -10 mV) was applied to check for channels in the Resting State followed by a brief recovery (20 ms to -120 mV), then holding the membrane voltage to $V_{1/2}$ (4 seconds at voltage to obtain half the channel at Rest and half Inactivated) with a subsequent second test pulse (P2; 20 ms to -10 mV) to check for channels in the Inactivated State, followed by another brief recovery (20 ms to -120 mV) and a final third test pulse (P3; 20 ms to -10 mV) to check for recovered channels.

To check for *frequency-dependent inhibition,* a 10 Hz protocol and a 20 Hz protocol were applied; namely,

From a resting membrane potential of -120 mV, 40-pulses (10 ms to -10 mV) was applied at 10 Hz (at 100 ms between pulses) and then at 20 Hz (at 50 ms between pulses). Each parameter was recorded (P1, P2, P3, P40 from 10Hz and P40 from 20Hz) during a control period (lasting ~ 5 minutes) when establishing the baseline and during compound period (lasting ~ 12 minutes) when test compound (or vehicle) was applied. For each parameter, the value at the end of the compound period was normalized to the vehicle baseline; as follows

$$Normalized\ Inhibition\ (Norm_{CPD}) = \frac{CPD\ Value_{end\ CPD\ period}}{VEH\ Value_{end\ Control\ Period}}$$

To adjust for any variance in the $Na^+$ current signal during the compound period (owing to cumulative inactivation independent of compound or shifts in biophysics over time), a dedicated segment of the recording wells in each 384 plate were dedicated to having only vehicle exposure. These vehicle-only recordings were used to correct for any apparent "run-up" or "run-down" in the experiment.

$$Normalized\ Inhibition\ (Norm_{VEH}) = \frac{VEH\ Value_{end\ CPD\ period}}{VEH\ Value_{end\ Control\ Period}}$$

The adjusted inhibition was calculated as follows:

$$\%\ Inhibition_{corrected} = 100 \times \frac{Norm_{CPD} - Norm_{VEH}}{100 - Norm_{VEH}}$$

Percent inhibition was determined and $IC_{50}$ values were calculated using a 4 parameter logistic model within XLFit Software (IDBS, Boston MA):

$$\%\ Inhibition_{corrected} = A + \frac{(B - A)}{(1 + \left(\frac{x}{C}\right)^{D}}$$

where A and B are the maximal and minimum inhibition respectively, C is the $IC_{50}$ concentration and D is the (Hill) slope.

The potency data of the example compounds are summarized in the table below (category **A:** human NaV1.8 $IC_{50} \leq 0.1$ μM; category **B:** 0.1 μM < human NaV1.8 $IC_{50} \leq 1$ μM; category **C:** 1 μM < human NaV1.8 $IC_{50}$ <_ 10 μM; "n.d.": not determined):

| Example | Potency category | Example | Potency category | Example | Potency category |
|---|---|---|---|---|---|
| 1 | B | 18 | A | 35 | A |
| 2 | B | 19 | B | 36 | A |
| 3 | B | 20 | B | 37 | C |
| 4 | A | 21 | A | 38 | C |
| 5 | n.d. | 22a | C | 39 | B |
| 6 | n.d. | 23 | B | 40 | B |
| 7 | n.d. | 24 | B | 41 | B |
| 8 | A | 25 | A | 42 | A |
| 9 | A | 26 | B | 43a | A |
| 10a | A | 27 | B | 43b | A |
| 10b | A | 28 | A | 43c | A |
| 10c | A | 29 | n.d. | 43d | A |
| 10d | A | 30 | C | 47 | C |
| 14 | B | 31 | C | 48 | C |
| 15 | A | 32 | B | 49 | B |
| 16 | A | 33 | C | | |
| 17a | B | 34 | B | | |

## Claims

**1.** A compound according to general formula (I)

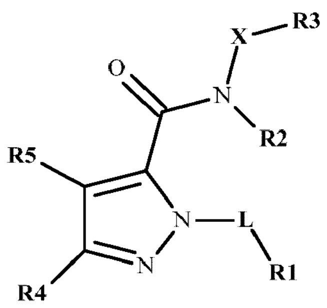

(I)

wherein

**L** represents $CH_2$, $CH(CH_3)$ or $CH(CH_2CH_3)$;
**R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl; with the proviso that when **R1** represents $C_{3-10}$-cycloalkyl or 4 to 10-membered heterocycloalkyl, said $C_{3-10}$-cycloalkyl or 4 to 10-membered heterocycloalkyl is substituted with at least two substituients, wherein said at least two substituents are independently from one another selected from $C_{1-6}$-alkyl, $CF_3$, $CF_2H$, and $CFH_2$;
**R2** represents H or $C_{1-6}$-alkyl;
**X** represents phenyl, or 5 to 10-membered heteroaryl;
**R3** represents $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), $S(O)$-$NH_2$, $S(O)$-$N(H)(C_{1-6}$-alkyl), $S(O)$-$N(C_{1-6}$-alkyl$)_2$, $S(O)_2$-$NH_2$,

$S(O)_2$-N(H)($C_{1-6}$-alkyl), $S(O)_2$-N(H)($C_{3-6}$-cycloalkyl), $S(O)_2$-N($C_{1-6}$-alkyl)$_2$, C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), $OCF_3$, $OCF_2H$, CN, OH, O-$C_{3-6}$-cycloalkyl, O-(4 to 6-membered heterocycloalkyl), S(O)-$C_{1-6}$-alkyl, S(O)-($C_{3-6}$-cycloalkyl), S(O)-(4 to 6-membered heterocycloalkyl), S(O)-phenyl, or S(O)-(5 or 6-membered heteroaryl);

**R4** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 11-membered bicycloalkyl, 5 to 11-membered spiroalkyl or bisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

**R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

wherein $C_{1-6}$-alkyl and $C_{1-6}$-alkylene in each case independently from one another is linear or branched;

wherein $C_{1-6}$-alkyl, $C_{1-6}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl, and 5 to 11-membered heterobicycloalkyl in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;

wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl;

in the form of the free compound or a physiologically acceptable salt thereof.

2. The compound according to claim 1, wherein **L** represents $CH_2$.

3. The compound according to claim 1 or 2, wherein **R1** represents $C_{3-10}$-cycloalkyl, substituted with two, three, four, or more substituents independently from one another selected from F, $CH_3$, $OCH_3$, CN, $CHF_2$ and $CF_3$; provided that at least two substituents are independently from one another selected from $CH_3$, $CHF_2$ and $CF_3$;
preferably selected from dimethyl cyclopropyl, trifluoromethyl methyl cyclopropyl, dimethyl difluoro cyclopropyl, dimethyl cyclobutyl, trifluoromethyl cyclobutyl, trifluoromethyl methyl cyclobutyl, dimethyl difluoro cyclobutyl, dimethyl cyclopentyl, trifluoromethyl cyclopentyl, trifluoromethyl methyl cyclopentyl, dimethyl difluoro cyclopentyl, dimethyl cyclohexyl, trifluoromethyl cyclohexyl, trifluoromethyl methyl cyclohexyl, dimethyl difluoro cyclohexyl, dimethyl cycloheptyl, trifluoromethyl cycloheptyl, trifluoromethyl methyl cycloheptyl, dimethyl difluoro cycloheptyl, dimethyl cyclooctyl, trifluoromethyl cyclooctyl, trifluoromethyl methyl cyclooctyl, dimethyl difluoro cyclooctyl, dimethyl cyclononyl, trifluoromethyl cyclononyl, trifluoromethyl methyl cyclononyl, dimethyl difluoro cyclononyl, dimethyl cyclodecyl, trifluoromethyl cyclodecyl, trifluoromethyl methyl cyclodecyl, and dimethyl difluoro cyclodecyl;
more preferably dimethyl cyclopropyl, trifluoromethyl methyl cyclopropyl, trifluoromethyl methyl cyclobutyl, and dimethyl difluoro cyclopentyl.

4. The compound according to claim 1 or 2, wherein **R1** represents 4 to 11-membered bicycloalkyl, unsubstituted or substituted with one, two, three, four, or more substituents independently from one another selected from F, $CH_3$, $OCH_3$, CN, $CHF_2$ and $CF_3$;
preferably selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethylbicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluoro bicyclo[2.1.0]pentyl, bicyclo[3.1.0]hexyl, methyl bicyclo[3.1.0]hexyl, trifluoromethyl bicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1]hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo [3.3.0]octyl, difluoro bicyclo [3.3.0]octyl, methyl difluoro bicyclo[3.3.0]octyl, and trifluoromethyl difluoro bicyclo[3.3.0]octyl;
more preferably bicyclo[1.1.1]pentyl, fluoro bicylo[1.1.1]pentyl, methoxy bicylo[1.1.1]pentyl, difluoromethyl bicylo[1.1.1]pentyl, trifluoromethyl bicylo[1.1.1]pentyl, bicyclo[2.1.0]pentyl, difluoro bicyclo [2.1.0]pentyl, difluoro bicyclo[3.1.0]hexyl, methyl difluoro bicyclo[3.1.0]hexyl, trifluoromethyl difluoro bicyclo[3.1.0]hexyl, methyl bicyclo[2.2.0]hexyl, bicyclo[2.1.1]hexyl, fluoro bicyclo[2.1.1]hexyl, difluoro bicyclo[2.1.1]hexyl, difluoro bicyclo[4.1.0]heptyl, fluoro bicyclo[2.2.1]heptyl, bicyclo [3.3.0]octyl or fluoro bicyclo [3.3.0]octyl.

5. The compound according to claim 1 or 2, wherein **R1** represents 5 to 11-membered spiroalkyl or dispiroalkyl, unsubstituted or substituted with one, two, three, four, or more substituents independently from one another selected from F, $CH_3$, $OCH_3$, CN, $CHF_2$ and $CF_3$;
preferably selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.0.2.1]heptyl;
more preferably spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, or dispiro[2.0.2.1]heptyl.

6. The compound according to claim 1 or 2, wherein **R1** represents 4 to 10-membered heterocycloalkyl, substituted with two, three, four, or more substituents independently from one another selected from F, $CH_3$, $OCH_3$, CN, $CHF_2$ and $CF_3$; provided that at least two substituents are independently from one another selected from $CH_3$, $CHF_2$ and $CF_3$;
preferably selected from oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl; in each case substituted with at least two substituients, wherein said at least two substituents are independently from one another selected from $C_{1-6}$-alkyl, $CF_3$, $CF_2H$, and $CFH_2$;
more preferably dimethyl oxetanyl.

7. The compound according to claim 1 or 2, wherein **R1** represents 5 to 11-membered heterobicycloalkyl, unsubstituted or substituted with one, two, three, four, or more substituents independently from one another selected from F, $CH_3$, $OCH_3$, CN, $CHF_2$ and $CF_3$;
preferably 2-oxa-bicyclo[2.1.1]hexyl or methyl 2-oxa-bicyclo[2.1.1]hexyl.

8. The compound according to any of the preceding claims, wherein **R2** represents H.

9. The compound according to any of the preceding claims, wherein **X** represents

- phenyl, wherein phenyl is unsubstituted or mono- or polysubstituted with one or more substituents selected from the group consisting of F, Cl, Br, CN, $CH_3$, $CF_3$, $CHF_2$, $CH_2F$, OH, $OCH_3$, $OCF_3$, $OCF_2H$, and $OCFH_2$; preferably F; or
- 5 to 10-membered heteroaryl selected from the group consisting of pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, and thiadiazolyl; preferably pyrazolyl or pyridyl;
wherein said 5 to 10-membered heteroaryl is unsubstituted or mono- or polysubstituted with one or more substituents selected from the group consisting of F, Cl, Br, CN, $CH_3$, $CF_3$, $CHF_2$, $CH_2F$, OH, $OCH_3$, $OCF_3$, $OCF_2H$, and $OCFH_2$; preferably F.

10. The compound according to any of the preceding claims, wherein **R3** represents $S(O)_2$-$C_{1-6}$-alkyl, $S(O)_2$-$NH_2$, or

C(O)-$NH_2$.

**11.** The compound according to any of the preceding claims, wherein **R4** represents

- H;
- $C_{1-6}$-alkyl;
preferably $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$;
more preferably $CF_2CH_3$, $CH_3$ or $CF_3$; or
- $C_{3-10}$-cycloalkyl, unsubstituted or substituted with one, two, three, four, or more substituents independently from one another selected from F, $CH_3$, $OCH_3$, CN, $CHF_2$ and $CF_3$;
preferably selected from cyclopropyl, methyl cyclopropyl, difluoromethyl cyclopropyl, trifluoromethyl cyclopropyl, cyano cyclopropyl, methoxy cyclopropyl, fluoro cyclopropyl, difluoro cyclopropyl, trifluoro cyclopropyl, cyclobutyl, methyl cyclobutyl, difluoromethyl cyclobutyl, trifluoromethyl cyclobutyl, cyano cyclobutyl, methoxy cyclobutyl, fluoro cyclobutyl, difluoro cyclobutyl, trifluoro cyclobutyl, cyclopentyl, methyl cyclopentyl, difluoromethyl cyclopentyl, trifluoromethyl cyclopentyl, cyano cyclopentyl, methoxy cyclopentyl, fluoro cyclopentyl, difluoro cyclopentyl, trifluoro cyclopentyl, cyclohexyl, methyl cyclohexyl, difluoromethyl cyclohexyl, trifluoromethyl cyclohexyl, cyano cyclohexyl, methoxy cyclohexyl, fluoro cyclohexyl, difluoro cyclohexyl, and trifluoro cyclohexyl;
more preferably cyclopropyl;
- 4 to 11-membered bicycloalkyl, unsubstituted or substituted with one, two, three, four, or more substituents independently from one another selected from F, $CH_3$, $OCH_3$, CN, $CHF_2$ and $CF_3$;
preferably bicyclo[1.1.1]pentyl;
- 5 to 11-membered spiroalkyl, unsubstituted or substituted with one, two, three, four, or more substituents independently from one another selected from F, $CH_3$, $OCH_3$, CN, $CHF_2$ and $CF_3$;
preferably spiro[2.2]pentyl or spiro[2.3]hexyl; or
- O-$C_{1-6}$-alkyl, unsubstituted or substituted with one, two, three, four, or more F;
preferably selected from O-$CHF_2$, O-$CH_2F$, O-$CF_3$, O-$CH_2CHF_2$, O-$CH_2CH_2F$, O-$CH_2CF_3$, O-$CF_2CH_3$, O-$CHFCH_3$, O-$CF_2CF_3$, O-$CHFCF_3$, O-$CH_3$, O-$CH_2CH_3$, or O-$CH(CH_3)_2$.

**12.** The compound according to any of the preceding claims, wherein **R5** represents $C_{1-6}$-alkyl or Cl; preferably $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$ or Cl; more preferably $CF_3$ or $CH_3$.

**13.** The compound according to any of the preceding claims, which is selected from the group consisting of

• 4-(1-((3-fluorobicyclo[1.1.1]pentan-1-yl)methyl)-3-methyl-4-(trifluoromethyl)-1H-pyrazole-5-carboxamido)picolinamide;
• 4-(3-(1,1-difluoroethyl)-1-((3-fluorobicyclo[1.1.1]pentan-1-yl)methyl)-4-methyl-1H-pyrazole-5-carboxamido)picolinamide;
• 1-((2-oxabicyclo[2.1.1]hexan-1-yl)methyl)-3-cyclopropyl-4-(trifluoromethyl)-1H-pyrazole-5-carboxamide;
• 3-(1,1- 4-(3-(1,1-difluoroethyl)-4-methyl-1-((3-(trifluoromethyl)bicyclo[1.1.1]pentan-1-yl)methyl)-1H-pyrazole-5-carboxamido)picolinamide;
• 4-(3-cyclopropyl-1-((1-methyl-2-oxabicyclo[2.1.1]hexan-4-yl)methyl)-4-(trifluoromethyl)-1H-pyrazole-5 -carboxamido)picolinamide;
• 4-(1-((3-methoxybicyclo[1.1.1]pentan-1-yl)methyl)-3-methyl-4-(trifluoromethyl)-1H-pyrazole-5-carboxamido)picolinamide;
• 4-(1-((3-(difluoromethyl)bicyclo[1.1.1]pentan-1-yl)methyl)-3-methyl-4-(trifluoromethyl)-1H-pyrazole-5 -carboxamido)picolinamide;
• 4-(4-methyl-3-(trifluoromethyl)-1-((3-(trifluoromethyl)bicyclo[1.1.1]pentan-1-yl)methyl)-1H-pyrazole-5 -carboxamido)picolinamide;
• 4-(3-cyclopropyl-4-(trifluoromethyl)-1-((3-(trifluoromethyl)bicyclo[1.1.1]pentan-1yl)methyl)-1H-pyrazole-5-carboxamido)picolinamide;
• 4-(1-((4,4-difluoro-1,2-dimethylcyclopentyl)methyl)-3-(1,1-difluoroethyl)-4-methyl-1H-pyrazole-5-carboxamido)picolinamide;
• 4-(3-cyclopropyl-1-((3-methoxybicyclo[1.1.1]pentan-1yl)methyl)-4-(trifluoromethyl)-1H-pyrazole-5 -carboxamido)picolinamide;
• 4-(3-cyclopropyl-1-((3-fluorobicyclo[1.1.1]pentan-1yl)methyl)-4-(trifluoromethyl)-1H-pyrazole-5-carboxamido)picolinamide ;

- 4-(3-cyclopropyl-1-((4-methyl-2-oxabicyclo[2.1.1]hexan-1-yl)methyl)-4-(trifluoromethyl)-1H-pyrazole-5 -carboxamido)picolinamide;
- 4-(3-cyclopropyl-1-((4,4-dimethyloxetan-2-yl)methyl)-4-(trifluoromethyl)-1H-pyrazole-5-carboxamido)-picolinamide;
- 4-(3-methyl-4-(trifluoromethyl)-1-((3-(trifluoromethyl)bicyclo[1.1.1]pentan-1-yl)methyl)-1H-pyrazole-5 -carboxamido)picolinamide;
- 3-cyclopropyl-1-((4-methyl-2-oxabicyclo[2.1.1]hexan-1-yl)methyl)-N-(2-(methylsulfonyl)pyridin-4-yl)-4-(trifluoromethyl)-1H-pyrazole-5-carboxamide;
- 3-cyclopropyl-1-((3-fluorobicyclo[1.1.1]pentan-1-yl)methyl)-N-(2-(methylsulfonyl)pyridin-4-yl)-4-(trifluoromethyl)-1H-pyrazole-5-carboxamide;
- 3-cyclopropyl-1-((3-fluorobicyclo[1.1.1]pentan-1-yl)methyl)-N-(2-(methylsulfonyl)pyridin-4-yl)-4-(trifluoromethyl)-1H-pyrazole-5-carboxamide;
- 3-cyclopropyl-1-((4,4-dimethyloxetan-2-yl)methyl)-N-(2-(methylsulfonyl)pyridin-4-yl)-4-(trifluoromethyl)-1H-pyrazole-5-carboxamide;
- 3-cyclopropyl-1-((1-methyl-2-oxabicyclo[2.1.1]hexan-4yl)methyl)-N-(2-(methylsulfonyl)pyridin-4-yl)-4-(trifluoromethyl)-1H-pyrazole-5-carboxamide;
- 1-((2-oxabicyclo[2.1.1]hexan-1-yl)methyl)-3-cyclopropyl-N-(2-(methylsulfonyl)pyridin-4-yl)-4-(trifluoromethyl)-1H-pyrazole-5-carboxamide;
- 3-(1,1-difluoroethyl)-4-methyl-N-(2-(methylsulfonyl)pyridin-4-yl)-1-((3-(trifluoromethyl)bicyclo-[1.1.1]pentan-1-yl)methyl)-1H-pyrazole-5-carboxamide;
- 3-methyl-N-(2-(methylsulfonyl)pyridin-4-yl)-4-(trifluoromethyl)-1-((3-(trifluoromethyl)bicyclo-[1.1.1]pentan-1-yl)methyl)-1H-pyrazole-5-carboxamide;
- 4-methyl-N-(2-(methylsulfonyl)pyridin-4-yl)-3-(trifluoromethyl)-1-((3-(trifluoromethyl)bicyclo-[1. 1. 1]pentan-1-yl)methyl)-1H-pyrazole-5-carboxamide;
- 3-cyclopropyl-N-(2-(methylsulfonyl)pyridin-4-yl)-4-(trifluoromethyl)-1-((3-(trifluoromethyl)bicyclo-[1.1.1]pentan-1-yl)methyl)-1H-pyrazole-5-carboxamide;
- 3-(1,1-difluoroethyl)-1-((3-fluorobicyclo[1.1.1]pentan-1-yl)methyl)-4-methyl-N-(2-(methylsulfonyl)-pyridin-4-yl)-1H-pyrazole-5-carboxamide;
- 1-((2-oxabicyclo[2.1.1]hexan-1-yl)methyl)-N-(3-sulfamoylphenyl)-4-(trifluoromethyl)-1H-pyrazole-5-carboxamide;
- 1-((1-methyl-2-oxabicyclo[2.1.1]hexan-4-yl)methyl)-N-(3-sulfamoylphenyl)-4-(trifluoromethyl)-1H-pyrazole-5-carboxamide;
- 1-(bicyclo[1.1.1]pentan-1-ylmethyl)-N-(3-sulfamoylphenyl)-4-(trifluoromethyl)-1H-pyrazole-5-carboxamide;
- 1-((2-oxabicyclo[2.1.1]hexan-1-yl)methyl)-3-methyl-N-(3-sulfamoylphenyl)-4-(trifluoromethyl)-1H-pyrazole-5-carboxamide;
- 1-(spiro[2.3]hexan-5-ylmethyl)-N-(3-sulfamoylphenyl)-4-(trifluoromethyl)-1H-pyrazole-5-carboxamide;
- 1-(bicyclo[2.1.1]hexan-1-ylmethyl)-N-(3-sulfamoylphenyl)-4-(trifluoromethyl)-1H-pyrazole-5-carboxamide;
- 1-(bicyclo[2.1.1]hexan-1-ylmethyl)-N-(3-carbamoyl-4-fluorophenyl)-3-methyl-4-(trifluoromethyl)-1H-pyrazole-5-carboxamide;
- 1-((2-oxabicyclo[2.1.1]hexan-1-yl)methyl)-N-(3-carbamoyl-4-fluorophenyl)-4-(trifluoromethyl)-1H-pyrazole-5-carboxamide;
- 1-(bicyclo[1.1.1]pentan-1-ylmethyl)-N-(3-carbamoyl-4-fluorophenyl)-4-(trifluoromethyl)-1H-pyrazole-5-carboxamide;
- 1-((2-oxabicyclo[2.1.1]hexan-1-yl)methyl)-N-(3-carbamoyl-4-fluorophenyl)-3-methyl-4-(trifluoromethyl)-1H-pyrazole-5-carboxamide;
- 1-(bicyclo [1.1. 1]pentan-1-ylmethyl)-N-(3 -carbamoyl-4-fluorophenyl)-3 -methyl-4-(trifluoromethyl)-1H-pyrazole-5-carboxamide;
- N-(3-carbamoyl-4-fluorophenyl)-1-(spiro[2.3]hexan-5-ylmethyl)-4-(trifluoromethyl)-1H-pyrazole-5-carboxamide;
- 1-(bicyclo[2.1.1]hexan-1-ylmethyl)-N-(3-carbamoyl-4-fluorophenyl)-4-(trifluoromethyl)-1H-pyrazole-5-carboxamide;
- 1-((4,4-difluoro-1,2-dimethylcyclopentyl)methyl)-3-(1,1-difluoroethyl)-4-methyl-N-(2-sulfamoylpyridin-4-yl)-1H-pyrazole-5-carboxamide;
- 1-(bicyclo[1.1.1]pentan-1-ylmethyl)-N-(1-(methylsulfonyl)-1H-pyrazol-4-yl)-4-(trifluoromethyl)-1H-pyrazole-5-carboxamide;
- N-(1-(methylsulfonyl)-1H-pyrazol-4-yl)-1-(spiro[2.3]hexan-5-ylmethyl)-4-(trifluoromethyl)-1H-pyrazole-5-carboxamide;
- 1-(bicyclo[2.1.1]hexan-1ylmethyl)-N-(1-(methylsulfonyl)-1H-pyrazol-4-yl)-4-(trifluoromethyl)-1H-pyrazole-5-carboxamide;

- 3-cyclopropyl-1-(spiro[2.2]pentan-1-ylmethyl)-N-(2-sulfamoylpyridin-4-yl)-4-(trifluoromethyl)-1H-pyrazole- S -carboxamide;
- 3-cyclopropyl-1-((2,2-difluorospiro[22]pentan-1-yl)methyl)-N-(2-sulfamoylpyridin-4-yl)-4-(trifluoromethyl)-1H-pyrazole-5-carboxamide;
- 3-cyclopropyl-1-(dispiro[2.02$^{4}$.1$^{3}$]heptan-7-ylmethyl)-N-(2-sulfamoylpyridin-4-yl)-4-(trifluoromethyl)-1H-pyrazole-5-carboxamide;
- 3 -cyclopropyl-1-((1,2-dimethylcyclopropyl)methyl)-N-(2-sulfamoylpyridin-4-yl)-4-(trifluoromethyl)-1H-pyrazole-5-carboxamide;
- 3-cyclopropyl-1-((1-methyl-2-(trifluoromethyl)cyclopropyl)methyl)-N-(2-sulfamoylpyridin-4 yl)-4-(trifluoromethyl)-1H-pyrazole-5-carboxamide;
- 1-(bicyclo[2.1.0]pentan-1-ylmethyl)-3-cyclopropyl-N-(2-sulfamoylpyridin-4-yl)-4-(trifluoromethyl)-1H-pyrazole-5-carboxamide;
- 3-cyclopropyl-1-(spiro[2.3]hexan-5-ylmethyl)-N-(2-sulfamoylpyridin-4-yl)-4-(trifluoromethyl)-1H-pyrazole-5-carboxamide;
- 3-cyclopropyl-1-((5-methylspiro[2.3]hexan-5-yl)methyl)-N-(2-sulfamoylpyridin-4-yl)-4-(trifluoromethyl)-1H-pyrazole-5-carboxamide;
- 3-cyclopropyl-1-((1-methyl-3-(trifluoromethyl)cyclobutyl)methyl)-N-(2-sulfamoylpyridin-4-yl)-4-(trifluoromethyl)-1H-pyrazole-5-carboxamide;
- 3-cyclopropyl-1-((1-fluorospiro[2.3]hexan-5-yl)methyl)-N-(2-sulfamoylpyridin-4-yl)-4-(trifluoromethyl)-1H-pyrazole-5-carboxamide;
- 3-cyclopropyl-1-((5,5-difluorobicyclo[2.1.0]pentan-2-yl)methyl)-N-(2-sulfamoylpyridin-4-yl)-4-(trifluoromethyl)-1H-pyrazole-5-carboxamide;
- 3-cyclopropyl-1-(spiro[2.3]hexan-4-ylmethyl)-N-(2-sulfamoylpyridin-4-yl)-4-(trifluoromethyl)-1H-pyrazole- S -carboxamide;
- 3-cyclopropyl-1-((6,6-difluoro-4-methylspiro[2.3]hexan-4-yl)methyl)-N-(2-sulfamoylpyridin-4-yl)-4-(trifluoromethyl)-1H-pyrazole-5-carboxamide;
- 3-cyclopropyl-1-(spiro[3.3]heptan-1-ylmethyl)-N-(2-sulfamoylpyridin-4-yl)-4-(trifluoromethyl)-1H-pyrazole-5-carboxamide;
- 3-cyclopropyl-1-((2-methylbicyclo[2.2.0]hexan-2yl)methyl)-N-(2-sulfamoylpyridin-4yl)-4-(trifluoromethyl)-1H-pyrazole-5-carboxamide;
- 3-cyclopropyl-1-((3,3-difluorobicyclo[3.1.0]hexan-1-yl)methyl)-N-(2-sulfamoylpyridin-4-yl)-4-(trifluoromethyl)-1H-pyrazole-5-carboxamide;
- 3-cyclopropyl-1-((4,4-difluorobicyclo[3.1.0]hexan-1-yl)methyl)-N-(2-sulfamoylpyridin-4-yl)-4-(trifluoromethyl)-1H-pyrazole-5-carboxamide;
- 3-cyclopropyl-1-((5-fluorooctahydropentalen-2-yl)methyl)-N-(2-sulfamoylpyridin-4-yl)-4-(trifluoromethyl)-1H-pyrazole-5-carboxamide;
- 3-cyclopropyl-1-((4,4-difluoro-1,2-dimethylcyclopentyl)methyl)-N-(2-sulfamoylpyridin-4-yl)-4-(trifluoromethyl)-1H-pyrazole-5-carboxamide;
- 3-cyclopropyl-1-((3,3-difluoro-5-methylbicyclo[3.1.0]hexan-1-yl)methyl)-N-(2-sulfamoylpyridin-4-yl)-4-(trifluoromethyl)-1H-pyrazole-5-carboxamide;
- 3-cyclopropyl-1-((3,3-difluoro-5-(trifluoromethyl)bicyclo[3.1.0]hexan-1-yl)methyl)-N-(2-sulfamoylpyridin-4-yl)-4-(trifluoromethyl)-1H-pyrazole-5-carboxamide;
- 3-cyclopropyl-1-((2,2-difluorobicyclo[2.1.1]hexan-1-yl)methyl)-N-(2-sulfamoylpyridin-4-yl)-4-(trifluoromethyl)-1H-pyrazole-5-carboxamide;
- 3-cyclopropyl-1-((3,3-difluorobicyclo[2.1.1]hexan-1-yl)methyl)-N-(2-sulfamoylpyridin-4-yl)-4-(trifluoromethyl)-1H-pyrazole-5-carboxamide;
- 3-cyclopropyl-1-((4-fluorobicyclo[2.1.1]hexan-1-yl)methyl)-N-(2-sulfamoylpyridin-4yl)-4-(trifluoromethyl)-1H-pyrazole-5-carboxamide;
- 3-cyclopropyl-1-((4-fluorobicyclo[2.1.1]hexan-1-yl)methyl)-N-(2-sulfamoylpyridin-4yl)-4-(trifluoromethyl)-1H-pyrazole-5-carboxamide;
- 3-cyclopropyl-1-((4,4-difluorobicyclo[4.1.0]heptan-1-yl)methyl)-N-(2-sulfamoylpyridin-4-yl)-4-(trifluoromethyl)-1H-pyrazole-5-carboxamide;
- 3-(1,1-difluoroethyl)-4-methyl-1-(spiro[2.2]pentan-1-ylmethyl)-N-(2-sulfamoylpyridin-4-yl)-1H-pyrazole-5-carboxamide;
- 3-(1,1-difluoroethyl)-1-((2,2-difluorospiro[2.2]pentan-1-yl)methyl)-4-methyl-N-(2-sulfamoylpyridin-4-yl)-1H-pyrazole-5-carboxamide;
- 3-(1,1-difluoroethyl)-1-(dispiro[2.0.2$^{4}$.1$^{3}$]heptan-7-ylmethyl)-4-methyl-N-(2-sulfamoylpyridin-4-yl)-1H-pyrazole-5-carboxamide;
- 3-(1,1-difluoroethyl)-1-((1,2-dimethylcyclopropyl)methyl)-4-methyl-N-(2-sulfamoylpyridin-4-yl)-1H-pyrazole-5-carboxamide;
- 3-(1,1-difluoroethyl)-4-methyl-1-((1-methyl-2-(trifluoromethyl)cyclopropyl)methyl)-N-(2-sulfamoylpyridin-4-yl)-1H-pyrazole-5-carboxamide;
- 1-(bicyclo[2.1.0]pentan-1-ylmethyl)-3-(1,1-difluoroethyl)-4-methyl-N-(2-sulfamoylpyridin-4-yl)-1H-pyrazole-5-carboxamide;
- 3-(1,1-difluoroethyl)-4-methyl-1-(spiro[2.3]hexan-5ylmethyl)-N-(2-sulfamoylpyridin-4yl)-1H-pyrazole-5-carboxamide;
- 3-(1,1-difluoroethyl)-4-methyl-1-((5-methylspiro[2.3]hexan-5-yl)methyl)-N-(2-sulfamoylpyridin-4-yl)-1H-pyrazole-5-carboxamide;
- 3-(1,1-difluoroethyl)-4-methyl-1-((1-methyl-3-(trifluoromethyl)cyclobutyl)methyl)-N-(2-sulfamoylpyridin-4-yl)-1H-pyrazole-5-carboxamide;
- 3-(1,1-difluoroethyl)-1-((1-fluorospiro[2.3]hexan-5-yl)methyl)-4-methyl-N-(2-sulfamoylpyridin-4-yl)-1H-pyrazole-5-carboxamide;
- 1-((5,5-difluorobicyclo[2.1.0]pentan-2-yl)methyl)-3-(1,1-difluoroethyl)-4-methyl-N-(2-sulfamoylpyridin-4-yl)-1H-pyrazole-5-carboxamide;
- 3-(1,1-difluoroethyl)-4-methyl-1-(spiro[2.3]hexan-4-ylmethyl)-N-(2-sulfamoylpyridin-4-yl)-1H-pyrazole-5-carboxamide;
- 1-((6,6-difluoro-4-methylspiro[2.3]hexan-4-yl)methyl)-3-(1,1-difluoroethyl)-4-methyl-N-(2-sulfamoylpyridin-4-yl)-1H-pyrazole-5-carboxamide;
- 3-(1,1-difluoroethyl)-4-methyl-1-(spiro[3.3]heptan-1-ylmethyl)-N-(2-sulfamoylpyridin-4-yl)-1H-pyrazole-5-carboxamide;
- 3-(1,1-difluoroethyl)-4-methyl-1-((2-methylbicyclo[2.2.0]hexan-2yl)methyl)-N-(2-sulfamoylpyridin-4-yl)-1H-pyrazole-5-carboxamide;
- 1-((3,3-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide;
- 1-((4,4-difluorobicyclo[3.1.0]hexan-1-yl)methyl)-3-(1,1-difluoroethyl)-4-methyl-N-(2-sulfamoylpyridin-4-yl)-1H-pyrazole-5-carboxamide;
- 3-(1, 1 -difluoroethyl)-1 -((5-fluorooctahydropentalen-2-yl)methyl)-4-methyl-N-(2-sulfamoylpyridin-4-yl)-1H-pyrazole-5-carboxamide;
- 1-((3,3-difluoro-5-methylbicyclo[3.1.0]hexan-1-yl)methyl)-3-(1,1-difluoroethyl)-4-methyl-N-(2-sulfamoylpyridin-4-yl)-1H-pyrazole-5-carboxamide;
- 1-((3,3-difluoro-5-(trifluoromethyl)bicyclo[3.1.0]hexan-1-yl)methyl)-3-(1,1-difluoroethyl)-4-methyl-N-(2-sulfamoylpyridin-4-yl)-1H-pyrazole-5-carboxamide;
- 1-((2,2-difluorobicyclo[2.1.1]hexan-1-yl)methyl)-3-(1,1-difluoroethyl)-4-methyl-N-(2-sulfamoylpyridin-4-yl)-1H-pyrazole-5-carboxamide;
- 1-((3,3-difluorobicyclo[2.1.1]hexan-1-yl)methyl)-3-(1,1-difluoroethyl)-4-methyl-N-(2-sulfamoylpyridin-4-yl)-1H-pyrazole-5-carboxamide;
- 3-(1,1-difluoroethyl)-1-((4-fluorobicyclo[2.1.1]hexan-1-yl)methyl)-4-methyl-N-(2-sulfamoylpyridin-4-yl)-1H-pyrazole-5-carboxamide;
- 3-(1,1-difluoroethyl)-1-((4-fluorobicyclo[2.2.1]heptan-1-yl)methyl)-4-methyl-N-(2-sulfamoylpyridin-4-yl)-1H-pyrazole-5-carboxamide;
- 1-((4,4-difluorobicyclo[4.1.0]heptan-1-yl)methyl)-3-(1,1-difluoroethyl)-4-methyl-N-(2-sulfamoylpyridin-4-yl)-1H-pyrazole-5-carboxamide;
- 4-(3-(1,1-difluoroethyl)-4-methyl-1-(spiro[2.2]pentan-1-ylmethyl)-1H-pyrazole-5-carboxamido)picolinamide;
- 4-(3-(1,1-difluoroethyl)-1-((2,2-difluorospiro[22]pentan-1-yl)methyl)-4-methyl-1H-pyrazole-5-carboxamido)picolinamide;
- 4-(3-(1,1-difluoroethyl)-1-(dispiro[2.0.$2^4$.$1^3$]heptan-7-ylmethyl)-4-methyl-1H-pyrazole-5-carboxamido)picolinamide;
- 4-(3-(1,1-difluoroethyl)-1-((1,2-dimethylcyclopropyl)methyl)-4-methyl-1H-pyrazole-5-carboxamido)picolinamide;
- 4-(3-(1,1-difluoroethyl)-4-methyl-1-((1-methyl-2-(trifluoromethyl)cyclopropyl)methyl)-1H-pyrazole-5-carboxamido)picolinamide;
- 4-(3-(1,1-difluoroethyl)-4-methyl-1-((1-methyl-2-(trifluoromethyl)cyclopropyl)methyl)-1H-pyrazole-5-carboxamido)picolinamide;
- 4-(3-(1,1-difluoroethyl)-4-methyl-1-(spiro[2.3]hexan-5-ylmethyl)-1H-pyrazole-5-carboxamido)picolinamide;
- 4-(3-(1,1-difluoroethyl)-4-methyl-1-((5-methylspiro[2.3]hexan-5-yl)methyl)-1H-pyrazole-5-carboxamido)picolinamide;

- 4-(3-(1,1-difluoroethyl)-4-methyl-1-((1-methyl-3-(trifluoromethyl)cyclobutyl)methyl)-1H-pyrazole-5-carboxamido)picolinamide;
- 4-(3-(1,1-difluoroethyl)-1-((1-fluorospiro[2.3]hexan-5-yl)methyl)-4-methyl-1H-pyrazole-5-carboxamido)picolinamide;
- 4-(1-((5,5-difluorobicyclo[2.1.0]pentan-2-yl)methyl)-3-(1,1-difluoroethyl)-4-methyl-1H-pyrazole-5-carboxamido)picolinamide;
- 4-(3-(1,1-difluoroethyl)-4-methyl-1-(spiro[2.3]hexan-4-ylmethyl)-1H-pyrazole-5-carboxamido)picolinamide;
- 4-(1-((6,6-difluoro-4-methylspiro[2.3]hexan-4-yl)methyl)-3-(1,1-difluoroethyl)-4-methyl-1H-pyrazole-5-carboxamido)picolinamide;
- 4-(3-(1,1-difluoroethyl)-4-methyl-1-(spiro[3.3]heptan-1-ylmethyl)-1H-pyrazole-5-carboxamido)picolinamide;
- 4-(3-(1,1-difluoroethyl)-4-methyl-1-((2-methylbicyclo[22.0]hexan-2-yl)methyl)-1H-pyrazole-5-carboxamido)picolinamide;
- 4-(1-((3,3-difluorobicyclo[3.1.0]hexan-1-yl)methyl)-3-(1,1-difluoroethyl)-4-methyl-1H-pyrazole-5-carboxamido)picolinamide;
- 4-(1-((4,4-difluorobicyclo[3.1.0]hexan-1-yl)methyl)-3-(1,1-difluoroethyl)-4-methyl-1H-pyrazole-5-carboxamido)picolinamide;
- 4-(1-((3,3-difluoro-5-methylbicyclo[3.1.0]hexan-1-yl)methyl)-3-(1,1-difluoroethyl)-4-methyl-1H-py razole-5 -carboxamido)picolinamide;
- 4-(1-((3,3-difluoro-5-(trifluoromethyl)bicyclo[3.1.0]hexan-1-yl)methyl)-3-(1,1-difluoroethyl)-4-methyl-1H-pyrazole-5-carboxamido)picolinamide;
- 4-(1-((2,2-difluorobicyclo[2.1.1]hexan-1-yl)methyl)-3-(1,1-difluoroethyl)-4-methyl-1H-pyrazole-5-carboxamido)picolinamide;
- 4-(1-((2,2-difluorobicyclo[2.1.1]hexan-1-yl)methyl)-3-(1,1-difluoroethyl)-4-methyl-1H-pyrazole-5-carboxamido)picolinamide;
- 4-(3-(1,1-difluoroethyl)-1-((4-fluorobicyclo[2.1.1]hexan-1-yl)methyl)-4-methyl-1H-pyrazole-5-carboxamido)picolinamide;
- 4-(3-(1,1-difluoroethyl)-1-((4-fluorobicyclo[2.2.1]heptan-1-yl)methyl)-4-methyl-1H-pyrazole-5-carboxamido)picolinamide; and
- 4-(1-((4,4-difluorobicyclo[4.1.0]heptan-1-yl)methyl)-3-(1,1-difluoroethyl)-4-methyl-1H-pyrazole-5-carboxamido)picolinamide;

in the form of the free compound or a physiologically acceptable salt thereof.

**14.** A pharmaceutical dosage form comprising a compound according to any of claims 1 to 13.

**15.** The pharmaceutical dosage form according to claim 14 for use in the treatment of pain.

Europäisches Patentamt
European Patent Office
Office européen des brevets

# EUROPEAN SEARCH REPORT

Application Number
EP 22 21 3346

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2009/049180 A2 (VERTEX PHARMA [US]; JOSHI PRAMOD [US] ET AL.) 16 April 2009 (2009-04-16) * paragraph [0016] * ----- | 1-15 | INV. C07D231/14 C07D401/12 C07D403/12 C07D405/14 A61P29/00 A61K31/4155 |
| A | WO 2020/092187 A1 (MERCK SHARP & DOHME [US]; BRESLIN MICHAEL J [US] ET AL.) 7 May 2020 (2020-05-07) * claims 1,18 * ----- | 1-15 | |
| A | WO 2020/092667 A1 (MERCK SHARP & DOHME [US]; ARASAPPAN ASHOK [US] ET AL.) 7 May 2020 (2020-05-07) * claims 1, 21 * ----- | 1-15 | |
| E | WO 2022/263498 A1 (GRUENENTHAL GMBH [DE]) 22 December 2022 (2022-12-22) * page 179 - page 182 * ----- | 1-15 | |
| | | | TECHNICAL FIELDS SEARCHED (IPC) |
| | | | C07D A61P |

The present search report has been drawn up for all claims

1

| Place of search | Date of completion of the search | Examiner |
|---|---|---|
| Munich | 26 May 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 21 3346

This annex lists the patent family members relating to the patent documents cited in the above-mentioned European search report. The members are as contained in the European Patent Office EDP file on
The European Patent Office is in no way liable for these particulars which are merely given for the purpose of information.

26-05-2023

| Patent document cited in search report | Publication date | Patent family member(s) | Publication date |
|---|---|---|---|
| WO 2009049180 A2 | 16-04-2009 | AU 2008310660 A1 | 16-04-2009 |
| | | CA 2701946 A1 | 16-04-2009 |
| | | CN 101883760 A | 10-11-2010 |
| | | EP 2227453 A2 | 15-09-2010 |
| | | JP 5555169 B2 | 23-07-2014 |
| | | JP 2011500598 A | 06-01-2011 |
| | | NZ 584475 A | 27-07-2012 |
| | | US 2009099233 A1 | 16-04-2009 |
| | | WO 2009049180 A2 | 16-04-2009 |
| WO 2020092187 A1 | 07-05-2020 | EP 3873468 A1 | 08-09-2021 |
| | | US 2022119363 A1 | 21-04-2022 |
| | | WO 2020092187 A1 | 07-05-2020 |
| WO 2020092667 A1 | 07-05-2020 | AR 116939 A1 | 30-06-2021 |
| | | AU 2019372057 A1 | 27-05-2021 |
| | | AU 2022287562 A1 | 02-02-2023 |
| | | BR 112021008524 A2 | 03-08-2021 |
| | | CA 3117927 A1 | 07-05-2020 |
| | | CL 2021001078 A1 | 29-10-2021 |
| | | CN 113272293 A | 17-08-2021 |
| | | CO 2021005553 A2 | 30-07-2021 |
| | | CR 20210209 A | 20-05-2021 |
| | | DO P2021000082 A | 22-07-2021 |
| | | EA 202191177 A1 | 28-07-2021 |
| | | EC SP21030066 A | 31-05-2021 |
| | | EP 3873893 A1 | 08-09-2021 |
| | | IL 282468 A | 31-05-2021 |
| | | JP 2022506146 A | 17-01-2022 |
| | | KR 20210086687 A | 08-07-2021 |
| | | MA 54076 A | 09-02-2022 |
| | | NI 202100029 A | 13-08-2021 |
| | | PE 20211693 A1 | 01-09-2021 |
| | | SG 11202104326T A | 28-05-2021 |
| | | TW 202031643 A | 01-09-2020 |
| | | US 2020140411 A1 | 07-05-2020 |
| | | US 2021387966 A1 | 16-12-2021 |
| | | US 2022289710 A1 | 15-09-2022 |
| | | WO 2020092667 A1 | 07-05-2020 |
| WO 2022263498 A1 | 22-12-2022 | US 2023025025 A1 | 26-01-2023 |
| | | WO 2022263498 A1 | 22-12-2022 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020092187 A
- WO 2020092667 A
- WO 2009049180 A
- WO 2009049183 A
- WO 2009049181 A
- WO 2021113627 A


### Non-patent literature cited in the description


- **LUMPKIN ; CATERINA.** *Nature,* 2007, vol. 445, 858-865
- **CRAWFORD ; CATERINA.** *Toxicologic Pathology,* 2020, vol. 48 (1), 174
- **GOODWIN G ; MCMAHON S.B.** *Nature Reviews Neuroscience,* 2021, vol. 22, 263-274
- **BENNETT D.L. et al.** *Physiol Rev,* 2019, vol. 99, 1079-1151
- **AKOPIAN A.N. et al.** *Nature,* 1996, vol. 379, 257-261
- **SANGAMESWARAN L. et al.** *J. Biol. Chem.,* 1996, vol. 271, 5953-5956
- **PERSSON A.K. et al.** *Mol. Pain.,* 2010, vol. 6, 84
- **RUSH A.M. et al.** *Eur.J.Neurosci,* 2005, vol. 22, 39-49
- **AKOPIAN A.N. et al.** *Nat. Neurosci,* 1999, vol. 2, 541-548
- **NOVAKOVIC S.D. et al.** *J. Neurosci.,* 1998, vol. 18, 2184-2187
- **BLAIR N.T. et al.** *J. Neurosci.,* 2003, vol. 23, 10338-10350
- **RENGANATHAN M et al.** *J. Neurophysiol,* 2001, vol. 86, 629-640
- **CHOI J.S.** *J. Neurophysiol,* 2011, vol. 106, 3173-3184
- **TAN Z.Y. et al.** *J. Neurosci.,* 2014, vol. 34, 7190-7197
- **FABER C.G. et al.** *Ann. Neurol,* 2012, vol. 71, 26-39
- **HAN C et al.** *J. Neurol Neurosurg Psychiatry,* 2014, vol. 85, 499-505
- **KIST A.M. et al.** *PLoS One,* 2016, vol. 11, e0161789
- **EIJKENBOOM I. et al.** *J. Neurol Neurosurg Psychiatry,* 2019, vol. 90 (3), 342-352
- **LAIRD J.M. et al.** *J. Neurosci,* 2002
- *J. Neurosci,* vol. 22, 8352-8356
- **JARVIS M.F. et al.** *Proc Natl Acad Sci USA,* 2007, vol. 104, 8520-8525
- **JOSHI S.K. et al.** *Pain,* 2006, vol. 123, 75-82
- **ROZA C. et al.** *J Physiol,* 2003, vol. 550, 921-926
- **KORT M.E. et al.** *Bioorg Med Chem Lett,* 2010, vol. 20, 6812-6815
- **SCANIO M.J. et al.** *Bioorg Med Chem,* 2010, vol. 18, 7816-7825
- **PAYNE C.E. et al.** *Br J Pharmacol,* 2015, vol. 172, 2654-2670
- **HILLE, B.** *J. Gen. Physiol.,* 1977, vol. 69, 497-515
- **HILLE. B.** *Ion Channels of Excitable Membranes,* 1992, 391-421
- **HONDEGHEM L.M. ; KATZUNG B.G.** *Annu. Rev. Pharmacol. Toxicol.,* 1984, vol. 24, 387-423
- **CATTERALL W.A.** *Trends Pharmacol. Sci.,* 1987, vol. 8, 57-65
- *Chem. Rev.,* 2009, vol. 109 (6), 2551-2651
- **T. W. GREEN ; P. G. M. WUTS.** Protective Groups in Organic Synthesis. Wiley-Interscience, 1999
- *Synthesis,* 2002, 2561-2578
- *Chem. Soc. Rev,* 2021, vol. 50, 8214-8247
- March's Advanced Organic Chemistry. 2007, 1427-1474
- *Current Organic Synthesis,* 2011, vol. 8, 53
- *Chem. Rev.,* 2016, vol. 116, 12564
- *Chem. Soc. Rev,* 2014, vol. 43, 3525
- *Chem. Sci.,* 2010, vol. 1, 13